(19) Europäisches Patentamt
European Patent Office
Office européen des brevets

(11) EP 2 056 799 B1

(12) **EUROPEAN PATENT SPECIFICATION**

(45) Date of publication and mention of the grant of the patent:
**31.07.2013 Bulletin 2013/31**

(21) Application number: **07841303.6**

(22) Date of filing: **24.08.2007**

(51) Int Cl.:
***A61K 31/00*** *(2006.01)* ***A61K 31/4709*** *(2006.01)*
***A61K 31/4725*** *(2006.01)* ***A61K 45/06*** *(2006.01)*
***A61P 27/06*** *(2006.01)*

(86) International application number:
**PCT/US2007/076706**

(87) International publication number:
**WO 2008/027796 (06.03.2008 Gazette 2008/10)**

(54) **COMPOSITIONS AND METHODS FOR TREATING OR PREVENTING GLAUCOMA OR PROGRESSION THEREOF**

ZUSAMMENSETZUNGEN UND VERFAHREN ZUR BEHANDLUNG ODER VORBEUGUNG VON GLAUKOMEN ODER DEREN WEITERENTWICKLUNG

COMPOSITIONS ET MÉTHODES DE TRAITEMENT OU DE PRÉVENTION DU GLAUCOME OU DE SON ÉVOLUTION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **31.08.2006 US 841437 P**

(43) Date of publication of application:
**13.05.2009 Bulletin 2009/20**

(60) Divisional application:
**11181339.0 / 2 397 140**

(73) Proprietor: **Bausch & Lomb Incorporated**
**Rochester, NY 14604-2701 (US)**

(72) Inventor: **BARTELS, Stephen, P.**
**Pittsford, NY 14534 (US)**

(74) Representative: **Glas, Holger et al**
**Maiwald Patentanwalts GmbH**
**Elisenhof**
**Elisenstrasse 3**
**80335 München (DE)**

(56) References cited:
**WO-A-03/059899**
**WO-A-03/082280**
**WO-A-03/082787**
**WO-A-03/104195**
**WO-A-2004/063163**
**WO-A-2004/075864**
**WO-A-2005/095401**
**WO-A-2008/021729**
**US-A1- 2006 116 396**
**US-B2- 6 903 215**
**US-B2- 6 960 581**

- **T.A. GRAUL ET AL.: "Inflammatory glaucoma", DUANE'S OPHTHALMOLOGY, vol. 3, no. Chapter 54D, 2006,**
- **B.J. GASKIN ET AL.: "Neovascular glaucoma", EYE, vol. 19, 2005, pages 599-601,**
- **X. ZHOU ET AL.: "Involvement of inflammation, degradation, and apoptosis in a mouse model of glaucoma", THE JOURNAL OF BIOLOGICAL CHEMISTRY, vol. 280, no. 35, 2005, pages 31240-31248,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

CROSS-REFERENCE

This application claims the benefit of Provisional Patent Application No. 60/841,437 filed August 31, 2006

BACKGROUND OF THE INVENTION

The present invention relates to compositions for treating or preventing glaucoma or progression thereof. In particular, the present invention relates to compositions that comprise dissociated glucocorticoid receptor agonists ("DIGRAs") for the treatment or prevention of glaucoma or its progression, wherein said glaucoma results from chronic inflammation.

Glaucoma is a group of diseases that are characterized by the death of retinal ganglion cells ("RGCs"), specific visual field loss, and optic nerve atrophy. Glaucoma is the third leading cause of blindness worldwide. An intraocular pressure ("IOP") that is high compared to the population mean is a risk factor for the development of glaucoma. However, many individuals with high IOP do not have glaucomatous loss of vision. Conversely, there are glaucoma patients with normal IOP. Therefore, continued efforts have been devoted to elucidate the pathogenic mechanisms of glaucomatous optic nerve degeneration.

It has been postulated that optic nerve fibers are compressed by high IOP, leading to an effective physiological axotomy and problems with axonal transport. High IOP also results in compression of blood vessels supplying the optic nerve heads ("ONHs"), leading to the progressive death of RGCs. See; e.g., M. Rudzinski and H.U. Saragovi, Curr. Med. Chem.-Central Nervous System Agents, Vol. 5, 43 (2005).

In addition, there is growing evidence that other molecular mechanisms also cause direct damage to RGCs: existence of high levels of neurotoxic substances such as glutamate and nitric oxide and pro-inflammatory processes. *Id.* At low concentrations, NO plays a beneficial role in neurotransmission and vasodilation, while at higher concentrations, it is implicated in having a role in the pathogenesis of stroke, demyelination, and other neurodegenerative diseases. R.N. Saha and K. Pahan, Antioxidants & Redox Signaling, Vol. 8, No. 5 & 6, 929 (2006). NO has been recognized as a mediator and regulator of inflammatory responses. It possesses cytotoxic properties and is produced by immune cells, including macrophages, with the aim of assisting in the destruction of pathogenic microorganisms, but it can also have damaging effects on host tissues. NO can also react with molecular oxygen and superoxide anion to produce reactive nitrogen species that can modify various cellular functions. R. Korhonen et al., Curr. Drug Target-Inflam. & Allergy, Vol. 4, 471 (2005). Furthermore, oxidative stress, occurring not only in the trabecular meshwork ("TM") but also in retinal cells, appears to be involved in the neuronal cell death affecting the optic nerve in primary open-angle glaucoma ("POAG"). A. Izzotti et al., Mutat. Res., Vol. 612, No. 2, 105 (2006).

In addition, tumor necrosis factor-a ("TNF-$\alpha$"), a proinflammatory cytokine, has recently been identified to be a mediator of RGC death. TNF-$\alpha$ and TNF-$\alpha$ receptor-1 are up-regulated in experimental rat models of glaucoma. In vitro studies have further identified that TNF-$\alpha$-mediated RGC death involves the activation of both receptor-mediated caspase cascade and mitochondria-mediated caspase-dependent and caspase-independent components of cell death cascade. G. Tezel and X. Yang, Expt'l Eye Res., Vol. 81, 207 (2005). Moreover, TNF-$\alpha$ and its receptor were found in greater amounts in retina sections of glaucomatous eyes than in control eyes of age-matched normal donors. G. Tezel et al., Invest. Ophthalmol. & Vis. Sci., Vol. 42, No.8,1787 (2001).

Therefore, there has been growing evidence that glaucoma may have a root cause in chronic inflammation. Failure to control the insult-induced immune response can result in autoimmune pathogenesis and likely initiates or sustains glaucomatous neurodegeneration in many patients.

A traditional therapy for glaucoma has been IOP-lowering medicaments, for example, by topical administration. However, in light of new evidence, such a course of treatment may not address the inflammatory root cause of the disease that the current body of evidence suggests.

Glucocorticoids (also referred to herein as "corticosteroids") represent one of the most effective clinical treatment for a range of inflammatory conditions, including acute inflammation. However, steroidal drugs can have side effects that threaten the overall health of the patient. Chronic administration of glucocorticoids can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

In addition, it is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevations associated with the topical ophthalmic use of

glucocorticoids increases over time. In other words, the chronic (i.e., long-term) use of these agents increases the risk of significant IOP elevations. Therefore, an inflammatory root cause of glaucoma would not be treated with glucocorticoids, as they would exacerbate the condition they are intended to treat.

US 6,903,215 B2, US 6,960,581 B2, WO 03/082787 A1, WO 03/082280 A1, WO 03/59899 A1, WO 03/04195 A1, WO 2004/063163 A1, WO 2004/075864 A1, and WO 2005/095401 A1 relate to glucocorticoid mimetics or ligands, methods of making such compounds, their use in pharmaceutical compositions, and their use in modulating the glucocorticoid receptor function, and treating disease-states or conditions mediated by the glucocorticoid receptor function in a patient in need of such treatment.

US 2006/0116396 A1 is concerned with 5-substituted quinoline and isoquinoline derivatives, a process for their production and their use as anti-inflammatory agents. WO 2008/021729 A2 describes a composition for treating, reducing, ameliorating, or alleviating a back-of-the- eye condition or disorder that has an etiology in inflammation comprises a dissociated glucocorticoid receptor agonist ("DIGRA").

Therefore, there is a continued need to provide compounds, compositions, for treating or preventing glaucoma or progression thereof. In addition, it is also very desirable to provide such compounds, compositions that at least have few or only low levels of side effects.

SUMMARY OF THE INVENTION

The present invention provides a composition comprising a dissociated glucocorticoid receptor agonist ("DIGRA"), a pharmaceutically acceptable salt thereof, or a pharmaceutically acceptable ester thereof for treating or preventing glaucoma or progression thereof in a subject, wherein the DIGRA has Formula I

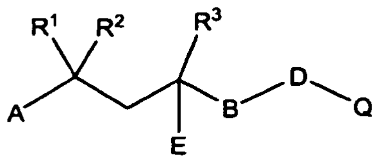

(I)

wherein A is a dihydrobenzofuranyl group substituted with a halogen atom; Q is a quinolinyl of isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; E is the hydroxy group, $R^3$ is a trifluoromethyl group, and wherein said glaucoma results from chronic inflammation.

According to another aspect, a composition is provided comprising a dissociated glucocorticoid receptor agonist ("DIGRA"), a pharmaceutically acceptable salt thereof, or a pharmaceutically acceptable ester thereof for treating or preventing glaucoma or progression thereof in a subject, wherein the DIGRA has Formula II

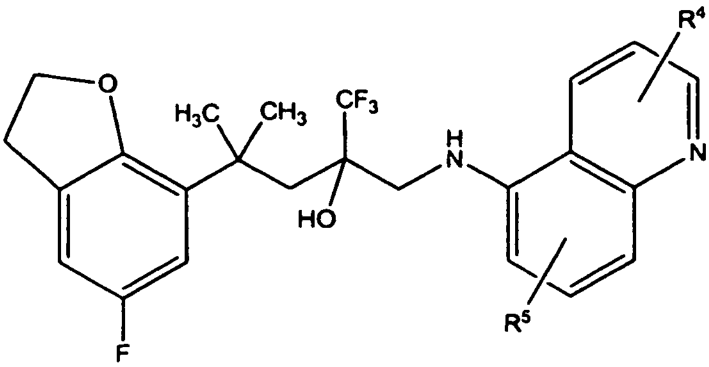

(II)

or Formula III

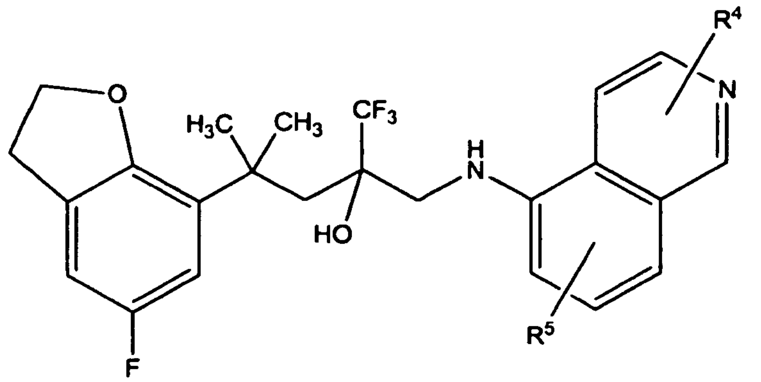

(III)

wherein $R^4$ and $R^5$ are independently selected from the group consisting of hydrogen, halogen, cyano, hydroxy, $C_1$-$C_{10}$ alkoxy groups, unsubstituted $C_1$-$C_{10}$ linear or branched alkyl groups, substituted $C_1$-$C_{10}$ linear or branched alkyl groups, unsubstituted $C_1$-$C_{10}$ cyclic alkyl groups, and substituted $C_1$-$C_{10}$ cyclic alkyl groups, wherein said glaucoma results from chronic inflammation.

According to still another aspect, the use of a DIGRA, a pharmaceutically acceptable salt thereof, or a pharmaceutically acceptable ester thereof for the preparation of a medicament for treating or preventing glaucoma or progression thereof is provided, wherein the DIGRA has Formula I

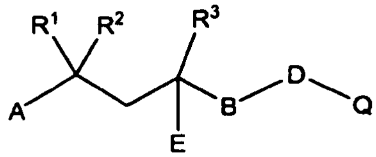

(I)

wherein A is a dihydrobenzofuranyl group substituted with a halogen atom; Q is a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; and E is the hydroxy group, $R^3$ is a trifluoromethyl group, and wherein said glaucoma results from chronic inflammation.

In one aspect, such glaucoma condition is selected from the group consisting of primary open-angle glaucoma, primary angle-closure glaucoma, secondary open-angle glaucoma, secondary angle-closure glaucoma, pigmentary glaucoma, neovascular glaucoma, pseudophakic glaucoma, malignant glaucoma, uveitic glaucoma, glaucoma due to peripheral anterior synechia, and combinations thereof.

In still another aspect, a composition of the present invention further comprises an additional anti-inflammatory agent selected from the group consisting of non-steroidal anti-inflammatory drugs ("NSAIDs"), peroxisome proliferator-activated receptor ("PPAR") ligands, anti-histaminic drugs, antagonists to or inhibitors of proinflammatory cytokines (such as anti-TNF, anti-interleukin, anti-NF-κB), nitric oxide synthase inhibitors, combinations thereof, and mixtures thereof.

Other features and advantages of the present invention will become apparent from the following detailed description and claims.

BRIEF DESCRIPTION OF THE FIGURES

Figures 1A-1F show the effects of BOL-303242-X and dexamethasone on the IL-1β-stimulated production of Il-6, IL-7, TGF-α, TNF-α, VGEF, and MCP-1 in human corneal epithelium cells ("HCECs") at $p < 0.05$.

Figure 2 shows the effects of BOL-303242-X and dexamethasone on the IL-1β-stimulated production of G-CSF in HCECs at $p < 0.05$.

Figures 3A-1C show the effects of BOL-303242-X and dexamethasone on the IL-1β-stimulated production ofGM-CSF, IL-8, and RANTES in HCECs at $p < 0.05$.

In these figures, "*" denotes comparison to control, and "**" to IL-1β.

DETAILED DESCRIPTION OF THE INVENTION

As used herein, a dissociated glucocorticoid receptor agonist ("DIGRA") is a compound that is capable of binding to the glucocorticoid receptor (which is a polypeptide) and, upon binding, is capable of producing differentiated levels of transrepression and transactivation of gene expression. A compound that binds to a polypeptide is sometimes herein referred to as a ligand.

As used herein, the term "alkyl" or "alkyl group" means a linear- or branched-chain saturated aliphatic hydrocarbon monovalent group, which may be unsubstituted or substituted. The group may be partially or completely substituted with halogen atoms (F, Cl, Br, or I). Non-limiting examples of alkyl groups include methyl, ethyl, n-propyl, 1-methylethyl (isopropyl), n-butyl, n-pentyl, 1,1-dimethylethyl (t-butyl), and the like. It may be abbreviated as "Alk".

As used herein, the term "alkenyl" or "alkenyl group" means a linear- or branched-chain aliphatic hydrocarbon monovalent radical containing at least one carbon-carbon double bond. This term is exemplified by groups such as ethenyl, propenyl, n-butenyl, isobutenyl, 3-methylbut-2-enyl, n-pentenyl, heptenyl, octenyl, decenyl, and the like.

As used herein, the term "alkynyl" or "alkynyl group" means a linear- or branched-chain aliphatic hydrocarbon monovalent radical containing at least one carbon-carbon triple bond. This term is exemplified by groups such as ethynyl, propynyl, n-butynyl, 2-butynyl, 3-methylbutynyl, n-pentynyl, heptynyl, octynyl, decynyl, and the like.

As used herein, the term "alkylene" or "alkylene group" means a linear- or branched-chain saturated aliphatic hydrocarbon divalent radical having the specified number of carbon atoms. This term is exemplified by groups such as methylene, ethylene, propylene, n-butylene, and the like, and may alternatively and equivalently be denoted herein as "-(alkyl)-".

The term "alkenylene" or "alkenylene group" means a linear- or branched-chain aliphatic hydrocarbon divalent radical having the specified number of carbon atoms and at least one carbon-carbon double bond. This term is exemplified by groups such as ethenylene, propenylene, n-butenylene, and the like, and may alternatively and equivalently be denoted herein as "-(alkylenyl)-".

The term "alkynylene" or "alkynylene group" means a linear- or branched-chain aliphatic hydrocarbon divalent radical containing at least one carbon-carbon triple bond. This term is exemplified by groups such as ethynylene, propynylene, n-butynylene, 2-butynylene, 3-methylbutynylene, n-pentynylene, heptynylene, octynylene, decynylene, and the like, and may alternatively and equivalently be denoted herein as "-(alkynyl)-".

As used herein, the term "aryl" or "aryl group" means an aromatic carbocyclic monovalent or divalent radical of from 5 to 14 carbon atoms having a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Non-limiting examples of aryl groups include phenyl, naphthyl, anthryl, phenanthryl, indanyl, indenyl, biphenyl, and the like. It may be abbreviated as "Ar".

The term "heteroaryl" or "heteroaryl group" means a stable aromatic 5- to 14-membered, monocyclic or polycyclic monovalent or divalent radical, which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Non-limiting examples of heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

The term "heterocycle", "heterocycle group", "heterocyclyl", "heterocyclyl group", "heterocyclic", or "heterocyclic group" means a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in at least one ring independently selected from nitrogen, oxygen,

and sulfur, wherein any sulfur, heteroatoms may optionally be oxidized and any nitrogen heteroatom may optionally be oxidized or be quaternized. As used herein, a heterocyclyl group excludes heterocycloalkyl, heterocycloalkenyl, and heterocycloalkynyl groups. Unless otherwise specified, the heterocyclyl ring may be attached at any suitable heteroatom or carbon atom which results in a stable structure and, if substituted, may be substituted at any suitable heteroatom or carbon atom which results in a stable structure. Non-limiting examples of heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl, and the like.

The term "cycloalkyl" or "cycloalkyl group" means a stable aliphatic saturated 3- to 15-membered monocyclic or polycyclic monovalent radical consisting solely of carbon and hydrogen atoms which may comprise one or more fused or bridged ring(s), preferably a 5- to 7-membered monocyclic or 7- to 10-membered bicyclic ring. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

The term "cycloalkenyl" or "cycloalkenyl group" means a stable aliphatic 5-to 15-membered monocyclic or polycyclic monovalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenyl ring may be attached at any carbon atom which results in a stable structure and, if substituted, may be substituted at any suitable carbon atom which results in a stable structure. Exemplary cycloalkenyl groups include cyclopentenyl, cyclohexenyl, cycloheptenyl, cyclooctenyl, cyclononenyl, cyclodecenyl, norbomenyl, 2-methylcyclopentenyl, 2-methylcyclooctenyl, and the like.

The term "cycloalkynyl" or "cycloalkynyl group" means a stable aliphatic 8-to 15-membered monocyclic or polycyclic monovalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynyl ring may be attached at any carbon atom which results in a stable structure and, if substituted, may be substituted at any suitable carbon atom which results in a stable structure. Exemplary cycloalkynyl groups include cyclooctynyl, cyclononynyl, cyclodecynyl, 2-methylcyclooctynyl, and the like.

The term "carbocycle" or "carbocyclic group" means a stable aliphatic 3- to 15-membered monocyclic or polycyclic monovalent or divalent radical consisting solely of carbon and hydrogen atoms which may comprise one or more fused or bridged rings, preferably a 5- to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene, and the like.

The terms "heterocycloalkyl", "heterocycloalkenyl", and "heterocycloalkynyl" mean cycloalkyl, cycloalkenyl, and cycloalkynyl group, respectively, having at least a heteroatom in at least one ring, respectively.

Glucocorticoids ("GCs") are among the most potent drugs used for the treatment of allergic and chronic inflammatory diseases or of inflammation resulting from infections. However, as mentioned above, long-term treatment with GCs is often associated with numerous adverse side effects, such as diabetes, osteoporosis, hypertension, glaucoma, or cataract. These side effects, like other physiological manifestations, are results of aberrant expression of genes responsible for such diseases. Research in the last decade has provided important insights into the molecular basis of GC-mediated actions on the expression of GC-responsive genes. GCs exert most of their genomic effects by binding to the cytoplasmic GC receptor ("GR"). The binding of GC to GR induces the translocation of the GC-GR complex to the cell nucleus where it modulates gene transcription either by a positive (transactivation) or negative (transrepression) mode of regulation. There has been growing evidence that both beneficial and undesirable effects of GC treatment are the results of undifferentiated levels of expression of these two mechanisms; in other words, they proceed at similar levels of effectiveness. Although it has not yet been possible to ascertain the most critical aspects of action of GCs in chronic inflammatory diseases, there has been evidence that it is likely that the inhibitory effects of GCs on cytokine synthesis are of particular importance. GCs inhibit the transcription, through the transrepression mechanism, of several cytokines that are relevant in inflammatory diseases, including IL-1β (interleukin-1β), IL-2, IL-3, IL-6, IL-11, TNF-α (tumor necrosis factor-α), GM-CSF (granulocyte-macrophage colony-stimulating factor), and chemokines that attract inflammatory cells to the site of inflammation, including IL-8, RANTES, MCP-1 (monocyte chemotactic protein-1), MCP-3, MCP-4, MIP-1α (macrophage-inflammatory protein-1α), and eotaxin. P.J. Barnes, Clin. Sci., Vol. 94, 557-572 (1998). On the other hand, there is persuasive evidence that the synthesis of IκBα, which are proteins having inhibitory effects on the NF-κB proinflammatory transcription factors, is increased by GCs. These proinflammatory transcription factors regulate the expression of genes that code for many inflammatory proteins, such as cytokines, inflammatory enzymes, adhesion molecules, and inflammatory receptors. S. Wissink et al., Mol. Endocrinol., Vol. 12, No. 3, 354-363 (1998);

P.J. Barnes and M. Karin, New Engl. J. Med., Vol. 336, 1066-1077 (1997). Thus, both the transrepression and transactivation functions of GCs directed to different genes produce the beneficial effect of inflammatory inhibition. On the other hand, steroid-induced diabetes and glaucoma appear to be produced by the transactivation action of GCs on genes responsible for these diseases. H. Schäcke et al., Pharmacol. Ther., Vol. 96, 23-43 (2002). Thus, while the transactivation of certain genes by GCs produces beneficial effects, the transactivation of other genes by the same GCs can produce undesired side effects, one of which is glaucoma. Therefore, GCs would not be employed to treat or prevent glaucoma or its progression. Consequently, it is very desirable to provide pharmaceutical compounds and compositions that produce differentiated levels of transactivation and transrepression activity on GC-responsive genes to treat or prevent glaucoma or its progression.

The present invention provides a composition comprising a dissociated glucocorticoid receptor agonist ("DIGRA"), a pharmaceutically acceptable salt thereof, or a pharmaceutically acceptable ester thereof for treating or preventing glaucoma or progression thereof in a subject, wherein the DIGRA has Formula I

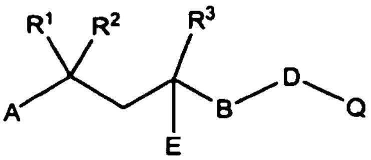

(I)

wherein A is a dihydrobenzofuranyl group substituted with a halogen atom; Q is a quinolinyl of isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; E is the hydroxy group, $R^3$ is a trifluoromethyl group, and wherein said glaucoma results from chronic inflammation.

According to another aspect, a composition is provided comprising a dissociated glucocorticoid receptor agonist ("DIGRA"), a pharmaceutically acceptable salt thereof, or a pharmaceutically acceptable ester thereof for treating or preventing glaucoma or progression thereof in a subject, wherein the DIGRA has Formula II

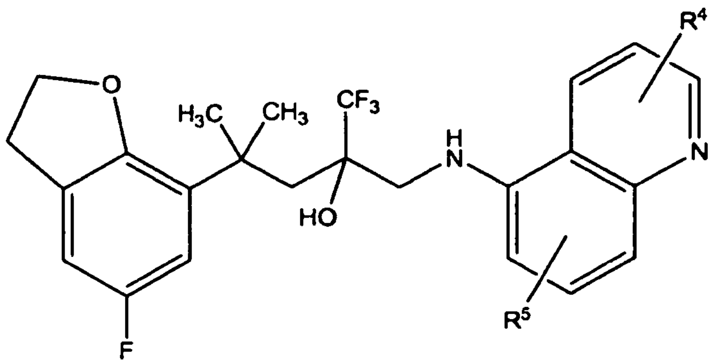

(II)

or Formula III

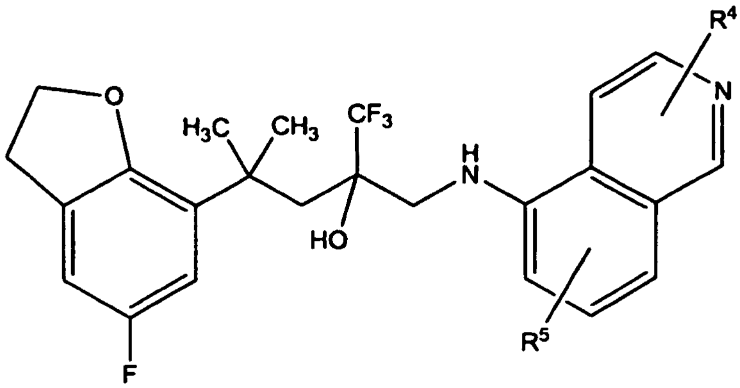

(III)

wherein $R^4$ and $R^5$ are independently selected from the group consisting of hydrogen, halogen, cyano, hydroxy, $C_1$-$C_{10}$

alkoxy groups, unsubstituted $C_1$-$C_{10}$ linear or branched alkyl groups, substituted $C_1$-$C_{10}$ linear or branched alkyl groups, unsubstituted $C_3$-$C_{10}$ cyclic alkyl groups, and substituted $C_3$-$C_{10}$ cyclic alkyl groups, wherein said glaucoma results from chronic inflammation.

According to still another aspect, the use of a DIGRA, a pharmaceutically acceptable salt thereof, or a pharmaceutically acceptable ester thereof for the preparation of a medicament for treating or preventing glaucoma or progression thereof is provided, wherein the DIGRA has Formula I

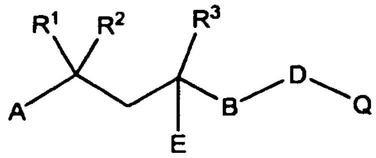

(I)

wherein A is a dihydrobenzofuranyl group substituted with a halogen atom; Q is a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; and E is the hydroxy group, $R^3$ is a trifluoromethyl group, and wherein said glaucoma results from chronic inflammation.

In still another aspect, the compounds or compositions comprise: (a) a DIGRA, a pharmaceutically acceptable salt thereof, or a pharmaceutically acceptable ester thereof as defined above; and (b) an anti-inflammatory agent other than said DIGRA, said pharmaceutically acceptable salt thereof, and said pharmaceutically acceptable ester thereof. Examples of such anti-inflammatory agents are disclosed herein below.

In still another aspect, said at least a DIGRA has Formula I.

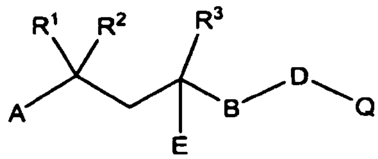

(I)

wherein A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups (preferably, $C_1$-$C_3$ alkyl groups); B is a $C_1$-$C_3$ alkylene group; D is the -NH- group; E is the hydroxy group; and $R^3$ comprises a trifluoromethyl group.

In a further embodiment, said at least a DIGRA has Formula II or III.

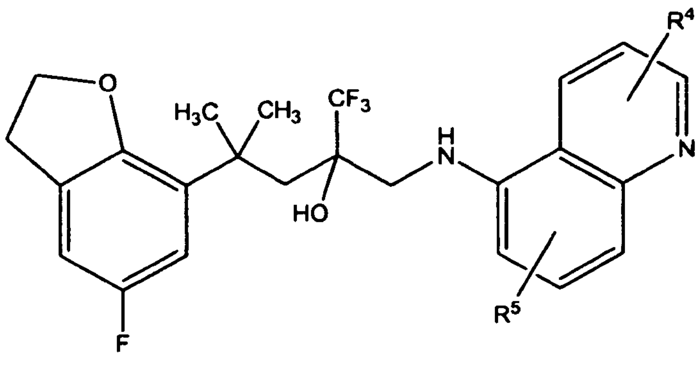

(II)

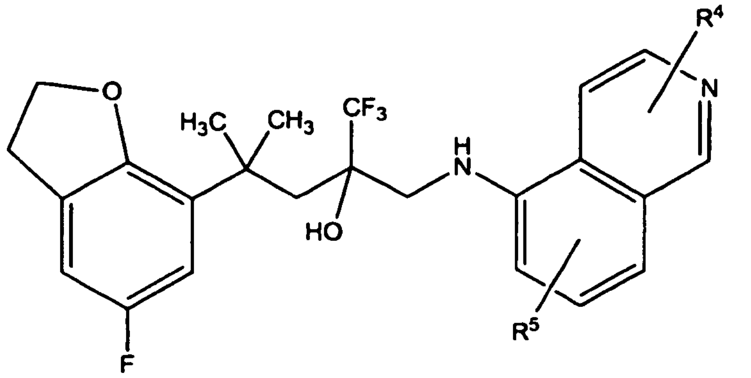

(III)

wherein $R^4$ and $R^5$ are independently selected from the group consisting of hydrogen, halogen, cyano, hydroxy, $C_1$-$C_{10}$ (alternatively, $C_1$-$C_5$ or $C_1$-$C_3$) alkoxy groups, unsubstituted $C_1$-$C_{10}$ (alternatively, $C_1$-$C_5$ or $C_1$-$C_3$) linear or branched alkyl groups, substituted $C_1$-$C_{10}$ (alternatively, $C_1$-$C_5$ or $C_1$-$C_3$) linear or branched alkyl groups, unsubstituted $C_1$-$C_{10}$ (alternatively, $C_3$-$C_6$ or $C_3$-$C_5$) cyclic alkyl groups, and substituted $C_3$-$C_{10}$ (alternatively, $C_3$-$C_6$ or $C_3$-$C_5$) cyclic alkyl groups.

In still another embodiment, said at least a DIGRA has Formula IV.

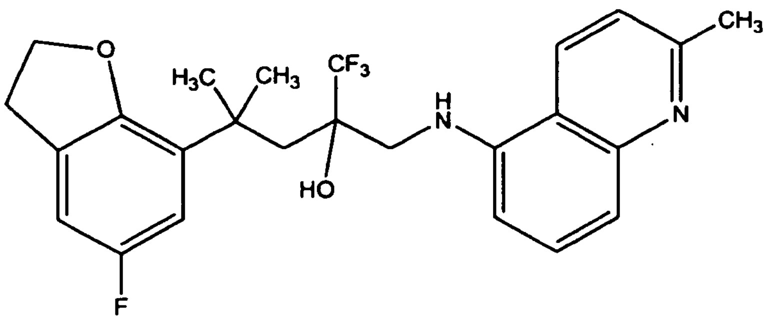

(IV)

Methods for preparing compounds of Formula I, II, III, or IV are disclosed, for example, in U.S. Patents 6,897,224; 6,903,215; 6,960,581. Still other methods for preparing such compounds also can be found in U.S. Patent Application Publication 2006/0116396, or PCT Patent Application WO 2006/050998 A1.

Examples of compounds having Formula I include 5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-1-methylisoquinoline, 5-[4-(5-fluoro-2,3-dihydrobenzofuran-7-yl)-2-hydroxy-4-methyl-2-trifluoromethyl-pentylamino]isoquinol-1(2H)-one, 5-[4-(5-fluoro-2,3-dihydrobenzofuran-7-yl)-2-hydroxy-4-methyl-2-trifluoromethyl-pentylamino]-2,6-dimethylquinoline, 5-[4-(5-fluoro-2,3-dihydrobenzofuran-7-yl)-2-hydroxy-4-methyl-2-trifluoromethyl-pentylamino]-6-chloro-2-methylquinoline, 5-[4-(5-fluoro-2,3-dihydrobenzofuran-7-yl)-2-hydroxy-4-methyl-2-trifluoromethyl-pentylamino]isoquinoline, 5-[4-(5-fluoro-2,3-dihydrobenzofuran-7-yl)-2-hydroxy-4-methyl-2-trifluoromethyl-pentylamino]quinoline, 5-[4-(2,3-dihydro-5-fluoro-7-benzofuranyl)-2-hydroxy-4-methyl-2-trifluoromethyl-pentylamino]quinolin-2[1H]-one, 6-fluro-5-[4-(5-fluoro-2,3-dihydrobenzofuran-7-yl)-2-hydroxy-4-methyl-2-trifluoromethyl-pentylamino]-2-methylquinoline, 8-fluoro-,5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-2-methylisoquinol-1-[2h]-one, and enantiomers thereof.

The following examples of DIGRAs are not part of the invention but reflect the generell technical knowledge:

For example, a DIGRA can have Formula I, wherein

(a) A is an aryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$

alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an azaindolyl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, halogen, hydroxy, oxo, cyano, amino, and trifluoromethyl.

Examples of these compounds include 1,1,1-trifluoro-4-(5-fluoro-2-methoxyphenyl)-4-methyl-2-(1H-pyrrolo [2,3-c] pyridin-2-ylmethyl)pentan-2-ol; 1,1,1-trifluoro-4-(5-fluoro-2-methoxyphenyl)-4-methyl-2-(1H-pyrrolo[3,2-c] pyridin-2-ylmethyl)pentan-2-ol; 1,1,1-trifluoro-4-methyl-4-phenyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[2,3-c] pyridin-2-ylmethyl)pentan-2-ol; 1,1,1-trifluoro4-methyl-4-phenyl-2-(1H-pyrrolo[3,2-c]pyridin-2-ylmethyl)pentan-2-ol; 1,1,1-trifluoro-4-(4-fluoro-2-methoxyphenyl)-4-methyl-2-(1H-pyrrolo[3,2-c] pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methoxyphenyl)-4-methyl-2-(1H-pyrrolo[3,2-c] pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; and 4-fluoro-2-[4,4,4-trifluoro-3-hydroxy-1,1-dimethyl-3-(1H-pyrrolo[2,3-c]pyridin-2-ylmethyl)butyl]phenol.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, C1-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) B is the methylene or carbonyl group;

(d) $R^3$ is a carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups;

(e) D is the -NH- group;

(f) E is the hydroxy group; and

(g) Q comprises a methylated benzoxazinone.

Examples of these compounds include 2-benzyl-4-(5-fluoro-2-methoxyphenyl)-2-hydroxy-4-methylpentanoic acid(4-methyl-1 -oxo-1H-benzo[d][1,2]oxazin-6-yl)amide; 2-benzyl-4-(5-fluoro-2-hydroxyphenyl)-2-hydroxy-4-methylpentanoic acid(4-methyl-1 -oxo-1H-benzo[d][1,2]oxazin-6-yl)amide; 2-cyclohexylmethyl-4-(5-fluoro-2-methoxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-hydroxyphenyl)-2-hydroxy-4-methylpentanoic acid(4-methyl-1-oxo-1H-benzo[d][1,2]oxazin-6-yl)amide; 2-benzyl-2-hydroxy-4-methyl-4-methylpentanoic acid(4-methyl-1-oxo-1H-benzo[d][1,2]oxazin-6-yl)amide; and 2-cyclohexylmethyl-2-hydroxy-4-methylpentanoic acid(4-methyl-1-oxo-1H-benzo[d][1,2]oxazin-6-yl)amide.

For example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an aryl or heteroaryl group one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, and trifluoromethyl.

Examples of these compounds include 2-(3,5-difluorobenzyl)-1,1,1-trifluoro-4-(5-fluoro-2-methoxyphenyl)-4-methylpentan-2-ol; 2-biphenyl-4-ylmethyl-1,1,1-trifluoro-4-(5-fluoro-2-methoxyphenyl)-4-methylpentan-2-ol; 2-(3,5-dimethylbenzyl)-1,1,1-trifluoro-4-(5-fluoro-2-methoxyphenyl)-4-methylpentan-2-ol; 2-(3-bromobenzyl)-1,1,1-trifluoro-4-(5-fluoro-2-methoxyphenyl)-4-methylpentan-2-ol; 2-(3,5-dichlorobenzyl)-1,1,1 -trifluoro-4-(5-fluoro-2-methoxyphenyl)-4-methylpentan-2-ol; 2-(3,5-bis-trifluoromethylbenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(3-fluoro-5-trifluoromethylbenzyl)-4-methylpentan-2-ol; 2-(3-chloro-2-fluoro-5-trifluoromethylbenzyl- )-1,1,1 -trifluoro-4-(5-fluoro-2-methoxyphenyl)-4-methylpentan-2-ol; 4-[4-(5-fluoro-2-methoxyphenyl)-2-hydroxy-4-methyl-2-trifluoromethylpentyl]benzonitrile; 2-(3,5-dibromobenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(2-fluoro-3-trifluoromethylbenzyl)-4-methylpentan-2-ol; 1,1,1-trifluoro-4-(5-fluoro-2-methoxyphenyl)-2-(2-fluoro-5-trifluoromethylbenzyl)-4-methylpentan-2-ol.

For example, a DIGRA can have Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_5$-$C_{15}$ arylalkyl, or $R^1$ and $R^2$ together with the carbon atom they are commonly attached to form a $C_3$-$C_8$ spiro cycloalkyl ring;

(c) $R^3$ is the trifluoromethyl group;

(d) B is the carbonyl group or methylene group, which is optionally independently substituted with one or two substituent groups selected from $C_1$-$C_5$ alkyl, hydroxy, and halogen;

(e) D is absent;

(f) E is the hydroxy group or amino group wherein the nitrogen atom is optionally independently mono- or di-substituted by $C_1$-$C_5$ alkyl; and

(g) Q comprises a pyrrolidine, morpholine, thiomorpholine, piperazine, piperidine, 1H-pyridin-4-one, 1H-pyridin-2-one, 1H-pyridin-4-ylideneamine, 1H-quinolin-4-ylideneamine, pyran, tetrahydropyran, 1,4-diazepane, 2,5-diazabicyclo[2.2.1]heptane, 2,3,4,5-tetrahydrobenzo[b][1,4]diazepine, dihydroquinoline, tetrahydroquinoline, 5,6,7,8-tetrahydro-1H-quinolin-4-one, tetrahydroisoquinoline, decahydroisoquinoline, 2,3-dihydro-1H-isoindole, 2,3-dihydro-1H-indole, chroman, 1,2,3,4-tetrahydroquinoxaline, 1,2-dihydroindazol-3-one, 3,4-dihydro-2H-benzo[1,4]oxazine, 4H-benzo[1,4]thiazine, 3,4-dihydro-2H-benzo[1,4]thiazine, 1,2-dihydrobenzo[d] [1,3]oxazin4-one, 3,4-dihydrobenzo [1,4]oxazin4-one, 3H-quinazolin4-one, 3,4-dihydro-1H-quinoxalin-2-one, 1H-quinnolin-4-one, 1H-quinazolin4-one, 1H-[1,5]naphthyridin-4-one, 5,6,7,8-tetrahydro- H-[1,-5]naphthyridin-4-one, 2,3-dihydro-1H-[1,5]naphthyridin-4-one, 1,2-dihydropyrido[3,2-d][1,3]oxazin-4-one, pyrrolo[3,4-c]pyridine-1,3-dione, 1,2-dihydropyrrolo[3,4-c]pyridin-3-one, or tetrahydro[b][1,4]diazepinone group, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, or ureido wherein either nitrogen atom is optionally independently substituted with $C_1$-$C_5$ alkyl.

Examples of these compounds include 2-(2,6-dimethylmorpholin-4-ylmethyl)-1,1,1-trifluoro-4-(5-fluoro-2-methoxyphenyl)-4-methylpentan-2-ol; 1-[4-(5-fluoro-2-methoxyphenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-3,5-dimethylpiperidin-4-one; 1-[4-(5-fluoro-2-methoxyphenyl)-2-hydroxy-4-methyl-2-trifluoromethylpentyl]-3-methyl-1H-quinolin-4-one; 1-[4-(5-fluoro-2-methoxyphenyl)-2-hydroxy-4-methyl-2-trifluoromethylpentyl]-2,3-dihydro-1H-quinolin-4-one; 1-[4-(4-fluorophenyl)-2-hydroxy-4-methyl-2-trifluoromethylpentyl]-1 H-quinolin-4-one; 1-[4-(3-fluorophenyl)-2-hydroxy-4-methyl-2-trifluoromethylpentyl]-1H-quinolin-4-one; 1-[4-(4-fluoro-2-hydroxyphenyl)-2-hydroxy-4-methyl-2-trifluoromethylpentyl]-1H-quinolin-4-one; 1-[4-phenyl-2-hydroxy-4-methyl- -2-trifluoromethylpentyl]-1H-quinolin-4-one; 1-[4-(5-fluoro-2,3-dihydrobenzofuran-7-yl)-2-hydroxy-4-methyl-2-trifluoromethylpentyl]-1H-quinolin-4-one; 1-[4-(5-bromo-2,3-dihydrobenzofuran-7-yl)-2-hydroxy-4-methyl-2-trifluoromethylpentyl]-1H-quinolin-4-one; 1-[4-(5-methyl-2,3-dihydrobenzofuran-7-yl)-2-hydroxy-4-methyl-2-trifluoromethylpentyl]-1H-quinolin-4-one; 1-[4-(5-chloro-2,3-dihydrobenzofuran-7-yl)-2-hydroxy-4-methyl-2-trifluoromethylpentyl]-1H-quinolin-4-one; 1-[4-(2,3-dihydrobenzofuran-7-yl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1H-[1,5]naphthyridin-4-one; 1-[4-(5-fluoro-2-methoxyphenyl)-2-hydroxy-2,4-dimethylpentyl]-3,5-dimethyl-1H-pyridin-4-one; 1-[2-hydroxy-4-(2-methoxy-5-thiophen-2-ylphenyl)-4-methyl-2-trifluoromethylpentyl]-1H-quinolin-4-one; 1-[4-(6-bromobenzo[1,3]dioxol-4-yl)-2-hydroxy-4-methyl-2-trifluoromethylpentyl]-1 H-quinolin-4-one; 1-[4-(5-fluoro-2-hydroxyphenyl)-2-hydroxy-4-methyl-2-trifluoromethylpentyl]-3-methyl-1H-quinolin-4-one; 1-[2-hydroxy-4-(4-hydroxybiphenyl-3-yl)-4-methyl-2-trifluoromethylpentyl]-1H-quinolin-4-one; 1-{4-[5-(3,5-dimethylisoxazol-4-yl)-2-hydroxyphenyl]-2-hydroxy-4-methyl-2-trifluoromethylpentyl}-1H-quinolin-4-one; 1-[2-hydroxy-4-(2-hydroxy-5-thiophen-3-ylphenyl)-4-methyl-2-trifluoromethylpentyl]-1H-quinolin-4-one; 1-{4-[5-(3,5-dimethylisoxazol-4-yl)-2-methoxyphenyl]-2-hydroxy-4-methyl-2-trifluoromethylpentyl}-1H-quinolin-4-one; 1-[2-hydroxy-4-methyl-4-(3-pyridin-3-ylphenyl)-2-trifluoromethylpentyl]-1H-quinolin-4-one; 4-methoxy-3-[4,4,4-trifluoro-3-hydroxy-1,1-dimethyl-3-(4-oxo-4H-quinolin-1-ylmethyl)butyl]benzaldehyde; 1-[2-hydroxy-4-(2-methoxy-5-thiophen-3-ylphenyl)-4-methyl-2-trifluoromethylpentyl]-1 H-quinolin-4-one; 1-[4-(5-furan-3-yl-2-methoxyphenyl)-2-hydroxy-4-methyl-2-trifluoromethylpentyl]-1H-quinolin-4-one; 1-[2-hydroxy-4-(4-methoxybiphenyl-3-yl)-4-methyl-2-trifluoromethylpentyl]-1H-quinolin-4-one; 1-[4-(5-acetyl-2-hydroxyphenyl)-2-hydroxy-4-methyl-2-trifluoromethylpentyl]-1H-quinolin-4-one; 1-[3,3,3-trifluoro-2-(6-fluoro-4-methylchroman-4-ylmethyl)-2-hydroxypropyl]-1H-quinolin-4-one; 1-(4-{3-[1-(benzyloxyimino)ethyl]phenyl}-2-hydroxy-4-methyl-2-trifluoromethylpentyl)-1H-quinolin-4-one; 1-[4-(5-acetyl-2-methoxyphenyl)-2-hydroxy-4-methyl-2-trifluoromethylpentyl]-1H-quinolin-4-one; 1-(2-hydroxy-4-{3-[1-(methoxyimino)ethyl]phenyl}-4-methyl-2-trifluoromethylpentyl)-1H-quinolin-4-one; 1-[4-(5-bromo-2-hydroxyphenyl)-2-hydroxy-4-methyl-2-trifluoromethylpentyl]-1 H-quinolin-4-one; 1-(2-hydroxy-4-{3-[1-(hydroxyimino) ethyl]phenyl}-4-methyl-2-trifluoromethylpentyl)-1H-quinolin-4-one; 1-[4-(5-bromo-2-methoxyphenyl)-2-hydroxy-4-methyl-2-trifluoromethylpentyl]-1H-quinolin-4-one; 1-[4-(3,5-difluorophenyl)-2-hydroxy-4-methyl-2-trifluoromethylpentyl]-1H-quinolin-4-one; 1-[4-(3,5-dimethylphenyl)-2-hydroxy-4-methyl-2-trifluoromethylpentyl]-1H-quinolin-4-one; 1-{2-hydroxy-4-methyl-4-[3-(2-methyl-[1,3]dioxolan-2-yl)phenyl]-2-trifluoromethylpentyl}-1H-quinolin-4-one; 1-[4-(2,3-dihydrobenzofuran-7-yl)-2-hydroxy-4-methyl-2-trifluoromethylpentyl]-1H-[1,5]naphthyridin-4-one; 1-[4-(3-[1,3]dioxan-2-ylphenyl)-2-hydroxy-4-methyl-2-trifluoromethylpentyl]-1H-quinolin-4-one; 1-{4-[3-(3,5-dimethylisoxazol-4-yl)phenyl]-2-hydroxy-4-methyl-2-trifluoromethylpentyl}-1 H-quinolin-4-one; 1-[4-(2,3-dihydrobenzofuran-7-yl)-2-hydroxy-4-methyl-2-trifluoromethylpentyl]-3,5-dimethyl-1H-pyridin-4-one; 1-[4-(5-fluoro-2-methoxyphenyl)-2-hydroxy-4-methyl-2-trifluoromethylpentyl]-2-hydroxymethyl-3,5-dimethyl-1H-pyridin-4-one; 1-[4-(5-fluoro-2-hydroxyphenyl)-2-hydroxy-4-methyl-2-trifluoromethylpentyl]-3-hydroxymethyl-1H-quinolin-4-one; 1-[4-(3-bromophenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-6-methyl-1H-quinolin-4-one; 6-chloro-1-[4-(5-fluoro-2-hydroxyphenyl)-2-hydroxy-4-methyl-2-trifluoromethylpentyl]-1H-quinolin-4-one; 1-[-4-(2-difluoromethoxy-5-fluorophenyl)-2-hydroxy-4-methyl-2-trifluoromethylpentyl]-1H-quinolin-4-one; 1-(4-biphenyl-3-yl-2-hydroxy-4-methyl-2-trifluoromethylpentyl)-1H-quinolin-4-one; 1-[2-hydroxy-4-(2-hydroxy-5-methylphenyl)-4-methyl-2-trifluoromethylpentyl]-1H-quinolin-4-one; 1-[2-hydroxy-4-(3-isopropoxyphenyl)-4-methyl-2-trifluoromethylpentyl]-1H-quinolin-4-one; 1-[4-(3-ethoxyphenyl)-2-hydroxy-4-methyl-2-trifluoromethylpentyl]-1H-quinolin-4-one; 1-[2-hydroxy-4-(2-methoxy-5-methylphenyl)-4-methyl-2-trifluoromethylpentyl]-1H-quinolin-4-one; 1-[4-(2,5-dimethylphenyl)-2-hydroxy-4-methyl-2-trifluoromethylpentyl]-1H-quinolin-4-one; 1-[2-hydroxy-4-(3-methoxyphenyl)-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1 ,2-dihydroindazol-3-one; 7-fluoro-1-[4-(5-fluoro-2-hydroxyphenyl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3,5-dimethyl-1H-pyridin-4-one; 7-fluoro-1-[4-(5-fluoro-2-methoxyphenyl)-2-hydroxy-4-methyl-2-trifluoromethylpentyl]-1H-quinolin-4-one; 1-(2-hydroxy-4-methyl-4-phenyl-2-trifluoromethylhexyl)-1H-quinolin-4-one; 1-[4-(4-fluoro-2-methylphenyl)-2-hydroxy-4-methyl-2-trifluoromethylpentyl]-H-quinolin-4-one; 1-[4-(3,4-dimethylphenyl)-2-hydroxy-4-methyl-2-trifluoromethylpentyl]-1H-quinolin-4-one; 8-fluoro-1-[4-(5-fluoro-2-hydroxyphenyl)-2-hydroxy-4-methyl-2-trifluoromethylpentyl]-1H-quinolin-4-one; 6-fluoro-1-[4-(5-fluoro-2-hydroxyphenyl)-2-hydroxy-4-methyl-2-trifluoromethylpentyl]-1H-quinolin-4-one; 7-chloro-1-[4-(5-fluoro-2-hydroxyphenyl)-2-hydroxy-4-methyl-2-trifluoromethylpentyl]-1H-quinolin-4-one; 1-[4-(5-fluoro-2-isopropoxyphenyl)-2-hydroxy-4-methyl-2-trifluoromethylpentyl]-H-quinolin-4-one; 1-[4-(2-ethoxy-5-fluorophenyl)-2-hydroxy-4-methyl-2-trifluoromethylpentyl]-1H-quinolin-4-one; 8-fluoro-1-[4-(5-fluoro-2-methoxyphenyl)-2-hydroxy-4-methyl-2-trifluoromethylpentyl]-1H-quinolin-4-one; 6-fluoro-1-[4-(5-fluoro-2-methoxyphenyl)-2-hydroxy-4-methyl-2-trifluoromethylpentyl]-1H-quinolin-4-one; 1-[2-hydroxy-4-(5-methanesulfonyl-2,3-dihydrobenzofuran-7-yl)-4-methyl-2-trifluoromethylpentyl]-1H-quinolin-4-one; 1-[2-hydroxy-4-methyl-4-(5-methylsulfanyl-2,3-dihydrobenzofuran-7-yl)-2-trifluorcmethylpentyl]-1H-quinolin-4-one; 7-chloro-1-[4-(5-fluoro-2-methoxyphenyl)-2-hydroxy-4-methyl-2-trifluoromethylpentyl]-1H-quinolin-4-one; 3-chloro-1-[4-(5-fluoro-2-methoxyphenyl)-2-hydroxy-4-methyl-2-trifluoromethylpentyl]-5-trifluoromethyl-1H-pyridin-2-one; 1-[2-hydroxy-4-(5-methanesulfonyl-2,3-dihydrobenzofuran-7-yl)-4-methyl-2-trifluoromethylpentyl]-3-methyl-1H-quinolin-4-one; 1-[2-hydroxy-4-(2-methoxy-5-pyridin-3-ylphenyl)-4-methyl-2-trifluoromethylpentyl]-1H-quinolin-4-one; 1-[2-hydroxy-4-(2-hydroxy-3,5-dimethylphenyl)-4-methyl-2-trifluoromethylpentyl]-H-quinolin-4-one; 1-[4-(3-[1,3]dioxan-2-yl-4-fluorophenyl)-2-hydroxy-4-methyl-2-trifluoromethylpentyl]-1H-quinolin-4-one; 2-(1,1-dioxo-2,3-dihydro-1H-1λ$^6$-benzo[1,4]thiazin-4-ylmethyl)-1,1,1-trifluoro-4-(5-fluoro-2-methoxyphenyl)-4-methylpentan-2-ol; 2-(2,3-dihydrobenzo[1,4]oxazin4-ylmethyl)-1,1,1-trifluoro-4-(5-fluoro-2-methoxyphenyl)-4-methylpentan-2-ol; 1-[4-(5-fluoro-2-hydroxyphenyl)-2-hydroxy-4-methyl-2-trifluoromethylpentyl]-H-quinolin-4-one; 1-[4-(5-fluoro-2-hydroxyphenyl)-2-hydroxy-4-methyl-2-trifluoromethylpentyl]-H-[1,5]naphthyridin-4-one; 1-[4-(5-fluoro-2-methylphenyl)-2-hydroxy-4-methyl-2-trifluoromethylpentyl]-H-quinolin-4-one; 1-[4-(2,4-dimethylphenyl)-2-hydroxy-4-methyl-2-trifluoromethylpentyl]-1H-quinolin-4-one; 1-[4-(4-fluoro-2-methoxyphenyl)-2-hydroxy-4-methyl-2-trifluoromethylpentyl]-H-quinolin-4-one; 1-[4-(3-fluoro-4-methoxyphenyl)-2-hydroxy-4-methyl-2-trifluoromethylpentyl]-1H-quinolin-4-one; 1-(4-benzo[1,3]dioxol-4-yl-2-hydroxy-4-methyl-2-trifluoromethylpentyl)-1H-quinolin-4-one; 1-[4-(5-fluoro-2-methoxyphenyl)-2-hydroxy-4-methyl-2-trifluoromethylpentyl]-1,2-dihydroindazol-3-one; 1,1,1-trifluoro-4-(5-fluoro-2-methoxyphenyl)-4-methyl-2-(1-oxo-2,3-dihydro-1H-1λ$^4$-benzo[1,4-]thiazin-4-ylmethyl)pentan-2-ol; 1-[4-(5-fluoro-2-methoxyphenyl)-2-hydroxy-4-methyl-2-trifluoromethylpentyl]-2-hydroxymethyl-3,5-dimethyl-1H-pyridin--4-one; 1-[4-(2,3-dihydrobenzofuran-7-yl)-2-hydroxy-4-methyl-2-trifluoromethylpentyl]-3-methyl-1H-quinolin-4-one; 1-[2-hydroxy-4-(2-methoxy-3,5-dimethylphenyl)-4-methyl-2-trifluoromethylpentyl]-1H-quinolin-4-one; 1-[2-hydroxy-4-(2-hydroxy-5-pyridin-3-ylphenyl)-4-methyl-2-trifluoromethylpentyl]-1H-quinolin-4-one; and 1-[2-hydroxy-4-(2-hydroxy-5-pyridin-5-ylphenyl)-4-methyl-2-trifluoromethylpentyl]-1H-quinolin-4-one.

For example, a DIGRA can have Formula I, wherein A, $R^1$, $R^2$ B, D, E, and Q have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

For example, a DIGRA can have Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is the carbonyl group;

(e) D is the -NH- group;

(f) E is the hydroxy group; and

(g) Q comprises an optionally substituted phenyl group having the formula

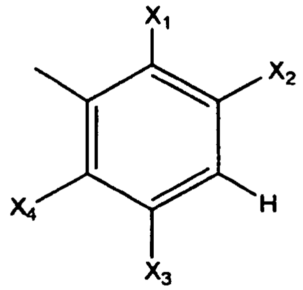

wherein $X_1$, $X_2$, $X_3$ and $X_4$ are each independently selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl, and amino wherein the nitrogen atom may be independently mono- or di-substituted by $C_1$-$C_5$ alkyl, and wherein said aryl group is optionally substituted by one or more hydroxy or $C_1$-$C_5$ alkoxy groups, and wherein either nitrogen atom of the urea group may be independently substituted by $C_1$-$C_5$ alkyl; or Q is an aromatic 5- to 7-membered monocyclic ring having from one to four heteroatoms in the ring independently selected from nitrogen, oxygen, and sulfur, optionally independently substituted with one to three substituent groups selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl optionally substituted by one or more hydroxy or $C_1$-$C_5$ alkoxy groups, and amino wherein the nitrogen atom may be independently mono- or di-substituted by $C_1$-$C_5$ alkyl, and wherein either nitrogen atom of the urea group may be independently substituted by $C_1$-$C_5$ alkyl.

Examples of these compounds include 4-(5-fluoro-2-hydroxy-phenyl)-2-hydroxy-4-methyl-2-trifluoromethyl-pentanoic acid (3,5-dichloro-phenyl)-amide; 4-(5-fluoro-2-hydroxy-phenyl)-2-hydroxy-4-methyl-2-trifluoromethyl-pentanoic acid (3-chloro-phenyl)-amide; 4-(5-fluoro-2-hydroxy-phenyl)-2-hydroxy-4-methyl-2-trifluoromethyl-pentanoic acid (2-chloro-phenyl)-amide; 4-(5-fluoro-2-hydroxy-phenyl)-2-hydroxy-4-methyl-2-trifluoromethyl-pentanoic acid (2,6-dichloro-pyrimidin-4-yl)-amide; 4-(5-fluoro-2-hydroxy-phenyl)-2-hydroxy-4-methyl-2-trifluoromethyl-pentanoic acid (2,6-dichloro-pyridin-4-yl)-amide; 4-(5-fluoro-2-hydroxy-phenyl)-2-hydroxy-4-methyl-2-trifluoromethyl-pentanoic acid (2,3-dichloro-phenyl)-amide; 4-(5-fluoro-2-hydroxy-phenyl)-2-hydroxy-4-methyl-2-trifluoromethyl-pentanoic acid (3,5-dimethylphenyl)-amide; 4-(5-fluoro-2-hydroxy-phenyl)-2-hydroxy-4-methyl-2-trifluoromethyl-pentanoic acid (3,5-bis-trifluoromethyl-phenyl)-amide; 4-(5-fluoro-2-hydroxy-phenyl)-2-hydroxy-4-methyl-2-trifluoromethyl-pentanoic acid (2,5-dichloro-phenyl)-amide; 4-(5-fluoro-2-hydroxy-phenyl)-2-hydroxy-4-methyl-2-trifluoromethyl-pentanoic acid (3-bromo-phenyl)-amide; 4-(5-fluoro-2-hydroxy-phenyl)-2-hydroxy-4-methyl-2-trifluoromethyl-pentanoic acid (3,5-difluoro-phenyl)-amide; 4-(5-fluoro-2-hydroxyphenyl)-2-hydroxy-4-methyl-2-trifluoromethyl-pentanoic acid (3,5-dibromo-phenyl)-amide.

In still another example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-C5 dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an azaindolyl group optionally independently substituted with one to three substituent groups, wherein each substituent group of Q is independently $C_1$-$C_5$ alkyl, $C_2$-$C_5$ alkenyl, $C_2$-$C_5$ alkynyl, **$C_3$-$C_8$** cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 1,1,1-trifluoro-4-(5-fluoro-2-methoxyphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(1H-pyrrolo[2,3-b]pyridin-2-ylmethyl)pentan-2-ol; 1,1,1-trifluoro-4-(5-fluoro-2-methoxyphenyl)-4-methyl-2-(1H-pyrrolo[3,2-c]pyridin-2-ylmethyl)pentan-2-ol; 1,1,1-trifluoro-4-(5-fluoro-2-methoxyphenyl)-4-methyl-2-(1H-pyrrolo[3,2-b]pyridin-2-ylmethyl)pentan-2-ol; 4-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-b]pyridin-2-ylmethyl)butyl]phenol; 4-fluoro-2-[4,4,4-trifluoro-3-hydroxy-1,1-dimethyl-3-(1H-pyrrolo[3,2-c]pyridin-2-ylmethyl)butyl]phenol; 4-fluoro-2-[4,4,4-trifluoro-3-hydroxy-1,1-dimethyl-3-(1H-pyrrolo[3,2-b]pyridin-2-ylmethyl)butyl]phenol; 1,1,1-trifluoro-4-(3-fluorophenyl)-4-methyl-2-(1H-pyrrolo[2,3-c]pyridin-2-ylmethyl)pentan-2-ol; 1,1,1-trifluoro-4-(4-fluorophenyl)-4-methyl-2-(1H-pyrrolo[2,3-c]pyridin-2-ylmethyl)pentan-2-ol; 4-(2,3-dihydrobenzofuran-7-yl)-1,1,1-trifluoro-4-methyl-2-(1H-pyrrolo[2,3-c]pyridin-2-yelmethyl)pentan-2-ol; 4-(2,3-dihydrobenzofuran-7-yl)-1,1,1-trifluoro-4-methyl-2-(1H-pyrrolo[3,2-c]pyridin-2-yelmethyl)pentan-2-ol; 1,1,1-trifluoro-4-methyl-4-phenyl-2-(1H-pyrrolo[2,3-c]pyridine-2-ylmethyl)pentan-2-ol; 1,1,1-trifluoro-4-(4-fluoro-2-methoxyphenyl)-4-methyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[3,2-c]pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluorophenyl)-4-methyl-2-(1H-pyrrolo[3,2-c]pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methylphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; 4-fluoro-2-[4,4,4-trifluoro-3-hydroxy-1,1-dimethyl-3-(3-methyl-1H-pyrrolo[2,3-c]pyridin-2-ylmethyl)butyl]phenol; 5-fluoro-2-[4,4,4-trifluoro-3-hydroxy-1,1-dimethyl-3-(1H-pyrrolo[3,2-c]pyridin-2-ylmethyl)butyl]pentan-2-ol; 1,1,1-trifluoro-4-(5-fluoro-2,3-dihydrobenzofuran-7-yl)-4-methyl-2-(1H-pyrrolo[2,3-c]pyridine-2-ylmethyl)pentan-2-ol; 4-fluoro-2-[4,4,4-trifluoro-3-hydroxy-1,1-dimethyl-3-(1H-pyrrolo[2,3-c]-[3-methylpyridin]-2-ylmethyl)butyl]phenol; 4-fluoro-2-[4,4,4-trifluoro-3-hydroxy-1,1-dimethyl-3-(1H-pyrrolo[2,3-c]-[2-fluoropyridin]-2-ylmethyl)butyl]phenol; and 4-fluoro-2-[4,4,4-trifluoro-3-hydroxy-1,1-dimethyl-3-(1H-pyrrolo[2,3-c]-[2-trifluoromethylpyridin]-2-ylmethyl)butyl]phenol.

In still another example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy,

acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 4-cyclohexyl-1,1,1-trifluoro-4-methyl-2-quinolin-4-ylmethylpentan-2-ol; 4-pyrimidin-5-yl-2-[4,4,4-trifluoro-3-hydroxy-1,1-dimethyl-3-(1H-pyrrolo[2,3-c]pyridin-2-ylmethyl)butyl]phenol; 4-pyrimidin-5-yl-2-[4,4,4-trifluoro-3-hydroxy-1,1-dimethyl-3-(1H-pyrrolo[3,2-c]pyridin-2-ylmethyl)butyl]phenol; 1,1,1-trifluoro-4-(5-fluoro-2-methoxyphenyl)-4-methyl-2-(3-methyl-1H-pyrrolo[3,2-c]pyridin-2-ylmethyl)pentan-2-ol; 1,1,1-trifluoro-4-(5-fluoro-2,3-dihydrobenzofuran-7-yl)-4-methyl-2-(1H-pyrrolo[3,2-c]pyridin-2-ylmethyl)pentan-2-ol; 1,1,1-trifluoro-4-(5-fluoro-2-methylphenyl)-4-methyl-2-(3-methyl-1H-pyrrolo[2,3-c]pyridin-2-ylmethyl)pentan-2-ol; 2-(4,6-dimethyl-1H-pyrrolo[3,2-c]pyridin-2-ylmethyl)-1,1,1-trifluoro-4-(5-fluoro-2-methoxyphenyl)-4-methylpentan-2-ol; 2-(5,7-dimethyl-1H-pyrrolo[2,3-c]pyridin-2-ylmethyl)-1,1,1-trifluoro-4-(5-fluoro-2-methoxyphenyl)-4-methylpentan-2-ol; 2-[4-(5-fluoro-2-methoxyphenyl)-2-hydroxy-4-methyl-2-trifluoromethylpentyl]-1H-pyrrolo[3,2-b]pyridine-5-carbonitrile; 1,1,1-trifluoro-4-(5-fluoro-2-methoxyphenyl)-4-methyl-2-(6-methyl-1H-pyrrolo[3,2-c]pyridin-2-ylmethyl)pentan-2-ol; 1,1,1-trifluoro-4-(5-fluoro-2-methoxyphenyl)-4-methyl-2-(4-methyl-1H-pyrrolo[3,2-c]pyridin-2-ylmethyl)pentan-2-ol; 2-[4-(5-fluoro-2-methoxyphenyl)-2-hydroxy-4-methyl-2-trifluoromethylpentyl]-4-methyl-1H-pyrrolo[3,2-c]pyridine-6-carbonitrile; 2-[4-(5-fluoro-2-methoxyphenyl)-2-hydroxy-4-methyl-2-trifluoromethylpentyl]-1H-pyrrolo[2,3-c]pyridine-5-carbonitrile; 2-[4-(5-fluoro-2-methoxyphenyl)-2-hydroxy-4-methyl-2-trifluoromethylpentyl]-1H-pyrrolo[3,2-c]pyridine-4-carbonitrile; 1,1,1-trifluoro-4-(5-fluoro-2-methoxyphenyl)-4-methyl-2-(5H-pyrrolo[3,2-d]pyrimidin-6-ylmethyl)pentan-2-ol; 1,1,1-trifluoro-4-(5-fluoro-2-methoxyphenyl)-4-methyl-2-thieno[2,3-d]pyridazin-2-ylmethylpentan-2-ol; 1,1,1-trifluoro-4-(5-fluoro-2-methoxyphenyl)-4-methyl-2-(5H-pyrrolo[3,2-c]pyridazin-6-ylmethyl)pentan-2-ol; 1,1,1-trifluoro-4-(5-fluoro-2-methoxyphenyl)-4-methyl-2-(2-methyl-5H-pyrrolo[3,2-d]pyrimidin-6-ylmethyl)pentan-2-ol; 1,1,1-trifluoro-4-(5-fluoro-2-methylphenyl)-4-methyl-2-(1H-pyrrolo[2,3-d]pyridazin-2-ylmethyl)pentan-2-ol; 2-(4,6-dimethyl-H-pyrrolo[3,2-c]pyridin-2-ylmethyl)-1,1,1-trifluoro-4-(5-fluoro-2-methylphenyl)-4-methylpentan-2-ol; 4-(5-chloro-2,3-dihydrobenzofuran-7-yl)-2-(4,6-dimethyl-1H-pyrrolo[3,2-c]pyridin-2-ylmethyl)-1,1,1-trifluoro-4-methylpentan-2-ol; 2-[4-(5-fluoro-2-methylphenyl)-2-hydroxy-4-methyl-2-trifluoromethylpentyl]-1H-pyrrolo[3,2-b]pyridine-5-carbonitrile; 4-(5-chloro-2,3-dihydrobenzofuran-7-yl)-1,1,1-trifluoro-4-methyl-2-(3-methyl-1H-pyrrolo[2,3-c]pyridin-2-ylmethyl)pentan-2-ol; 1,1,1-trifluoro-4-(5-fluoro-2-methylphenyl)-4-methyl-2-(5H-pyrrolo[3,2-c]-pyridazin-6-ylmethyl)pentan-2-ol; 4-(5-chloro-2,3-dihydrobenzofuran-7-yl)-1,1,1-trifluoro-4-methyl-2-(5H-pyrrolo[3,2-c]pyridazin-6-ylmethyl)pentan-2-ol; 4-(5-chloro-2,3-dihydrobenzofuran-7-yl)-1,1,1-trifluoro-4-methyl-2-(1-H-pyrrolo[2,3-d]pyridazin-2-ylmethyl)pentan-2-ol; 1,1,1-trifluoro-4-(5-fluoro-2-methoxyphenyl)-2-(7-fluoro-1H-pyrrolo[2,3-c]pyridin-2ylmethyl)-4-methylpentan-2-ol; 1,1,1-trifluoro-4-(5-fluoro-2-methoxyphenyl)-4-methyl-2-(4-methyl-1H-pyrrolo[2,3-c]pyridin-2-ylmethyl)pentan-2-ol; 2-(5,7-dichloro-1H-pyrrolo[2,3-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-(5-trifluoromethyl-1H-pyrrolo[2,3-c]pyridin-2-ylmethyl)pentan-2-ol; 1,1,1-trifluoro-4-(5-fluoro-2-methoxyphenyl)-2-(5-methoxy-1H-pyrrolo[2,3-c]pyridin-2-ylmethyl)-4-methylpentan-2-ol; 1,1,1-trifluoro-4-(5-fluoro-2-methylphenyl)-4-methyl-2-(4-methyl-1H-pyrrolo[2,3-c]pyridin-2-ylmethyl)pentan-2-ol; 1,1,1-trifluoro-4-(5-fluoro-2-methylphenyl)-2-(5-isopropoxy-1H-pyrrolo[2,3-c]pyridin-2-ylmethyl)-4-methylpentan-2-ol; 1,1,1-trifluoro-4-(5-fluoro-2-methylphenyl)-2-(5-methoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(5-methoxy-1H-pyrrolo[2,3-c]pyridin-2-ylmethyl)-4-methylpentan-2-ol; 1,1,1-trifluoro-4-(5-fluoro-2-methylphenyl)-2-(7-fluoro-1H-pyrrolo[2,3-c]pyridin-2-ylmethyl)-4-methylpentan-2-ol; 4-(5-chloro-2,3-dihydrobenzofuran-7-yl)-1-trifluoro-4-methyl-2-(5-trifluoromethyl-1H-pyrrolo[2,3-c]pyridin-2-ylmethyl)pentan-2-ol; 1,1,1-trifluoro-4-(5-fluoro-2-methylphenyl)-4-methyl-2-(5-trifluoromethyl-1H-pyrrolo[2,3-c]pyridin-2-ylmethyl)pentan-2-ol; 4-(5-chloro-2,3-dihydrobenzofuran-7-yl)-1,1,1-trifluoro-2-(5-isopropoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(7-fluoro-1H-pyrrolo[2,3-c]pyridin-2-ylmethyl)-4-methylpentan-2-ol; 4-(5-chloro-2,3-dihydrobenzofuran-7-yl)-2-(5-dimethylamino-1H-pyrrolo[2,3-c]pyridin-2-ylmethyl)-1,1,1-trifluoro-4-methylpentan-2-ol; 4-(5-chloro-2,3-dihydrobenzofuran-7-yl)-1,1,1-trifluoro-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(5-morpholin-4-yl-1H-pyrrolo[2,3-c]pyridin-2-ylmethyl)pentan-2-ol; 1,1,1-trifluoro-4-(5-fluoro-2-methylphenyl)-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-2-(5-ethoxy-1H-pyrrolo[2,3-c]pyridin-2-ylmethyl)-1,1,1-trifluoro-4-methylpentan-2-ol; 2-(5-benzyloxy-1H-pyrrolo[2,3-c]pyridin-2-ylmethyl)-1,1,1-trifluoro-4-(5-fluoro-2-methylphenyl)-4-methylpentan-2-ol; 2-(5-benzyloxy-1H-pyrrolo[2,3-c]pyridin-2-ylmethyl)-4-(5-chloro-2,3-dihydrobenzofiran-7-yl)-1,1,1-trifluoro-4-methylpentan-2-ol; 1,1,1-trifluoro-4-(5-fluoro-2-methoxyphenyl)-2-(5-chloro-1H-pyrrolo[2,3-c-]pyridin-2-ylmethyl)-4-methylpentan-2-ol; 1,1,1-trifluoro-4-(5-fluoro-2-methoxyphenyl)-4-methyl-2-[5-(methylamino)-1H-pyrrolo[2,3-c]pyridin-2-ylmethyl]pentan-2-ol; 1,1,1-trifluoro-4-(5-fluoro-2-methoxyphenyl)-4-methyl-2-(5-amino-1H-pyrrolo [2,3-c]pyridin-2-ylmethyl)pentan-2-ol; 1,1,1-trifluoro-4-(5-fluoro-2-methylphenyl)-4-methyl-2-(6-amino-1H-pyrrol- o[2,3-c]pyridin-2-ylmethyl)pentan-2-ol; 4-(5-chloro-2,3-dihydrobenzofuran-7-yl)-1,1,1-trifluoro-2-(5-amino-1H-pyrrolo[2,3-c] pyridin-2-ylmethyl)-4-methylpentan-2-ol; 4-(5-chloro-2,3-dihydrobenzofuran-7-yl)-1,1,1-trifluoro-4-methyl-2-(5-methylamino-1H-pyrrolo[2,3-c]pyridin-2-ylmethyl)pentan-2-ol; 7-[4-(5-fluoro-2-methoxyphenyl)-2-hydroxy-4-methyl-2-trifluoromethylpentyl]-1H-pyrrolo[2,3-b]pyridin-7-ium chloride; 6-[4-(5-fluoro-2-methoxyphenyl)-2-hydroxy-4-methyl-2-trifluoromethylpentyl]-2-methyl-1H-pyrrolo[2,3-c]pyridin-6-ium chloride; 4-(5-bromo-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-methyl-4-(5-methyl-2,3-dihydrobenzofuran-7-yl)-2-(1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(1 H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-b]pyridin-1-ylmethylpentan-2-ol; 1,1,1-trifluoro-4-(5-fluoro-2-methoxyphenyl)-4-methyl-2-(6-oxy-1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-c]pyridin-1-ylmethylpentan-2-ol; 2-benzo[b]thiophen-2-ylmethyl-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-thieno[2,3-c]pyridin-2-ylmethylpentan-2-ol; 1,1,1-trifluoro-4-(5-fluoro-2-methoxyphenyl)-2-indazol-1-ylmethyl-4-methylpentan-2-ol; 1,1,1-trifluoro-4-(5-fluoro-2-methoxyphenyl)-4-methyl-2-pyrazolo[1,5-a]pyridin-2-ylmethylpentan-2-ol; 4-(5-chloro-2,3-dihydrobenzofuran-7-yl)-2,4-dimethyl-1-thieno[2,3-c]pyridin-2-ylpentan-2-ol; 4-(5-fluoro-2-methylphenyl)-2,4-dimethyl-1-thieno[2,3-c]pyridin-2-ylpentan-2-ol; 1,1,1-trifluoro-4-(5-fluoro-2-methoxyphenyl)-2-furo[2,3-c]pyridin-2-ylmethy-1-4-methylpentan-2-ol; 4-(5-chloro-2,3-dihydrobenzofuran-7-yl)-1-furo[2,3-c]pyridin-2-yl-2,4-dimethylpentan-2-ol; 4-(5-fluoro-2-methylphenyl)-1-furo-[2,3-c]pyridin-2-yl-2,4-dimethylpentan-2-ol; 1,1,1-trifluoro-4-(5-fluoro-2-methylphenyl)-4-methyl-2-(1H-pyrrolo[3,2-c]pyridin-2-ylmethyl)pentan-2-ol-; 1,1,1-trifluoro-4-methyl-4-(5-methyl-2,3-dihydrobenzofuran-7-yl)-2-(1H-pyrrolo[3,2-c]pyridin-2-ylmethyl)pentan-2-ol; 4-(5-chloro-2,3-dihydrobenzofuran-7-yl)-1,1,1-trifluoro-4-methyl-2-(1H-pyrrolo[3,2-c]pyridin-2-ylmethyl)pentan-2-ol; 4-(5-bromo-2,3-dihydrobenzofuran-7-yl)-1,1,1-trifluoro-4-methyl-2-(1H-pyrrolo[3,2-c]pyridin-2-ylmethyl)pentan-2-ol; 2-(3-dimethylaminomethyl-1H-pyrrolo[3,2-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-pyrrolo[3,2-c]pyridin-1-ylmethylpentan-2-ol; 1,1,1-trifluoro-4-(5-fluoro-2-methoxyphenyl)-4-methyl-2-pyrrolo[3,2-b]pyridin-1-ylmethylpentan-2-ol; 1,1,1-trifluoro-4-(5-fluoro-2-methoxyphenyl)-2-furo[3,2-c]pyridin-2-ylmethyl-4-methylpentan-2-ol; 4-(5-chloro-2,3-dihydrobenzofuran-7-yl)-1,1,1-trifluoro-4-methyl-2-pyrrolo[3,2-b]pyridin-1-ylmethylpentan-2-ol; 1,1,1-trifluoro-4-(5-fluoro-2-methoxyphenyl)-4-methyl-2-thieno[3,2-c]pyridin-2-ylmethylpentan-2-ol; 4-(5-chloro-2,3-dihydrobenzofuran-7-yl)-1,1,1-trifluoro-4-methyl-2-thieno[3,2-c]pyridin-2-ylmethylpentan-2-ol; 1,1,1-trifluoro-4-(5-fluoro-2-methylphenyl)-4-methyl-2-pyrrolo[3,2-b]pyridin-1-ylmethylpentan-2-ol; 1,1,1-trifluoro-4-(5-fluoro-2-methylphenyl)-4-methyl-2-thieno[3,2-c]pyridin-2-ylmethylpentan-2-ol; 4-fluoro-2-(4,4,4-trifluoro-3-hydroxy-1,1-dimethyl-3-thieno[3,2-c]pyridin-2-ylmethylbutyl)phenol;

4-fluoro-2-(4,4,4-trifluoro-3-furo[3,2-c]pyridin-2-ylmethyl-3-hydroxy-1,1-dimethylbutyl)phenol; 4-fluoro-2-(4,4,4-trifluoro-3-hydroxy-1,1-dimethyl-3-pyrrolo[3,2-b]pyridin-1-ylmethylbutyl)phenol; 2-[4-(5-fluoro-2-hydroxyphenyl)-2-hydroxy-4-methyl-2-trifluoromethylpentyl]-1H-indole-6-carboxylic acid; 2-[4-(5-fluoro-2-hydroxyphenyl)-2-hydroxy-4-methyl-2-trifluoromethylpentyl]-1H-indole-6-carboxylic acid dimethylamide; {2-[4-(5-fluoro-2-hydroxyphenyl)-2-hydroxy-4-methyl-2-trifluoromethylpentyl]-1H-indol-6-yl}morpholin-4-ylmethanone; 2-[4-(5-fluoro-2-methoxyphenyl)-2-hydroxy-4-methyl-2-trifluoromethylpentyl]-1H-indole-6-carboxylic acid dimethylamide; {2-[4-(5-fluoro-2-methoxyphenyl)-2-hydroxy-4-methyl-2-trifluoromethylpentyl]-1H-indol-6-yl}morpholin-4-ylmethanone; 2-[4-(5-fluoro-2-hydroxyphenyl)-2-hydroxy-4-methyl-2-trifluoromethylpentyl]-1H-indole-6-carboxylic acid amide; 2-[4-(5-fluoro-2-methoxyphenyl)-2-hydroxy-4-methyl-2-trifluoromethylpentyl]-1H-indole-6-carboxylic acid amide; 4-fluoro-2-[4,4,4-trifluoro-3-hydroxy-1,1-dimethyl-3-(5-nitro-1H-indol-2-ylmethyl)butyl]phenol; 2-[4-(5-fluoro-2-methoxyphenyl)-2-hydroxy-4-methyl-2-trifluoromethylpentyl]-1H-indole-6-carbonitrile; 2-[4-(5-fluoro-2-hydroxyphenyl)-2-hydroxy-4-methyl-2-trifluoromethylpentyl]-1H-indole-6-carbonitrile; N-{2-[4-(5-fluoro-2-methoxyphenyl)-2-hydroxy-4-methyl-2-trifluoromethylpentyl]-1H-indol-5-yl}acetamide; 1,1,1-trifluoro-4-(4-fluoro-2-methoxyphenyl)-2-(7-fluoro-4-methyl-1H-indo-1-2-ylmethyl)-4-methylpentan-2-ol; 5-fluoro-2-[4,4,4-trifluoro-3-(7-fluoro-4-methyl-1H-indol-2-ylmethyl)-3-hydroxy-1,1-dimethylbutyl]phenol; 2-[4-(3-[1,3]dioxolan-2-ylphenyl)-2-hydroxy-4-methyl-2-trifluoromethylpentyl]-1H-indole-5-carbonitrile; 2-[4-(5-fluoro-2-methoxyphenyl)-2-hydroxy-4-methyl-2-trifluoromethylpentyl]-1H-indole-5-carboxylic acid-2-trimethylsilanylethyl ester; 2-[4-(5-fluoro-2-methoxyphenyl)-2-hydroxy-4-methyl-2-trifluoromethylpentyl]-1H-indole-5-carboxylic acid; 2-[4-(4-fluoro-2-hydroxyphenyl)-2-hydroxy-4-methyl-2-trifluoromethylpenty-1]-4-methyl-1H-indole-6-carbonitrile; {2-[4-(5-Fluoro-2-methoxyphenyl)-2-hydroxy-4-methyl-2-trifluoromethylpentyl]-1H-indol-5-yl}piperidin-1-ylmethanone; 2-[4-(5-fluoro-2-methoxyphenyl)-2-hydroxy-4-methyl-2-trifluoromethylpentyl]-1H-indole-5-carboxylic acid methylamide; {2-[4-(5-fluoro-2-methoxyphenyl)-2-hydroxy-4-methyl-2-trifluoromethylpentyl]-1H-indol-5-yl}pyrrolidin-1-ylmethanone; 1-{2-[4-(5-fluoro-2-methoxyphenyl)-2-hydroxy-4-methyl-2-trifluoromethylpentyl]1H-indole-5-carbonyl}piperidin-4-one; 2-[4-(5-fluoro-2-methoxyphenyl)-2-hydroxy-4-methyl-2-trifluoromethylpentyl]-1H-indole-5-carboxylic acid (2-hydroxyethyl)amide; {2-[4-(5-fluoro-2-methoxyphenyl)-2-hydroxy-4-methyl-2-trifluoromethylpentyl]-1H-indol-5-yl}(4-hydroxypiperidin-1-yl)methanone; {2-[4-(5-fluoro-2-methoxyphenyl)-2-hydroxy-4-methyl-2-trifluoromethylpentyl]-1H-indol-5-yl}(3-hydroxypyrrolidin-1-yl)methanone; 2-[4-(5-fluoro-2-methoxyphenyl)-2-hydroxy-4-methyl-2-trifluoromethylpentyl]-1H-indole-5-carboxylic acid cyanomethylamide; 2-[4-(5-fluoro-2-methoxyphenyl)-2-hydroxy-4-methyl-2-trifluoromethylpentyl]-1H-indole-5-carboxylic acid (2-dimethylaminoethyl)amide; {2-[4-(5-fluoro-2-methoxyphenyl)-2-hydroxy-4-methyl-2-trifluoromethylpentyl]-1H-indol-5-yl}(4-methylpiperazin-1-yl)methanone; ({2-[4-(5-fluoro-2-methoxyphenyl)-2-hydroxy-4-methyl-2-trifluoromethylpentyl]-1H-indole-5-carbonyl}amino)acetic acid methyl ester; 2-[4-(5-fluoro-2-methoxyphenyl)-2-hydroxy-4-methyl-2-trifluoromethylpentyl]-1H-indole-5-carboxylic acid carbamoylmethylamide; 4-({2-[4-(5-fluoro-2-methoxyphenyl)-2-hydroxy-4-methyl-2-trifluoromethylpentyl]-1H-indole-5-carbonyl}amino)butyric acid methyl ester; ({2-[4-(5-fluoro-2-methoxyphenyl)-2-hydroxy-4-methyl-2-trifluoromethylpentyl]-1H-indole-5-carbonyl}amino)acetic acid; 4-({2-[4-(5-fluoro-2-methoxyphenyl)-2-hydroxy-4-methyl-2-trifluoromethylpentyl]-1H-indole-5-carbonyl}amino)butyric acid; 2-[4-(3-dimethylaminomethylphenyl)-2-hydroxy-4-methyl-2-trifluoromethylpentyl]-1H-indole-5-carbonitrile; 4-fluoro-2-[4,4,4-trifluoro-3-hydroxy-1,1-dimethyl-3-(5-trifluoromethyl-1H-indol-2-ylmethyl)butyl]phenol; 2-[4-(5-bromo-2,3-dihydrobenzofuran-7-yl)-2-hydroxy-4-methyl-2-trifluoromethylpentyl]-4-methyl-1H-indole-6-carbonitrile; 2-[2-hydroxy-4-(5-methanesulfonyl-2,3-dihydrobenzofuran-7-yl)-4-methyl-2-trifluoromethylpentyl]-4-methyl-1H-indole-6-carbonitrile; 2-[4-(5-bromo-2,3-dihydrobenzofuran-7-yl)-2-hydroxy-4-methyl-2-trifluoromethylpentyl]-1H-indole-5-carboxylic acid; 2-[4-(5-bromo-2,3-dihydrobenzofuran-7-yl)-2-hydroxy-4-methyl-2-trifluoromethylpentyl]-1H-indole-5-carboxylic acid amide; 2-[4-(5-bromo-2,3-dihydrobenzofuran-7-yl)-2-hydroxy-4-methyl-2-trifluoromethylpentyl]-1H-indole-5-carboxylic acid dimethylamide; 2-[4-(5-Bromo-2,3-dihydrobenzofuran-7-yl)-2-hydroxy-4-methyl-2-trifluoromethylpentyl]-1H-indole-5-carboxylic acid cyanomethylamide; {2-[4-(5-bromo-2,3-dihydrobenzofuran-7-yl)-2-hydroxy-4-methyl-2-trifluoromethylpentyl]-1H-indol-5-yl}pyrrolidin-1-ylmethanone; {2-[4-(5-bromo-2,3-dihydrobenzofuran-7-yl)-2-hydroxy-4-methyl-2-trifluoro-methylpentyl]-1H-indol-5-yl}morpholin-4-ylmethanone; 2-[4-(5-fluoro-2-methoxyphenyl)-2-hydroxy-4-methyl-2-trifluoromethylpentyl]-1H-indole-5-carboxylic acid amide; {2-[4-(5-fluoro-2-methoxyphenyl)-2-hydroxy-4-methyl-2-trifluoromethylpentyl]-1H-indol-5-yl}morpholin-4-ylmethanone; 2-(4-benzo[1,3]dioxol-4-yl-2-hydroxy-4-methyl-2-trifluoromethylpentyl)-4-methyl-1H-indole-6-carbonitrile; 1,1,1-trifluoro-4-methyl-4-phenyl-2-quinolin-4-ylmethylhexan-2-ol; 2-[2-hydroxy-4-methyl-4-(5-methylsulfanyl-2- ,3-dihydrobenzofuran-7-yl)-2-trifluoromethylpentyl]-1H-indole-3-carbonitrile; 7-(4,4,4-trifluoro-3-hydroxy-1,1-dimethyl-3-quinolin-4-ylmethylbutyl)-2,3-dihydrobenzofuran-5-carbonitrile; 2-[2-hydroxy-4-(5-methanesulfonyl-2,3-dihydrobenzofuran-7-yl)-4-methyl-2-trifluoromethylpentyl]-1H-indole-3-carbonitrile; 2-[2-hydroxy-4-(2-hydroxy-5-methylphenyl)-4-methyl-2-trifluoro-methylpentyl]-4-methyl-1H-indole-6-carbonitrile; 1,1,1-trifluoro-4-(5-fluoro-2,3-dihydrobenzofuran-7-yl)-4-methyl-2-(5-methylsulfanyl-1H-indol-2-ylmethyl)pentan-2-ol; 2-[2-hydroxy-4-(2-methoxy-5-methylsulfanylphenyl)-4-methyl-2-trifluoromethylpentyl]-1H-indole-3-carbonitrile; 2-[2-Hydroxy-4-(5-methanesulfonyl-2-methoxyphenyl)-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-5-sulfonic acid dimethylamide; 1,1,1-trifluoro-4-(5-fluoro-2,3-dihydrobenzofuran-7-y-1)-4-methyl-2-(5-phenyl-1H-indol-2-ylmethyl)pentan-2-ol; 2-[4-(5-tert-butyl-2-hydroxyphenyl)-2-hydroxy-4-methyl-2-trifluoromethylpentyl]-1H-indole-3-carbonitrile; 2-[2-hydroxy-4-(2-hydroxy-5-isopropylphenyl)-4-methyl-2-trifluoromethylpentyl]-1H-indole-3-carbonitrile; 2-[2-hydroxy-4-(2-hydroxy-3,5-dimethylphenyl)-4-methyl-2-trifluoromethylpentyl]-1H-indole-3-carbonitrile; 2-[2-hydroxy-4-(5-hydroxy-2,4-dimethylphenyl)-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1-methyl-1H-indole-3-carbonitrile; 2-[2-hydroxy-4-(5-isopropyl-2-methoxyphenyl)-4-methyl-2-trifluoromethylpentyl]-1H-indole-3-carbonitrile; 2-[2-hydroxy-4-(5-isopropyl-2-methoxyphenyl)-4-methyl-2-trifluoromethylpentyl]-1-methyl-1H-indole-3-carbonitrile; 2-[2-hydroxy-4-(2-hydroxy-5-methanesulfonylphenyl)-4-methyl-2-trifluoromethylpentyl]-1H-indole-3-carbonitrile; 2-[2-hydroxy-4-(2-methoxy-5-methylphenyl)-4-methyl-2-trifluoromethylpentyl]-4-methyl-1H-indole-6-carbonitrile; 1,1,1-trifluoro-4-methyl-2-quinolin-4-ylmethyl-4-o-tolylpentan-2-ol; 1,1,1-trifluoro-4-methyl-2-quinolin-4-ylmethyl-4-m-tolylpentan-2-ol; 1,1,1-trifluoro-4-(2-fluorophenyl)-2-(1H-indol-2-ylmethyl)-4-methylpentan-2-ol; 1,1,1-trifluoro-4-(2-fluorophenyl)-4-methyl-2-quinolin-4-ylmethylpentan-2-ol; 1,1,1-trifluoro-4-(3-fluorophenyl)-2-(1H-indol-2-ylmethyl)-4-methylpentan-2-ol; 1,1,1-trifluoro-4-(3-fluorophenyl)-4-methyl-2-quinolin-4-ylmethylpentan-2-ol; 1,1,1-trifluoro-4-(4-fluorophenyl)-2-(1H-indol-2-ylmethyl)-4-methylpentan-2-ol; 1,1,1-trifluoro-4-(4-fluorophenyl)-4-methyl-2-quinolin-4-ylmethylpentan-2-ol; 3-(4,4,4-trifluoro-3-hydroxy-1,1-dimethyl-3-quinolin-4-ylmethylbutyl)phenol; 1,1,1-trifluoro-4-methyl-2-quinolin-4-ylmethyl-4-(2-trifluoromethylphenyl)pentan-2-ol; 1,1,1-trifluoro-2-(1H-indol-2-ylmethyl)-4-methyl-4-(4-trifluoromethylphenyl)pentan-2-ol; 1,1,1-trifluoro-4-methyl-2-quinolin-4-ylmethyl-4-(4-trifluoromethylphenyl)pentan-2-ol; 4-(3-chlorophenyl)-1,1,1-trifluoro-2-(1H-indol-2-ylmethyl)-4-methylpentan-2-ol; 4-(3-chlorophenyl)-1,1,1,-trifluoro-4-methyl-2-quinolin-4-ylmethylpentan-2-ol; 4-(4-dimethylaminophenyl)-1,1,1-trifluoro-2-(1H-indol-2-ylmethyl)-4-methylpentan-2-ol; 4-biphenyl-3-yl-1,1,1-trifluoro-4-methyl-2-quinolin-4-ylmethylpentan-2-ol; 4-(3-bromophenyl)-1,1,1-trifluoro-2-(1H-indol-2-ylmethyl)-4-methylpentan-2-ol; 4-(2-difluoromethoxy-5-fluorophenyl)-1,1,1-trifluoro-2-(1H-indol-2-ylmethyl)-4-methylpentan-2-ol; 4-biphenyl-3-yl-1,1,1-trifluoro-2-(1H-indol-2-ylmethyl)-4-methylpentan-2-ol; 4-(4-dimethylaminophenyl)-1,1,1-trifluoro-4-methyl-2-quinolin-4-ylmethylpentan-2-ol; 2-[4-(5-fluoro-2-methylphenyl)-2-hydroxy-4-methyl-2-trifluoromethylpentyl]-1,6-dihydropyrrolo[2,3-c]pyridin-5-one; 2-[4-(5-Fluoro-2-methylphenyl)-2-hydroxy-4-methyl-2-trifluoromethylpentyl]-6-methyl-1,6-dihydropyrrolo[2,3-c]pyridin-5-one; 2-[4-(5-fluoro-2-methyl- phenyl)-2-hydroxy-4-methyl-2-trifluoromethylpentyl]-4-methyl-1,4-dihydropyrrolo[3,2-b]pyridin-5-one; 1,1,1-trifluoro-4-(5-fluoro-2-methylphenyl)-2-(6-methoxy-1H-pyrrolo[3,2-c]pyridin-2-ylmethyl)-4-methylpentan-2-ol; 2-[4-(5-fluoro-2-methylphenyl)-2-hydroxy-4-methyl-2-trifluoromethylpentyl]-5-methyl-1,5-dihydropyrrolo[3,2-c]pyridin-6-one; 2-[4-(5-fluoro-2-methylphenyl)-2-hydroxy-4-methyl-2-trifluoromethylpentyl]-1,3a-dihydropyrrolo[3,-2-c]pyridin-6-one; 2-[4-(5-fluoro-2-methylphenyl)-2-hydroxy-4-methyl-2-trifluoromethylpentyl]-1,7-dihydropyrrolo[3,2-c]pyridine-4,6-dione; 6-[4-(5-fluoro-2-methylphenyl)-2-hydroxy-4-methyl-2-trfluoromethylpentyl]-3-methyl-1,7-dihydropyrrolo[2,3-d]pyrimidine-2,4-dione; 2-[4-(5-chloro-2,3-dihydrobenzofuran-7-yl)-2-hydroxy-4-methyl-2-trifluoro- methylpentyl]-1,6-dihydropycrolo[2,3-c]pyridin-5-one; 2-[4-(5-chloro-2,3-dihydrobenzofuran-7-yl)-2-hydroxy-4-methyl-2-trifluoromethylpentyl]-6-methyl-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-1,4-dihydropyrrolo[3,2-b]pyridin-5-one; 2-[4-(5-chloro-2,3-dihydrobenzofiran-7-yl)-2-hydroxy-4-methyl-2-trifluoromethylpentyl]-4-methyl-1,4-dihydropyrrolo[3,2-b]pyridin-5-one; 2-[4-(5-chloro-2,3-dihydrobenzofuran-7-yl)-2-hydroxy-4-methyl-2-trifluoro-methylpentyl]-1,5-dihydropyrrolo[3,2-c]pyridin-6-one; 2-[4-(5-chloro-2,3-dihydrobenzofuran-7-yl)-2-hydroxy-4-methyl-2-trifluoromethylpentyl]-5-methyl-1,5-dihydropyrrolo[3,2-c]pyridin-6-one; 4-(5-chloro-2,3-dihydrobenzofuran-7-yl)-1,1,1-trifluoro-2-(6-methoxy-5,6-dihydro-1H-pyrrolo[3,2-c]pyridin-2-ylmethyl)-4-methylpentan-2-ol; 2-[4-(5-chloro-2,3-dihydrobenzofuran-7-yl)-2-hydroxy-4-methyl-2-trifluoromethylpentyl]-1,7-dihydropyrrolo[3,2-c]pyridine-4,6-dione; 6-[4-(5-chloro-2,3-dihydrobenzofuran-7-yl)-2-hydroxy-4-methyl-2-trifluoromethylpentyl]-3-methyl-1,7-dihydropyrrolo[2,3-d]pyrimidine-2,4-dione; 2-[4-(3-dimethylaminomethylphenyl)-2-hydroxy-4-methyl-2-trifluoromethylpentyl]-1H-indole-5-carbonitrile; 1,1,1-trifluoro-2-(1H-indol-2-ylmethyl)-4-methyl-4-(3-morpholin-4-ylmethylphenyl)pentan-2-ol; 1,1,1-trifluoro-4-methyl-4-(3-morpholin-4-ylmethylphenyl)-2-(1H-pyrrolo[2-,3-d]pyridazin-2-ylmethyl)pentan-2-ol; 1,1,1-trifluoro-4-(5-fluoro-2-methylphenyl)-4-methyl-2-(5-morpholin-4-ylmethyl-1H-indol-2-ylmethyl)pentan-2-ol; 1,1,1-trifluoro-4-(5-fluoro-2-methylphenyl)-4-methyl-2-(5-morpholin-4-ylmethyl-1H-pyrrolo[2,3-c]pyridin-2-ylmethyl)pentan-2-ol; {2-[4-(5-fluoro-2-methylphenyl)-2-hydroxy-4-methyl-2-trifuoromethylpentyl]-1H-indol-5-yl}phenylmethanone; {2-[4-(5-fluoro-2-methylphenyl)-2-hydroxy-4-methyl-2-trifluoromethylpenty-1]-1H-pyrrolo[2,3-c]pyridin-5-yl}phenylmethanone; {2-[4-(5-fluoro-2-methylphenyl)-2-hydroxy-4-methyl-2-trifluoromethylpentyl]-1H-indol-5-yl}furan-2-ylmethanone; (2-[4-(5-fluoro-2-methylphenyl)-2-hydroxy-4-methyl-2-trifluoromethylpentyl]-1H-pyrrolo[2,3-c]pyridin-5-yl}furan-2-ylmethanone; 1,1,1-trifluoro-2-(1H-indol-2-ylmethyl)-4-methyl-4-pyridin-2-ylpentan-2-ol; 1,1,1-trifluoro-4-methyl-4-pyridin-4-yl-2-quinolin-4-ylmethylpentan-2-ol; 2-(2,6-dimethylpyridin-4-ylmethyl)-1,1,1-trifluoro-4-(5-fluoro-2-methoxyphenyl)-4-methylpentan-2-ol; 2-[3-(2,6-dimethylpyridin-4-ylmethyl)-4,4,4-trifluoro-3-hydroxy-1,1-dimethylbutyl]-4-fluorophenol; 1,1,1-trifluoro-4,4-dimethyl-5-phenyl-2-quinolin-4-ylmethylpentan-2-ol; 1,1,1-trifluoro-4-(5-fluoro-2-methoxyphenyl)-4-methyl-2-pyridin-4-ylmethylpentan-2-ol; 4-fluoro-2-[4,4,4-trifluoro-3-(2-fluoropyridin-4-ylmethyl)-3-hydroxy-1,1-dimethylbutyl]phenol; 2-[3-(2-bromopyridin-4-ylmethyl)-4,4,4-trifluoro-3-hydroxy-1,1-dimethylbutyl]-4-fluorophenol; 2-(6,8-dimethylquinolin-4-ylmethyl)-1,1,1-trifluoro-4-(5-fluoro-2-methoxy- phenyl)-4-methylpentan-2-ol; 4-[4-(5-fluoro-2-methoxyphenyl)-2-hydroxy-4-methyl-2-trifluoromethylpentyl]pyridine-2-carbonitrile; 2,6-dichloro-4-[4-(5-fluoro-2-methoxyphenyl)-2-hydroxy-4-methyl-2-trifluoromethylpentyl]nicotinonitrile; 4-[4-(5-fluoro-2-methoxyphenyl)-2-hydroxy-4-methyl-2-trifluoromethylpentyl]quinolin-2-ol; 2,6-dichloro-4-[4-(5-fluoro-2-hydroxyphenyl)-2-hydroxy-4-methyl-2-trifluoromethylpentyl]nicotinonitrile; 2-(2-chloro-8-methylquinolin-4-ylmethyl)-1,1,1-trifluoro-4-(5-fluoro-2-methoxyphenyl)-4-methylpentan-2-ol; 2-(2,6-dichloroquinolin-4-ylmethyl)-1,1,1-trifluoro-4-(5-fluoro-2-methoxyphenyl)-4-methylpentan-2-ol; 2-[3-(2-chloro-8-methylquinolin-4-ylmethyl)-4,4,4-trifluoro-3-hydroxy-1,1-dimethylbutyl]-4-fluorophenol; 2-[3-(2,6-dichloroquinolin-4-ylmethyl)-4,4,4-trifluoro-3-hydroxy-1,1-dimethylbutyl]-4-fluorophenol; 4-(2,3-dihydrobenzofuran-7-yl)-2-(2,6-dimethylpyridin-4-ylmethyl)-1,1,1-trifluoro-4-methylpentan-2-ol; 2-(2,6-dimethylpyridin-4-ylmethyl)-1,1,1-trifluoro-4-(3-fluorophenyl)-4-methylpentan-2-ol; 2-(2,6-dimethylpyndin-4-ylmethyl)-1,1,1-trifluoro-4-(4-fluorophenyl)-4-methylpentan-2-ol; 1,1,1-trifluoro-4-(5-fluoro-2-methylphenyl)-4-methyl-2-quinolin-4-ylmethylpentan-2-ol; 2-(2,6-dimethylpyridin-4-ylmethyl)-1,1,1-trifluoro-4-(5-fluoro-2-methylphenyl)-4-methylpentan-2-ol; 2-(2,6-dimethylpyridin-4-ylmethyl)-1,1,1-trifluoro-4-methyl-4-m-tolylpentan-2-ol; 1,1,1-trifluoro-4-(5-fluoro-2-methoxyphenyl)-4-methyl-2-(2-methylquinolin-4-ylmethyl)pentan-2-ol; 4-fluoro-2-(4,4,4-trifluoro-3-hydroxy-1,1,1-dimetilyl-3-quinolin-4-ylmethylbutyl)phenol; 4-fluoro-2-[4,4,4-trifluoro-3-hydroxy-1,1-dimethyl-3-(2-methylquinolin-4-ylmethyl)butyl]phenol; 2-(2,6-dimethylpyridin-4-ylmethyl)-1,1,1-trifluoro-4-(4-fluoro-2-methoxyphenyl)-4-methylpentan-2-ol; 1,1,1-trifluoro-4-(5-fluoro-2-methoxyphenyl)-4-methyl-2-(7-methylquinolin-4-ylmethyl)pentan-2-ol; 2-[3-(2,6-dimethylpyridin-4-ylmethyl)-4,4,4-trifluoro-3-hydroxy-1,1-dimethylbutyl]-5-fluorophenol; and 2-(5,7-dimethylquinolin-4-ylmethyl)-1,1,1-trifluoro-4-(5-fluoro-2-methoxyphenyl)-4-methylpentan-2-ol.

In still another example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono-

or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 2-cyclopropyl-4-(5-fluoro-2-methoxyphenyl)-4-methyl-1-(1H-pyrrolo [3,2-c]pyridin-2-yl)pentan-2-ol; 4-(5-fluoro-2-methoxyphenyl)-2-hydroxy-4-methyl-2-(1H-pyrrolo[2,3-c]pyridin-2-ylmethyl)pentanoic acid; 4-(5-fluoro-2-methoxyphenyl)-2-hydroxy-4-methyl-2-(1H-pyrrolo[2,3-c]pyridin-2-ylmethyl)pentanoic acid methyl ester, 2-cyclopropyl-4-(5-fluoro-2-methylphenyl)-4-methyl-1-(1H-pyrrolo[2,3-c]pyridin-2-yl)pentan-2-ol; 4-(5-chloro-2,3-dihydrobenzofuran-7-yl)-2-cyclopropyl-4-methyl-1-(1H-pyrrolo[2,3-c]pyridin-2-yl)pentan-2-ol; 2-cyclopropyl-4-(5-fluoro-2-methylphenyl)-4-methyl-1-(1H-pyrrolo[3,2-c]pyridin-2-yl)pentan-2-ol; 4-(5-chloro-2,3-dihydrobenzofuran-7-yl)-2-cyclopropyl-4-methyl-1-(1H-pyrrolo[3,2-c]pyridin-2-yl)pentan-2-ol; 4-(5-fluoro-2-methoxyphenyl)-2,4-dimethyl-1-(1H-pyrrolo[2,3-c]pyridin-2-yl)pentan-2-ol; 5-(5-fluoro-2-methoxyphenyl)-2,5-dimethyl-3-(1H-pyrrolo[2,3-c]pyridin-2-ylmethyl)hexan-3-ol; 5-(5-fluoro-2-methoxyphenyl)-2,2,5-trimethyl-3-(1H-pyrrolo[2,3-c]pyridin-2-ylmethyl)hexan-3-ol; 2-cyclohexyl-4-(5-fluoro-2-methoxyphenyl)-4-methyl-1-(1H-pyrrolo[2,3-c]pyridin-2-yl)pentan-2-ol; 2-cyclopentyl-4-(5-fluoro-2-methoxyphenyl)-4-methyl-1-(1H-pyrrolo[2,3-c]pyridin-2-yl)pentan-2-ol; 5-(5-fluoro-2-methoxyphenyl)-5-methyl-3-(1H-pyrrolo[2,3-c]pyridin-2-ylmethyl)hexan-3-ol; 2-(5-fluoro-2-methoxyphenyl)-2,6-dimethyl-4-(1H-pyrrolo[2,3-c]pyridin-2-ylmethyl)heptan-4-ol; 2-(5-fluoro-2-methoxyphenyl)-2,5,5-trimethyl-4-(1H-pyrrolo[2,3-c]pyridin-2-ylmethyl)heptan-4-ol; 1,1-difluoro-4-(5-fluoro-2-methoxyphenyl)-4-methyl-2-(1H-pyrrolo[2,3-c]pyridin-2-ylmethyl)pentan-2-ol; 1-cyclohexyl-4-(5-fluoro-2-methoxyphenyl)-4-methyl-2-(1H-pyrrolo[2,3-c]pyridin-2-ylmethyl)pentan-2-ol; 5-(5-fluoro-2-methylphenyl)-2,5-dimethyl-3-(1H-pyrrolo[2,3-c]pyridin-2-ylmethyl)hexan-3-ol; 5-(5-fuoro-2-methylphenyl-)-2,2,5-trimethyl-3-(1H-pyrrolo[2,3-c]pyridin-2-ylmethyl)hexan-3-ol; 5-(5-chloro-2,3-dihydrobenzofuran-7-yl)-2,5-dimethyl-3-(1H-pyrrolo [2,3-c]pyridin-2-ylmethyl)hexan-3-ol; 2-cyclobutyl-4-(5-fluoro-2-methoxyphenyl)-4-methyl-1-(1H-pyrrolo[2,3-c]pyridin-2-yl)pentan-2-ol; 2-(5-fluoro-2-methoxyphenyl)-2,6,6-trimethyl-4-(1H-pyrrolo[2,3-c]pyridin-2-ylmethyl)heptan-4-ol; 5-(5-fluoro-2-methoxyphenyl)-5-methyl-3-(1H-pyrrolo[2,3-c]pyridin-2-ylmethyl)hex-1-en-3-ol; 5-(5-fluoro-2-methoxyphenyl)-5-methyl-3-(1H-pyrrolo[2,3-c]pyridin-2-ylmethyl)hex-1-yn-3-ol; 1-fluoro-4-(5-fluoro-2-methoxyphenyl)-4-methyl-2-(1H-pyrrolo[2,3-c]pyridin-2-ylmethyl)pentan-2-ol; 2,2-difluoro-5-(5-fluoro-2-methoxyphenyl)-5-methyl-3-(1H-pyrrolo[2,3-c] pyridin-2-ylmethyl)hexan-3-ol; 2-fluoro-5-(5-fluoro-2-methoxyphenyl)-2,5-dimethyl-3-(1H-pyrrolo[2,3-c]pyridin-2-ylmethyl)hexan-3-ol; 2-fluoro-5-(5-fluoro-2-methoxyphenyl)-5-methyl-3-(1H-pyrrolo[2,3-c]pyridin-2-ylmethyl)hexan-3-ol; 5-(5-fluoro-2-methoxyphenyl)-2,5-dimethyl-3-(1H-pyrrolo[2,3-c]pyridin-2-ylmethyl)hex-1-en-3-ol; 1,1,1-trifluoro-5-(5-fluoro-2-methoxyphenyl)-5-methyl-3-(1H-pyrrolo[2,3-c]pyridin-2-ylmethyl)hexan-3-ol; 4-(5-fluoro-2-methoxyphenyl)-4-methyl-2-phenyl-1-(1H-pyrrolo[2,3-c]pyridin-2-yl)pentan-2-ol; 5-(5-chloro-2,3-dihydrobenzofuran-7-yl)-2,2,5-trimethyl-3-(1H-pyrrolo[2,3-c]pyridin-2-ylmethyl)hexan-3-ol; 5-(5-fluoro-2-methylphenyl)-2,2,5-trimethyl-3-thieno[2,3-c]pyridin-2-ylmethylhexan-3-ol; 1,1-difluoro-4-(5-fluoro-2-methoxyphenyl)4-methyl-2-(1H-pyrrolo[3,2-c]pyridin-2-ylmethyl)pentan-2-ol; 5-(5-fluoro-2-methoxyphenyl)-2,5-dimethyl-3-(1H-pyrrolo[3,2-c]pyridin-2-ylmethyl)hexan-3-ol; 5-(5-fluoro-2-methoxyphenyl)-2,2,5-trimethyl-3-(1H-pyrrolo[3,2-c]pyridin-2-ylmethyl)hexan-3-ol; 2-(1-fluorocyclopropyl)-4-(5-fluoro-2-methoxyphenyl)-4-methyl-1-(1H-pyrrolo[2,3-c]pyridin-2-yl)pentan-2-ol; 2-(1-fluorocyclopropyl)-4-(4-fluorophenyl)-4-methyl-1-quinolin-4-ylpentan-2-ol; 2-[4,4-difluoro-3-hydroxy-1,1-dimethyl-3-(1H-pyrrolo[3,2-c]pyridin-2-ylmethyl)butyl]-4-fluorophenol; 5-(5-chloro-2,3-dihydrobenzofuran-7-yl)-2,5-dimethyl-3-(1H-pyrrolo[3,2-c]pyridin-2-ylmethyl)hexan-3-ol; 5-(5-fluoro-2-methylphenyl)-2,5-dimethyl-3-(1H-pyrrolo[3,2-c]pyridin-2-ylmethyl)hexan-3-ol; 5-(5-fluoro-2-methylphenyl)-2,2,5-trimethyl-3-(1H-pyrrolo[3,2-c]pyridin-2-ylmethyl)hexan-3-ol; 4-(5-chloro-2,3-dihydrobenzofuran-7-yl)-1,1-difluoro-4-methyl-2-(1H-pyrrolo[3,2-c]pyridin-2-ylmethyl)pentan-2-ol; 4-(5-chloro-2,3-dihydrobenzofuran-7-yl)-1,1-difluoro-4-methyl-2-pyrrolo[3,2-b]pyridin-1-ylmethylpentan-2-ol; 5-(5-chloro-2,3-dihydrobenzofuran-7-yl)-2,2,5-trimethyl-3-(1H-pyrrolo[3,2-c]pyridin-2-ylmethyl)hexan-3-ol; 5-(5-fluoro-2-methylphenyl)-2,2,5-trimethyl-3-(3-methyl-1H-pyrrolo [2,3-c]pyridin-2-ylmethyl)hexan-3-ol; 5-(5-chloro-2,3-dihydrobenzofuran-7-yl)-2,5-dimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(5-phenyl-1H-pyrrolo[2,3-c]pyridin-2-ylmethyl)hexan-3-ol; 5-(5-fluoro-2-methylphenyl)-2,2,5-trimethyl-3-(5-phenyl-1H-pyrrolo[2,3-c]pyridin-2-ylmethyl)hexan-3-ol; 5-(5-fluoro-2-methylphenyl)-2,5-dimethyl-3-(5-phenyl-1H-pyrrolo[2,3-c]pyridin-2-ylmethyl)hexan-3-ol; 5-(5-fluoro-2-methylphenyl)-5-methyl-3-(5-phenyl-1H-pyrrolo[2,3-c]pyridin-2-ylmethyl)hexan-3-ol; 4-(5-fluoro-2-methylphenyl)-2,4-dimethyl-1-(5-phenyl-1H-pyrrolo[2,3-c]pyridin-2-yl)pentan-2-ol; 4-(5-chloro-2,3-dihydrobenzofuran-7-yl)-1, 1-difluoro-4-methyl-2-(6-methyl-1H-pyrrolo[3,2-c]pyridin-2-ylmethyl)pentan-2-ol; 5-(5-fluoro-2-methylphenyl)-2,5-dimethyl-3-(5-pyridin-3-yl-1H-pyrrolo[2,3-c]pyridin-2-ylmethyl)hexan-3-ol; 5-(5-chloro-2,3-dihydrobenzofuran-7-yl)-5-methyl-3-(5-phenyl-1H-pyrrolo[2,3-c]pyridin-2-ylmethyl)hexan-3-ol; 4-(5-chloro-2,3-dihydrobenzofuran-7-yl)-2,4-dimethyl-1-(5-phenyl-1H-pyrrolo[2,3-c]pyridin-2-yl)pentan-2-ol; 1,1-difluoro-4-(5-methanesulfonyl-2,3-dihydrobenzofuran-7-yl)-4-methyl-2-(1H-pyrrolo[2,3-c]pyridin-2-ylmethyl)pentan-2-ol; 5-(5-chloro-2,3-dihydrobenzofuran-7-yl)-2,5-dimethyl-3-(5-pyridin-3-yl-1H-pyrrolo[2,3-c]pyridin-2-ylmethyl)hexan-3-ol; 2-(5-bromo-1H-indol-2-ylmethyl)-1,1-difluoro-4-(5-methanesulfonyl-2,3-dihydrobenzofuran-7-yl)-4-methylpentan-2-ol; and 2-[2-difluoromethyl-2-hydroxy-4-(5-methanesulfonyl-2,3-dihydrobenzofuran-7-yl)-4-methylpentyl]-4-methyl-1H-indole-6-carbonitrile.

In still another example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently $C_1$-$C_5$ alkyl, wherein one or both are independently substituted with hydroxy, $C_1$-$C_5$ alkoxy, $C_1$-$C_5$ alkylthio wherein the sulfur atom is optionally oxidized to a sulfoxide or sulfone, amino wherein the nitrogen atom is optionally independently mono- or di-substituted by $C_1$-$C_5$ alkyl or aryl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, -$C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

In still another example, a DIGRA can have Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$

alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_5$-$C_{15}$ arylalkyl, or $R^1$ and $R^2$ together with the carbon atom they are commonly attached to form a $C_3$-$C_8$ spiro cycloalkyl ring;

(c) B is the carbonyl group or methylene group, which is optionally independently substituted with one or two substituent groups selected from the group consisting of $C_1$-$C_3$ alkyl, hydroxy, and halogen;

(d) $R^3$ is the trifluoromethyl group;

(e) D is absent;

(f) E is the hydroxy group or amino group wherein the nitrogen atom is optionally independently mono- or di-substituted by $C_1$-$C_5$ alkyl; and

(g) Q comprises a 5- to 7-membered heterocyclyl ring fused to a 5- to 7-membered heteroaryl or heterocyclyl ring, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, and ureido wherein either nitrogen atom is optionally independently substituted with $C_1$-$C_5$ alkyl or trifluoromethyl, wherein Q cannot be 1H-[1,5]naphthyridin-4-one.

Examples of these compounds include 4-[4-(5-fluoro-2-methoxyphenyl)-2-hydroxy-4-methyl-2-trifluoromethylpentyl]-4H-thieno[3,2-b]pyridin-7-one; 4-[4-(5-fluoro-2-hydroxyphenyl)-2-hydroxy-4-methyl-2-trifluoromethylpenty-1]-4H-thieno[3,2-b]pyridin-7-one; 4-[4-(2,3-dihydrobenzofuran-7-yl)-2-hydroxy-4-methyl-2-trifluoromethylpentyl]-4H-thieno[3,2-b]pyridin-7-one; 1-[4-(5-fluoro-2-methoxyphenyl)-2-hydroxy-4-methyl-2-trifluoromethylpentyl]-1H-[1,6]naphthyridin-4-one; 1-[4-(5-fluoro-2-hydroxyphenyl)-2-hydroxy-4-methyl-2-trifluoromethylpentyl]-1H-[1,6]naphthyridin-4-one; 4-[4-(5-fluoro-2-methylphenyl)-2-hydroxy-4-methyl-2-trifluoromethylpentyl]-4H-thieno[3,2-b]pyridin-7-one; 4-[2-hydroxy-4-(5-methanesulfonyl-2,3-dihydrobenzofuran-7-yl)-4-methyl-2-trifluoromethylpentyl]-4H-thieno[3,2-b]pyridin-7-one; 1-[2-hydroxy-4-(5-methanesulfonyl-2,3-dihydrobenzofuran-7-yl)-4-methyl-2-trifluoromethylpentyl]-1H-[1,6]naphthyridin-4-one; 1-[4-(5-fluoro-2-methylphenyl)-2-hydroxy-4-methyl-2-trifluoromethylpentyl]-1H-[1,6]naphthyridin-4-one; 4-[2-hydroxy-4-(2-methoxy-3-methylphenyl)-4-methyl-2-trifluoromethylpentyl]-4H-thieno[3,2-b]pyridin-7-one; 4-[2-hydroxy-4-(2-methoxyphenyl)-4-methyl-2-trifluoromethylpentyl]-4H-thieno[3,2-b]pyridin-7-one; 4-[4-(3-bromo-2-methoxyphenyl)-2-hydroxy-4-methyl-2-trifluoromethylpentyl]-4H-thieno[3,2-b]pyridin-7-one; 4-[2-hydroxy-4-(2-hydroxy-3-methylphenyl)-4-methyl-2-trifluoromethylpentyl]-4H-thieno[3,2-b]pyridin-7-one; 4-[4-(3-bromo-2-hydroxyphenyl)-2-hydroxy-4-methyl-2-trifluoromethylpentyl]-4H-thieno[3,2-b]pyridin-7-one; 3-bromo-1-[4-(5-chloro-2,3-dihydrobenzofuran-7-yl)-2-hydroxy-4-methyl-2-trifluoromethylpentyl]-1H-[1,6]naphthyridin-4-one; 6-chloro-4-[4-(2,3-dihydrobenzofuran-7-yl)-2-hydroxy-4-methyl-2-trifluoromethylpentyl]-4H-thieno[3,2-b]pyridin-7-one; 6-bromo-4-[4-(2,3-dihydrobenzofuran-7-yl)-2-hydroxy-4-methyl-2-trifluoromethylpentyl]-4H-thieno[3,2-b]pyridin-7-one; 3-chloro-1-[4-(5-fluoro-2-hydroxyphenyl)-2-hydroxy-4-methyl-2-trifluoromethylpentyl]-1H-[1,6]naphthyridin-4-one; 1-[4-(5-chloro-2,3-dihydrobenzofuran-7-yl)-2-hydroxy-4-methyl-2-trifluoromethylpentyl]-3-methyl-1H-[1,6]naphthyridin-4-one; 1-[4-(5-Chloro-2,3-dihydrobenzofuran-7-yl)-2-hydroxy-4-methyl-2-trifluoromethylpentyl]-3-methyl-1H-[1,7]naphthyridin-4-one; 1-[2-hydroxy-4-(2-methoxy-3,5-dimethylphenyl)-4-methyl-2-trifluoromethylpentyl]-3-methyl-1H-[1,6]naphthyridin-4-one; 1-[2-hydroxy-4-(2-methoxy-3,5-dimethylphenyl)-4-methy1-2-trifluoromethylpentyl]-3-methyl-1H-[1,7]naphthyridin-4-one; 1-[2-hydroxy-4-(2-hydroxy-3,5-dimethylphenyl)-4-methyl-2-trifluoromethylpentyl]-3-methyl-1H-[1,6]naphthyridin-4-one; 1-[4-(5-fluoro-2-methylphenyl)-2-hydroxy-4-methyl-2-trifluoromethylpentyl]-1H-[1,8]naphthyridin-4-one; 1-[4-(5-fluoro-2-methylphenyl)-2-hydroxy-4-methyl-2-trifluoromethylpentyl]-1H-[1,7]naphthyridin-4-one; 4-[4-(5-fluoro-2-hydroxyphenyl)-2-hydroxy-4-methyl-2-trifluoromethylpenty-1]-4H-thiazolo[4,5-b]pyridin-7-one; 4-[4-(5-fluoro-2-hydroxyphenyl)-2-hydroxy-4-methyl-2-trifluoromethylpentyl]-4H-oxazolo[4,5-b]pyridin-7-one; 4-[4-(5-fluoro-2-methylphenyl)-2-hydroxy-4-methyl-2-trifluoromethylpentyl]-4H-furo[3,2-b]pyridin-7-one; 7-[4-(5-fluoro-2-methylphenyl)-2-hydroxy-4-methyl-2-trifluoromethylpentyl]-7H-thieno[2,3-b]pyridin-4-one; 4-[4-(5-fluoro-2-hydroxyphenyl)-2-hydroxy-4-methyl-2-trifluoromethylpentyl]-4H-oxazolo[5,4-b]pyridin-7-one; 4-[4-(5-fluoro-2-hydroxyphenyl)-2-hydroxy-4-methyl-2-trifluoromethylpentyl]-4H-thiazolo [5,4-b]pyridin-7-one; 7-[4-(5-fluoro-2-methylphenyl)-2-hydroxy-4-methyl-2-trifluoromethylpentyl]-7H-furo[2,3-b]pyridin-4-one; 4-[4-(5-fluoro-2-methylphenyl)-2-hydroxy-4-methyl-2-trifluoromethylpentyl]-1,4-dihydropyrrolo[3,2-b]pyridin-7-one; 1-[4-(5-fluoro-2-hydroxyphenyl)-2-hydroxy-4-methyl-2-trifluoromethylpentyl]-5,6,7,8-tetrahydro-1H-[1,6]naphthyridin-4-one; 1-[4-(5-fluoro-2-methylphenyl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-1H-[1,8] naphthyridin-4-one; 1-[2-hydroxy-4-(5-methanesulfonyl-2,3-dihydrobenzofuran-7-yl)-4-methyl-2-trifluoromethylpentyl]-1H-[1,7]naphthyridin-4-one; 4-[2-hydroxy-4-(5-methanesulfonyl-2,3-dihydrobenzofuran-7-yl)-4-methyl-2-trifluoromethylpentyl]-4-H-thiazolo[4,5-b]pyridin-7-one; 4-[4-(2,3-dihydrobenzofuran-7-yl)-2-hydroxy-4-methyl-2-trifluoromethylpentyl]-4H-oxazolo[4,5-b]pyridin-7-one; 4-[2-hydroxy-4-(5-methanesulfonyl-2,3-dihydrobenzofuran-7-yl)-4-methyl-2-trifluoromethylpentyl]-4H-furo[3,2-b]pyridin-7-one; 7-[4-(2,3-dihydrobenzofuran-7-yl)-2-hydroxy-4-methyl-2-trifluoromethylpentyl]-7H-thieno[2,3-b]pyridin-4-one; 4-[2-hydroxy-4-(5-methanesulfonyl-2,3-dihydrobenzofuran-7-yl)-4-methyl-2-trifluoromethylpentyl]-4H-oxazolo[5,4-b]pyridin-7-one; 4-[2-hydroxy-4-(5-methanesulfonyl-2,3-dihydrobenzofuran-7-yl)-4-methyl-2-trifluoromethylpentyl]-4H-thiazolo[5,4-b]pyridin-7-one; 7-[4-(2,3-dihydrobenzofuran-7-yl)-2-hydroxy-4-methyl-2-trifluoromethylpentyl]-7H-furo[2,3-b]pyridin-4-one; 4-[4-(2,3-dihydrobenzofuran-7-yl)-2-hydroxy-4-methyl-2-trifluoromethylpentyl]-1,4-dihydropyrrolo[3,2-b]pyridin-7-one; 1-[2-hydroxy-4-(5-methanesulfonyl-2,3-dihydrobenzofuran-7-yl)-4-methyl-2-trifluoromethylpentyl]-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 4-[2-hydroxy-4-(4-methoxybiphenyl-3-yl)-4-methyl-2-trifluoromethylpentyl]-4H-thieno[3,2-b]pyridin-7-one; 4-[2-hydroxy-4-(2-methoxy-5-pyridin-3-ylphenyl)-4-methyl-2-trifluoromethylpentyl]-4H-thieno[3,2-b]pyridin-7-one; 4-[2-hydroxy-4-(2-methoxy-5-pyrimidin-5-ylphenyl)-4-methyl-2-trifluoromethylpentyl]-4H-thieno[3,2-b]pyridin-7-one; 4-[2-hydroxy-4-(2-methoxy-5-thiophen-3-ylphenyl)-4-methyl-2-trifluoromethylpentyl]-4H-thieno[3,2-b]pyridin-7-one; 4-[2-hydroxy-4-(4-hydroxybiphenyl-3-yl)-4-methyl-2-trifluoromethylpentyl]-4H-thieno[3,2-b]pyridin-7-one; 4-[2-hydroxy-4-(2-hydroxy-5-pyridin-3-ylphenyl)-4-methyl-2-trifluoromethylpentyl]-4H-thieno[3,2-b]pyridin-7-one; 4-[2-hydroxy-4-(2-hydroxy-5-pyrimidin-5-ylphenyl)-4-methyl-2-trifluoromethylpentyl]-4H-thieno[3,2-b]pyridin-7-one; 4-[2-Hydroxy-4-(2-hydroxy-5-thiophen-3-ylphenyl)-4-methyl-2-trifluoromethylpentyl]-4H-thieno[3,2-b]pyridin-7-one; 1-[2-hydroxy-4-(4-methoxybiphenyl-3-yl)-4-methyl-2-trifluoromethylpentyl]-1H-[1,6]naphthyridin-4-one; 1-(2-hydroxy-4-(2-methoxy-5-pyridin-3-ylphenyl)-4-methyl-2-trifluoromethylpentyl]-1H-[1,6]naphthyridin-4-one; 1-[2-hydroxy-4-(2-methoxy-5-pyrimidin-5-ylphenyl)-4-methyl-2-trifluoromethylpentyl]-1H-[1,6]naphthyridin-4-one; 1-[2-hydroxy-4-(2-methoxy-5-thiophen-3-ylphenyl)-4-methyl-2-trifluoromethylpentyl]-1H-[1,6]naphthyridin-4-one-; 1-[2-hydroxy-4-(2-methoxy-5-thiophen-3-ylphenyl)-4-methyl-2-trifluoromethylpentyl]-1H-[1,6]naphthyridin-4-one; 1-[2-hydroxy-4-(2-hydroxy-5-pyridin-3-ylphenyl)-4-methyl-2-trifluoromethylpentyl]-1H-[1,6]naphthyridin-4-one; 1-[2-hydroxy-4-(2-hydroxy-5-pyrimidin-5-ylphenyl)-4-methyl-2-trifluoromethylpentyl]-1H-[1,6]naphthyridin-4-one; 1-[2-hydroxy-4-(2-hydroxy-5-thiophen-3-yphenyl)-4-methyl-2-trifluoromethylpentyl]-1H-[1,6]naphthyridin-4-one; 5-[4-(5-fluoro-2-methoxyphenyl)-2-hydroxy-4-methyl-2-trifluoromethylpentyl]-5H-pyrido[3,2-d]pyrimidin-8-one; 1-[4-(5-fluoro-2-methoxyphenyl)-2-hydroxy-4-methyl-2-trifluoromethylpentyl]-1H-pyrido[2,3-d]pyridazin-4-one; 5-[4-(5-fluoro-2-methoxyphenyl)-2-hydroxy-4-methyl-2-trifluoromethylpenty-1]-5H-pyrido[3,2-c]pyridazin-8-one; 4-[4-(2-fifluoromethoxy-3-methylphenyl-)-2-hydroxy-4-methyl-2-trifluoromethylpentyl]-4H-thieno[3,2-b]pyridin-7-one; 3-chloro-1-[4-(2,3-dihydrobenzofuran-7-yl)-2-hydroxy-4-methyl-2-trifluoromethylpentyl]-1H-[1,6]naphthyridin-4-one; 4-(4-benzo[1,3]dioxol-4-yl-2-hydroxy-4-methyl-2-trifluoromethylpentyl)-6-bromo-4H-thieno[3,2-b]pyridin-7-one; 4-(4-benzo[1,3]dioxol-4-yl-2-hydroxy-4-methyl-2-trifluoromethylpentyl)-6-chloro-4H-thieno[3,2-b]pyridin-7-one; 6-chloro-4-[2-hydroxy-4-methyl-4-(5-pyridin-3-yl-2,3-dihydrobenzofuran-7-yl)-2-trifluoromethylpentyl]-4H-thieno[3,2-b]pyridin-7-one; 1-(4-benzo[1,3]dioxol-4-yl-2-hydroxy-4-methyl-2-trifluoromethylpentyl)-3-chloro-1H-[1,6]naphthyridin-4-one; 6-chloro-4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 3-chloro-1-[2-hydroxy-4-methyl-4-(5-pyridin-3-yl-2,3-dihydrobenzofuran-7-yl)-2-trifluoromethylpentyl]-1H-[1,6]naphthyridin-4-one; 4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 6-chloro-4-[2-hydroxy-4-(2-methoxy-5-pyridin-3-ylphenyl)-4-methyl-2-trifluoromethylpentyl]-4H-thieno[3,2-b]pyridin-7-one; 6-chloro-4-[2-hydroxy-4-(2-methoxy-5-pyrimidin-5-ylphenyl)-4-methyl-2-trifluoromethylpentyl]-4H-thieno[3,2-b]pyridin-7-one; 6-chloro-4-[2-hydroxy-4-(2-hydroxy-5-pyridin-3-ylphenyl)-4-methyl-2-trifluoromethylpentyl]-4H-thieno[3,2-b]pyridin-7-one; 6-chloro-4-[2-hydroxy-4-(-2-hydroxy-5-pyrimidin-5-ylphenyl)-4-methyl-2-trifluoromethylpentyl]-4H-thieno[3,2-b]pyridin-7-one; 4-(4-biphenyl-3-yl-2-hydroxy-4-methyl-2-trifluoro-methylpentyl)-6-chloro-4H-thieno[3,2-b]pyridin-7-one; 4-(4-biphenyl-3-yl-2-hydroxy-4-methyl-2-trifluoromethylpentyl)-4H-thieno[3,2-b]pyridin-7-one; 3-chloro-1-{4-[5-(5-chloropyridin-3-yl)-2,3-dihydrobenzofuran-7-yl]-2-hydroxy-4-methyl-2-trifluoromethylpentyl}-1H-[1,6]naphthyridin-4-one; 6-chloro-4-{4-[5-(2,6-dimethylpyridin-4-yl)-2-methoxyphenyl]-2-hydroxy-4-methyl-2-trifluoromethylpentyl}-4H-thieno[3,2-b]pyridin-7-one-; 4-[2-hydroxy-4-(2-hydroxy-5-pyridin-2-ylphenyl)-4-methyl-2-trifluoromethylpentyl]-4H-thieno[3,2-b]pyridin-7-one; 6-chloro-4-[2-hydroxy-4-methyl-4-(5-pyrazin-2-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-2-yl-2,3-dihydrobenzofuran-7-yl)-2-trifluoromethylpentyl]-1H-[1,6]naphthyridin-4-one; 5-{7-[3-(6-chloro-7-oxo-7H-thieno[3,2-b]pyridin-4-ylmethyl)-4,4,-4-trifluoro-3-hydroxy-1,1-dimethylbutyl]-2,3-dihydrobenzofuran-5-yl}nicotinonitrile; 4-{4-Methoxy-3-[4,4,4-trifluoro-3-hydroxy-1,1-dimethyl-3-(7-oxo-7H-thieno[3,2-b]pyridin-4-ylmethyl)butyl]phenyl}pyridine-2-carbonitrile; 6-chloro-4-{4-[5-(2-fluoro-6-methylpyridin-4-yl)-2-methoxyphenyl]-2-hydroxy-4-methyl-2-trifluoromethylpentyl}-4H-thieno[3,2-b]pyridin-7-one; 3-chloro-1-{2-hydroxy-4-[5-(1H-imidazol-4-yl)-2,3-dihydrobenzofuran-7-yl]-4-methyl-2-trifluoromethylpentyl}-1H-[1,6]naphthyridin-4-one; 6-chloro-4-[2-hydroxy-4-methyl-4-(5-morpholin-4-yl-2,3-dihydrobenzofuran-7-yl)-2-trifluoromethylpentyl]-4H-thieno[3,2-b]pyridin-7-one; and 1-[2-hydroxy-4-methyl-4-(5-piperidin-1-yl-2,3-dihydrobenzofuran-7-yl)-2-trifluoromethylpentyl]-1H-[1,6]naphthyridin-4-one.

In yet another example, a DIGRA can have Formula I, wherein A, B, D, E, $R^1$, and $R^2$ have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

In yet another example, a DIGRA has Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an indolyl group optionally substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, halogen, hydroxy, oxo, cyano, amino, and trifluoromethyl.

Examples of these compounds include 4-(5-bromo-2,3-dihydrobenzofuran-7-yl)-1,1,1-trifluoro-2-(1H-indol-2-ylmethyl)-4-methylpentan-2-ol; 1,1,1-trifluoro-2-(1H-indol-2-ylmethyl)-4-methyl-4-pyridin-2-ylpentan-2-ol; 4-(2,3-dihydro-5-cyanobenzofuran-7-yl)-1,1,1-trifluoro-2-(1H-indol-2-yl-methyl)-4-methylpentan-2-ol; 4-(2,3-dihydrobenzofuran-7-yl)-1,1,1-trifluoro-2-(1H-indol-2-ylmethyl)-4-methylpentan-2-ol; 1,1,1-trifluoro-4-(5-fluoro-2,3-dihydrobenzofuran-7-yl)-2-(1H-indol-2-ylmethyl)-4-methylpentan-2-ol; 1,1,1-trifluoro-2-(1H-indol-2-ylmethyl)-4-methyl-4-(5-methyl-2,3-dihydrobenzofuran-7-yl)pentan-2-ol; 4-(2,3-dihydrobenzofuran-5-yl)-1,1,1-trifluoro-2-(1H-indol-2-ylmethyl)-4-methylpentan-2-ol; 2-[4-(2,3-dihydrobenzofuran-7-yl)-2-hydroxy-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-3-carbonitrile; 2-[4-(5-bromo-2,3-dihydrobenzofuran-7-yl)-2-hydroxy-4-methyl-2-trifluoromethylpentyl]-1H-indole-3-carbonitrile; 2-[4-(2,3-dihydrobenzofuran-7-yl)-2-hydroxy-4-methyl-2-trifluoromethylpentyl]-4-methyl-1H-indole-6-carbonitrile; 2-[4-(2,3-dihydrobenzofuran-7-yl)-2-hydroxy-4-methyl-2-trifluoromethylpentyl]-1H-indole-5-carbonitrile; 4-(2,3-dihydrobenzofuran-7-yl)-1,1,1-trifluoro-2-(7-fluoro-1H-indol-2-ylmethyl)4-methylpentan-2-ol; 1-[4-(2,3-dihydrobenzofuran-7-yl)-2-hydroxy-4-methyl-2-trifluoromethylpentyl]-1H-indole-3-carbonitrile; 4-(2,3-dihydrobenzofuran-7-yl)-1,1,1-trifluoro-4-methyl-2-(5-trifluoromet-hyl-1H-indol-2-ylmethyl)pentan-2-ol; and 1,1,1-trifluoro-2-(1H-indol-2-ylmethyl)-4-methyl-4-thiophen-3-ylpentan-2-ol.

In a further example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is the methylene or carbonyl group;

(e) D is the -NH- group;

(f) E is the hydroxy group; and

(g) Q comprises the group

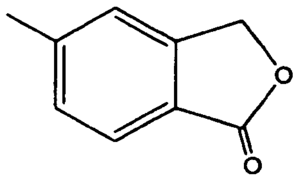

.

Examples of these compounds include 2-benzyl-2-hydroxy-4-methyl-4-phenylpentanoic acid (1-oxo-1,3-dihydroisobenzofuran-5-yl)amide; 2-hydroxy-4-methyl-2,4-diphenylpentanoic acid (1-oxo-1,3-dihydroisobenzofuran-5-yl)amide; 2-hydroxy-4-methyl-2-phenethyl-4-phenylpentanoic acid (1-oxo-1,3-dihydroisobenzofuran-5-yl)amide; 2-hydroxy-2-(3-methoxybenayl)$_4$-methyl-4-phenylpentanoic acid (1-oxo-1,3-dihydroisobenzofuran-5-yl)amide; 2-hydroxy-2-(4-methoxybenzyl)-4-methyl-4-phenylpentanoic acid (1-oxo-1,3-dihydroisobenzofuran-5-yl)amide; 2-hydroxy-2-[2-(4-methoxyphenyl)ethyl]4-methyl-4-phenylpentanoic acid (1-oxo-1,3-dihydroisobenzofuran-5-yl)amide; 2-cyclohexylmethyl-2-hydroxy-4-methyl-4-phenylpentanoic acid (1-oxo-1,3-dihydroisobenzofuran-5-yl)amide; 2-(4-tert-butylbenzyl)-2-hydroxy-4-methyl-4-phenylpentanoic acid (1-oxo-1,3-dihydroisobenzofuran-5-yl)amide; 2-biphenyl-4-ylmethyl-2-hydroxy-4-methyl-4-phenylpentanoic acid (1-oxo-1,3-dihydroisobenzofuran-5-yl)amide; 2-hydroxy-4-methyl-2-naphthalen-2-ylmethyl-4-phenylpentanoic acid (1-oxo-1,3-dihydroisobenzofuran-5-yl)amide; 2-hydroxy-2-(3-hydroxybenzyl)-4-methyl-4-phenylpentanoic acid (1-oxo-1,3-dihydroisobenzofuran-5-yl)amide; 2-hydroxy-4-methyl-2-(2-methyl-2-phenylpropyl)-4-phenylpentanoic acid (1-oxo-1,3-dihydroisobenzofuran-5-yl)amide; 2-benryl-4-(5-fluoro-2-methoxyphenyl)-2-hydroxy-4-methylpentanoic acid (1-oxo-1,3-dihydroisobenzofuran-5-yl)amide; 2-cyclohexylmethyl-4-(5-fluoro-2-methoxyphenyl)-2-hydroxy-4-methylpentanoic acid (1-oxo-1,3-dihydroisobenzofuran-5-yl)amide; 2-benzyl-4-(5-fluoro-2-hydroxyphenyl)-2-hydroxy-4-methylpentanoic acid (1-oxo-1,3-dihydroisobenzofuran-5-yl)amide; 2-cyclohexylmethyl-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methyl-2-phenylpropyl)pentanoic acid (1-oxo-1,3-dihydroisobenzofuran-5-yl)amide; 2-(2-chloro-6-fluorobenzyl)-4-(5-fluoro-2-methoxyphenyl)-2-hydroxy-4-methylpentanoic acid (1-oxo-1,3-dihydroisobenzofuran-5-yl)amide; 2-(3-fluorobenzyl)-4-(5-fluoro-2-methoxyphenyl)-2-hydroxy-4-methylpentanoic acid (1-oxo-1,3-dihydroisobenzofuran-5-yl)amide; 2-(2-fluorobenzyl)-4-(5-fluoro-2-methoxyphenyl)-2-hydroxy-4-methylpentanoic acid (1-oxo-1,3-dihydroisobenzofuran-5-yl)amide; 2-(3,4-difluorobenzyl)-4-(5-fluoro-2-methoxyphenyl)-2-hydroxy-4-methylpentanoic acid (1-oxo-1,3-dihydroisobenzofuran-5-yl)amide; 2-(2-chloro-6-fluorobenzyl)-4-(5-fluoro-2-hydroxyphenyl)-2-hydroxy-4-methylpentanoic acid (1-oxo-1,3-dihydroisobenzofuran-5-yl)amide; 2-(3-fluorobenzyl)-4-(5-fluoro-2-hydroxyphenyl)-2-hydroxy-4-methylpentanoic acid (1-oxo-1,3-dihydroisobenzofuran-5-yl)amide; 2-(2-fluorobenzyl)-4-(5-fluoro-2-hydroxyphenyl)-2-hydroxy-4-methylpentanoic acid (1-oxo-1,3-dihydroisobenzofuran-5-yl)amide; 2-(3,4-difluorobenzyl)-4-(5-fluoro-2-hydroxyphenyl)-2-hydroxy-4-methylpentanoic acid (1-oxo-1,3-dihydroisobenzofuran-5-yl)amide; 2-(4-fluorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(3-methylbenzyl)pentanoic acid (1-oxo-1,3-dihydroisobenzofuran-5-yl)amide; 2-(4-fluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(3-methylbenzyl)pentanoic acid (1-oxo-1,3-dihydroisobenzofuran-5-yl)amide; 2-(3,5-difluorophenyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-hydroxyphenyl)-2-hydroxy-4-methylpentanoic acid (1-oxo-1,3-dihydroisobenzofuran-5-yl)amide; 2-(3-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-2-[2-(4-methoxyphenyl)ethyl]-4-methylpentanoic acid (1-oxo-1,3-dihydroisobenzofuran-5-yl)amide; 4-(5-fluoro-2-methoxyphenyl)-2-hydroxy-2-(2-methoxybenzyl)$_4$-methylpentanoic acid (1-oxo-1,3-dihydroisobenzofuran-5-yl)amide; 4-(5-fluoro-2-methoxyphenyl)-2-hydroxy-4-methyl-2-phenethylpentanoic acid (1-oxo-1,3-dihydroisobenzofuran-5-yl)amide; 2-(2-chlorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-phenethylpentanoic acid (1-oxo-1,3-dihydroisobenzofuran-5-yl)amide; 4-(5-fluoro-2-hydroxyphenyl)-2-hydroxy-2-[2-(4-hydroxyphenyl)ethyl]-4-methylpentanoic acid (1-oxo-1,3-dihydroisobenzofuran-5-yl)amide; 2-(2-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-2-(2-hydroxybenzyl)-4-methylpentanoic acid (1-oxo-1,3-dihydroisobenzofuran-5-yl)amide; 2-(2-bromobenzyl)-4-(5-fluoro-2-methoxyphenyl)-2-hydroxy-4-methylpentanoic acid (1-oxo-1,3-dihydroisobenzofuran-5-yl)amide; 2-(2-bromobenzyl)-4-(5-fluoro-2-hydroxyphenyl)-2-hydroxy-4-methylpentanoic acid (1-oxo-1,3-dihydroisobenzofuran-5-yl)amide; 2-(5-fluoro-2-methoxybenzyl)-2-hydroxy-4-methyl-4-phenylpentanoic acid (1-oxo-1,3-dihydroisobenzofuran-5-yl)amide; 2-(5-fluoro-2-hydroxybenzyl)-2-hydroxy-4-methyl-4-phenylpentanoic acid (1-oxo-1,3-dihydroisobenzofuran-5-yl)amide; 2-(5-fluoro-2-methoxybenzyl)-4-(5-fluoro-2-methoxyphenyl)-2-hydroxy-4-methylpentanoic acid (1-oxo-1,3-dihydroisobenzofuran-5-yl)amide; 2-(5-fluoro-2-hydroxybenzyl)-4-(5-fluoro-2-hydroxyphenyl)-2-hydroxy-4-methylpentanoic acid (1-oxo-1,3-dihydroisobenzofuran-5-yl) amide; 2-(3,5-dimethoxybenzyl)-2-hydroxy-4-methyl-4-phenylpentanoic acid (1-oxo-1,3-dihydroisobenzofuran-5-yl) amide; 2-(3,5-dihydroxybenzyl)-2-hydroxy-4-methyl-4-phenylpentanoic acid (1-oxo-1,3-dihydroisobenzofuran-5-yl)-amide; 2-hydroxy-2-(2-methoxybenzyl)$_4$-methyl-4-phenylpentanoic acid (1-oxo-1,3-dihydroisobenzofuran-5-yl) amide; 12-hydroxy-2-(2-hydroxybenzyl)-4-methyl-4-phenylpentanoic acid (1-oxo-1,3-dihydroisobenzofuran-5-yl)amide; 2-hydroxy-2-[2-(4-hydroxyphenyl)ethyl]-4-methyl-4-phenylpentanoic acid (1-oxo-1,3-dihydroisobenzofuran-5-yl)amide; 15-[2-benzyl-4-(5-fluoro-2-methoxyphenyl)-2-hydroxy-4-methylpentylamino]-3H-isobenzofuran-1-one; 4-(5-fluoro-2-methoxyphenyl)-2-hydroxy-4-methyl-2-(1-phenylvinyl)pentanoic acid (1-oxo-1,3-dihydroisobenzofuran-5-yl)amide; 2-hydroxy-4-methyl-4-phenyl-2-pyridin-2-ylmethylpentanoic acid(1-oxo-1,3-dihydroisobenzofuran-5-yl)amide; 4-(5-fluoro-2-methoxyphenyl)-2-hydroxy-4-methyl-2-(1-phenylethyl-)pentanoic acid(1-oxo-1,3-dihydroisobenzofuran-5-yl)amide; 4-(5-fluoro-2-hydroxyphenyl)-2-hydroxy-4-methyl-2-(1-phenylethyl)pentanoic acid(1-oxo-1,3-dihydroisobenzofuran-5-yl)amide; 2-cyclopentyl-4-(5-fluoro-2-methoxyphenyl)-2-hydroxy-4-methylpentanoic acid(1-oxo-1,3-dihydroisobenzofuran-5-yl)amide; 2-cyclopentyl-4-(5-fluoro-2-hydroxyphenyl)-2-hydroxy-4-methylpentanoic acid(1-oxo-1,3-dihydroisobenzofuran-5-yl)amide; 2-cyclopentylmethyl-4-(5-fluoro-2-hydroxyphenyl)-2-hydroxy-4-methylpentanoic acid(1-oxo-1,3-dihydroisobenzofuran-5-yl)amide; and 2-benzyl-2-hydroxy-N-(1-oxo-1,3-dihydroisobenzofiuan-5-yl)$_4$-phenyl-butyramide.

In still another example, a DIGRA can have Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is -$NR^6R^7$, wherein $R^6$ and $R^7$ are each independently hydrogen, $C_1$-$C_8$ alkyl, $C_2$-$C_8$ alkenyl, $C_2$-$C_8$ alkynyl, $C_1$-$C_8$ alkoxy, $C_2$-$C_8$ alkenyloxy, $C_2$-$C_8$ alkynyloxy, hydroxy, carbocyclyl, heterocyclyl, aryl, aryloxy, acyl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, heteroaryl-$C_2$-$C_8$ alkenyl, or $C_1$-$C_5$ alkylthio wherein the sulfur atom is oxidized to a sulfoxide or sulfone, each optionally independently substituted with one to three substituent groups, wherein each substituent group of $R^6$ and $R^7$ are independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, oxo, trifluoromethyl, trifluoromethoxy, nitro, amino wherein the nitrogen atom is optionally independently mono- or di-substituted by $C_1$-$C_5$ alkyl, ureido wherein either nitrogen atom is optionally independently substituted

with $C_1$-$C_5$ alkyl, or $C_1$-$C_5$ alkylthio wherein the sulfur atom is optionally oxidized to a sulfoxide or sulfone; and

(g) Q comprises a heteroaryl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl; or ureido wherein either nitrogen atom is optionally independently substituted with $C_1$-$C_5$ alkyl; or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 3-(5-fluoro-2-methoxy-phenyl)-3-methyl-1-(pyridin-2-ylmethyl)-1-trifluoromethyl-butylamine; 3-(5-fluoro-2-methoxyphenyl)-1-(1H-indol-2-ylmethyl)-3-methyl-1-trifluoromethyl-butylamine; 1-(2,6-dichloro-pyridin-4-ylmethyl)-3-(5-fluoro-2-methoxy-phenyl)-3-methyl-1-trifluoromethyl-butylamine; 1-(4,6-dimethyl-pyridin-2-ylmethyl)-3-(5-fluoro-2-methoxy-phenyl)-3-methyl-1-trifluoromethyl-butylamine; 1-(2-chloro-pyridin-4-ylmethyl)-3-(5-fluoro-2-methoxy-phenyl)-3-methyl-1-trifluoromethyl-butylamine; 3-(5-fluoro-2-methyl-phenyl)-3-methyl-1-(3-methyl-1H-indol-2-ylmethyl)-1-trifluoromethyl-butylamine; 3-(5-fluoro-2-methoxy-phenyl)-3-methyl-1-(3-methyl-1H-indol-2-ylmethyl)-1-trifluoromethyl-butylamine; 1-(6-fluoro-1H-indol-2-ylmethyl)-3-(5-fluoro-2-methoxy-phenyl)-3-methyl-1-trifluoromethyl-butylamine; 3-(4-fluoro-phenyl)-3-methyl-1-(3-methyl-1H-indol-2-ylmethyl)-1-trifluoro-methyl-butylamine; 3-benzofuran-7-yl-1-(2,6-dichloro-pyridin-4-ylmethyl)-3-methyl-1-trifluoromethyl-butylamine; 3-(2,3-dihydro-benzofuran-7-yl)-1-(6-fluoro-1H-indol-2-ylmethyl)-3-methyl-1-trifluoromethyl-butylamine; 3-(5-fluoro-2-methoxy-phenyl)-3-methyl-1-quinolin-4-ylmethyl-1-trifluoromethyl-butylamine; 1-(2-chloro-quinolin-4-ylmethyl)-3-(5-fluoro-2-methylphenyl)-3-methyl-1-trifluoromethyl-butylamine; 3-(4-fluoro-phenyl)-3-methyl-1-quinolin-4-ylmethyl-1-trifluoromethyl-butylamine; 7-[3-amino-3-(1H-benzoimidazol-2-ylmethyl)-4,4,4-trifluoro-1,1-dimethyl-butyl]-2,3-dihydrobenzofuran-5-carbonitrile; 1-(6-fluoro-1H-benzoimidazol-2-ylmethyl)-3-(5-fluoro-2-methyl-phenyl)-3-methyl-1-trifluoromethyl-butylamine; 2-[3-amino-3-(1H-benzoimidazol-2-ylmethyl)-4,4,4-trifluoro-1,1-dimethyl-butyl]4-fluoro-phenol; 1-(1H-benzoimidazol-2-ylmethyl)-3-(4-fluoro-phenyl)-3-methyl-1-trifluoromethyl-butylamine; 1-(1H-indol-2-ylmethyl)-3-methyl-3-pyridin-3-yl-1-trifluoromethyl-butylamine; 1-(1H-benzoimidazol-2-ylmethyl)-3-methyl-3-pyridin-4-yl-1-trifluoromethyl-butylamine; 3-methyl-1-(3-methyl-1H-indol-2-ylmethyl)-3-pyridin-3-yl-1-trifluoromethyl-butylamine; 1-(6-fluoro-1H-indol-2-ylmethyl)-3-methyl-3-pyridin-3-yl-1-trifluoromethyl-butylamine; 3-(2,3-dihydrobenzofuran-7-yl)-1-(1H-indol-2-ylmethyl)-3-methyl-1-trifluoromethyl-butylamine; [3-(5-fluoro-2-methoxy-phenyl)-3-methyl-1-quinolin-4-ylmethyl-1-trifluoromethyl-butyl]-methyl-amine; ethyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-propylamine; [3-(5-fluoro-2-methoxy-phenyl)-3-methyl-1-quinolin-4-ylmethyl-1-trifluoromethyl-butyl]-isobutylamine; butyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-dimethylamine; N-[3-(5-fluoro-2-methoxy-phenyl)-3-methyl-1-quinolin-4-ylmethyl-1-trifluoromethyl-butyl]-acetamide; N-[3-(5-fluoro-2-methoxy-phenyl)-3-methyl-1-quinolin-4-ylmethyl-1-trifluoromethyl-butyl]-formamide; N-[3-(5-fluoro-2-methoxy-phenyl)-3-methyl-1-quinolin-4-ylmethyl-1-trifluoromethyl-butyl]-methanesulfonamide; 1-(2,6-dimethyl-pyridin-4-ylmethyl)-3-(5-fluoro-2-methoxyphenyl)-3-methyl-1-trifluoromethyl-butylamine; 3-(5-fluoro-2-methoxy-phenyl)-3-methyl-1-(1H-pyrrolo[2,3-c]pyridin-2-ylmethyl)-1-trifluoromethyl-butylamine; 2-[2-amino-4-(5-fluoro-2-methoxy-phenyl)-4-methyl-2-trifluoromethyl-pentyl]-4-methyl-1H-indole-6-carbonitrile; N-[3-(5-fluoro-2-methoxy-phenyl)-3-methyl-1-quinolin-4-ylmethyl-1-trifluoromethyl-butyl]-hydroxylamine; and 2-(3-amino-4,4,4-trifluoro-1,1-dimethyl-3-quinolin-4-ylmethyl-butyl)-4-fluoro-phenol.

In yet another example, a DIGRA has Formula I, wherein A, B, D, E, $R^1$, $R^2$, $R^6$, and $R^7$ have the meanings disclosed immediately above, and $R^3$ is $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, **$C_3$-$C_8$** cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be

trifluoromethyl.

Examples of these compounds include 1-(2,6-dichloro-pyridin-4-ylmethyl)-3-(5-fluoro-2-methoxy-phenyl)-1,3-dimethyl-butylamine; 1-ethyl-3-(5-fluoro-2-methoxy-phenyl)-3-methyl-1-quinolin-4-ylmethyl-butylamine; 1-cyclohexylmethyl-3-(5-fluoro-2-methoxy-phenyl)-1-(1H-indol-2-ylmethyl)-3-methyl-butylamine; 1-(2-chloro-quinolin-4-ylmethyl)-1-cyclopentyl-3-(5-fluoro-2-methoxy-phenyl)-3-methylbutylamine; 1-(2-chloro-pyridin-4-ylmethyl)-1-cyclopentylmethyl-3-(5-fluoro-2-methoxy-phenyl)-3-methyl-butylamine; 3-(5-fluoro-2-methoxy-phenyl)-1,3-dimethyl-1-quinolin-4-ylmethyl-butylamine; 1-cyclopropyl-3-(5-fluoro-2-methoxy-phenyl)-3-methyl-1-quinolin-4-ylmethyl-butylamine; 3-(5-fluoro-2-methoxy-phenyl)-1,3-dimethyl-1-(1H-pyrrolo[2,3-c]pyridin-2-ylmethyl)-butylamine; 1-cyclopropyl-3-(5-fluoro-2-methoxy-phenyl)-3-methyl-1-(1H-pyrrolo[2,3-c]-pyridin-2-ylmethyl)-butylamine; 2-[3-amino-1,1,3-trimethyl-4-(1H-pyrrolo [2,3-c]pyridin-2-yl)-butyl]-4-fluoro-phenol; 2-[2-amino-4-(5-fluoro-2-methoxy-phenyl)-2,4-dimethyl-pentyl]-4-methyl-1H-indole-6-carbonitrile.

Other compounds that can function as DIGRAs and methods for their manufacture are disclosed, for example, in U.S. Patent Application Publications 2004/0029932, 2004/0162321, 2004/0224992, 2005/0059714, 2005/0176706, 2005/0203128, 2005/0234091, 2005/0282881, 2006/0014787, 2006/0030561, and 2006/0116396.

In another aspect of the present disclosure an ophthalmic pharmaceutical composition for treating or preventing glaucoma or progression thereof is provided. The ophthalmic pharmaceutical composition can comprise: (a) at least a DIGRA, , a pharmaceutically acceptable salt thereof, or a pharmaceutically acceptable ester thereof; and (b) an anti-inflammatory agent other than said DIGRA, said pharmaceutically acceptable salt thereof, and said pharmaceutically acceptable ester thereof. In one aspect of the present disclosure, the pharmaceutical composition further comprises a pharmaceutically acceptable carrier. For example, said carrier is an ophthalmically acceptable carrier.

The concentration of a DIGRA, a prodrug thereof, a pharmaceutically acceptable salt thereof, or a pharmaceutically acceptable ester thereof in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In one embodiment, a composition of the present invention is in a form of a suspension or dispersion. In another embodiment, the suspension or dispersion is based on an aqueous solution. For example, a composition of the present invention can comprise sterile saline solution. In still another embodiment, micrometer- or nanometer-sized particles of a DIGRA, a pharmaceutically acceptable salt thereof, or a pharmaceutically acceptable ester thereof and an anti-inflammatory agent can be coated with a physiologically acceptable surfactant (examples are disclosed below), then the coated particles are dispersed in a liquid medium. The coating can keep the particles in a suspension. Such a liquid medium can be selected to produce a sustained-release suspension. For example, the liquid medium can be one that is sparingly soluble in the ocular environment into which the suspension is administered. In still another embodiment, the active ingredient or ingredients are suspended or dispersed in a hydrophobic medium, such as an oil.

The DIGRA and anti-inflammatory agent other than said DIGRA, pharmaceutically acceptable salt thereof, and pharmaceutically acceptable ester thereof are present in amounts effective to treat, control, reduce, ameliorate, alleviate, or prevent the condition. In one embodiment, such an anti-inflammatory agent is selected from the group consisting of non-steroidal anti-inflammatory drugs ("NSAIDs"); peroxisome proliferator-activated receptor ("PPAR") ligands (such as PPAR$\alpha$, PPAR$\delta$, or PPAR$\gamma$ ligands); anti-histaminic drugs; antagonists to or inhibitors of proinflammatory cytokines (such as anti-TNF, anti-interleukin, anti-NF-$\kappa$B); nitric oxide synthase inhibitors; combinations thereof; and mixtures thereof. Examples of anti-histaminic drugs include Patanol® (olopatadine), Emadine® (emedastine), and Livostin® (levocabastine). Examples of anti-TNF drugs include Remicade® (infliximab), Enbrel® (etanercept), and Humira® (adalimumab). Examples of anti-interleukin drugs include Kineret (anakinra), Zenapax (daclizumab), Simulect (basixilimab), cyclosporine, and tacrolimus.

Examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lomoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

In another aspect of the present invention, an anti-inflammatory agent is a PPAR-binding molecule. In one embodiment, such a PPAR-binding molecule is a PPARα-, PPARδ-, or PPARγ-binding molecule. In another embodiment, such a PPAR-binding molecule is a PPARα, PPARδ, or PPARγ agonist. Such a PPAR ligand binds to and activates PPAR to modulate the expression of genes containing the appropriate peroxisome proliferator response element in its promoter region.

PPARγ agonists can inhibit the production of TNF-α and other inflammatory cytokines by human macrophages (C-Y. Jiang et al., Nature, Vol. 391, 82-86 (1998)) and T lymphocytes (A.E. Giorgini et al., Horm. Metab. Res. Vol. 31, 1-4 (1999)). More recently, the natural PPARγ agonist 15-deoxy-Δ-12,14-prostaglandin J2 (or "15-deoxy-Δ-12,14-PG J2"), has been shown to inhibit neovascularization and angiogenesis (X. Xin et al., J. Biol. Chem. Vol. 274:9116-9121 (1999)) in the rat cornea. Spiegelman et al., in U.S. Patent 6,242,196, disclose methods for inhibiting proliferation of PPARγ-responsive hyperproliferative cells by using PPARγ agonists; numerous synthetic PPARγ agonists are disclosed by Spiegelman et al., as well as methods for diagnosing PPARγ-responsive hyperproliferative cells. PPARs are differentially expressed in diseased versus normal cells. PPARγ is expressed to different degrees in the various tissues of the eye, such as some layers of the retina and the cornea, the choriocapillaris, uveal tract, conjunctival epidermis, and intraocular muscles (see, e.g., U.S. Patent 6,316,465).

In one aspect, a PPARγ agonist used in a composition of the present invention is a thiazolidinedione, a derivative thereof, or an analog thereof. Examples of thiazolidinedione-based PPARγ agonists include pioglitazone, troglitazone, ciglitazone, englitazone, rosiglitazone, and chemical derivatives thereof. Other PPARγ agonists include Clofibrate (ethyl 2-(4-chlorophenoxy)-2-methylpropionate), clofibric acid (2-(4-chlorophenoxy)-2-methylpropanoic acid), GW 1929 (N-(2-benzoylphenyl)-O-{2-(methyl-2-pyridinylamino)ethyl}-L-tyrosine), GW 7647 (2-{{4-{2-{{(cyclohexylamino)carbonyl}(4-cyclohexylbutyl)amino}ethyl}phenyl}thio}-2-methylpropanoic acid), and WY 14643 ({{4-chloro-6-{(2,3-dimethylphenyl) amino}-2-pyrimidinyl}thio}acetic acid). GW 1929, GW 7647, and WY 14643 are commercially available, for example, from Koma Biotechnology, Inc. (Seoul, Korea). In one embodiment, the PPARγ agonist is 15-deoxy-Δ-12, 14-PG J2.

Examples of PPAR-α agonists include the fibrates, such as fenofibrate and gemfibrozil. An example of PPAR-δ agonist is GW501516 (available from Axxora LLC, San Diego, California or EMD Biosciences, Inc., San Diego, California).

In another aspect, a composition of the present invention further comprises an anti-infective agent (such as an antibacterial, antiviral, antiprotozoal, or antifungal agent, or a combination thereof).

The concentration of such an NSAID, PPAR-binding molecule, anti-histaminic drug, antagonist to or inhibitor of proinflammatory cytokines, nitric oxide synthase inhibitor, or anti-infective agent in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

Examples of biologically-derived antibacterial agents include aminoglycosides (e.g., amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (e.g., azidamfenicol, chloramphenicol, florfenicol, thiamphenicol), ansamycins (e.g., rifamide, rifampin, rifamycin sv, rifapentine, rifaximin), β-lactams (e.g., carbacephems (e.g., loracarbef), carbapenems (e.g., biapenem, imipenem, meropenem, panipenem), cephalosporins (e.g., cefaclor, cefadroxil, cefamandole, cefatrizine, cefazedone, cefazolin, cefcapene pivoxil, cefclidin, cefdinir, cefditoren, cefepime, cefetamet, cefixime, cefinenoxime, cefodizime, cefonicid, cefoperazone, ceforanide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (e.g., cefbuperazone, cefinetazole, cefininox, cefotetan, cefoxitin), monobactams (e.g., aztreonam, carumonam, tigemonam), oxacephems, flomoxef, moxalactam), penicillins (e.g., amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine,

penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, ticarcillin), ritipenem, lincosamides (e.g., clindamycin, lincomycin), macrolides (e.g., azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, josamycin, leucomycins, midecamycins, miokamycin, oleandomycin, primycin, rokitamycin, rosaramicin, roxithromycin, spiramycin, troleandomycin), polypeptides (e.g., amphomycin, bacitracin, capreomycin, colistin, enduracidin, enviomycin, fusafungine, gramicidin s, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (e.g., apicycline, chlortetracycline, clomocycline, demeclocycline, doxycycline, guarnecycline, lymecycline, meclocycline, methacycline, minocycline, oxytetracycline, penimepicycline, pipacycline, rolitetracycline, sancycline, tetracycline), cycloserine, mupirocin, and tuberin.

Examples of synthetic antibacterial agents include 2,4-diaminopyrimidines (e.g., brodimoprirn, tetroxoprim, trimethoprim), nitrofurans (e.g., furaltadone, furazolium chloride, nifuradene, nifuratel, nifurfoline, nifurpirinol, nifurprazine, nifurtoinol, nitrofuirantoin), quinolones and analogs (e.g., cinoxacin, ciprofloxacin, clinafloxacin, difloxacin, enoxacin, fleroxacin, flumequine, gatifloxacin, grepafloxacin, levofloxacin, lomefloxacin, miloxacin, moxifloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin, or a fluoroquinolone having the chemical name of 7-[(3R)-3-aminohexahydro-1H-azepin-1-yl]-8-chloro-1-cyclopropyl-6-fluoro-1,4-dihydro-4-oxo-3-quinolinecarboxylic acid monohydrochloride), sulfonamides (e.g., acetyl sulfamethoxypyrazine, benzylsulfamide, chloramines B, chloramines T, dichloramine T, $n^2$-formylsulfisomidine, $n^4$-β-D-glucosylsulfanilamide, mafenide, 4'-(methylsulfamoyl)sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, 4-sulfanilamidosalicylic acid, $n^4$-sulfanilylsulfanilamide, sulfanilylurea, N-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (e.g., acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), clofoctol, hexedine, methenamine, methenamine anhydromethylene citrate, methenamine hippurate, methenamine mandelate, methenamine sulfosalicylate, nitroxoline, taurolidine, and xibomol. In one embodiment, a compostion of the present invention comprises an anti-infective agent selected from the group consiting of cinoxacin, ciprofloxacin, clinafloxacin, difloxacin, enoxacin, fleroxacin, flumequine, gatifloxacin, grepafloxacin, levofloxacin, lomefloxacin, miloxacin, moxifloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin, and a fluoroquinolone having the chemical name of 7-[(3R)-3-aminohexahydro-1H-azepin-1-yl]-8-chloro-1-cyclopropyl-6-fluoro-1,4-dihydro-4-oxo-3-quinolinecarboxylic acid monohydrochloride.

Examples of antiviral agents include Rifampin, Ribavirin, Pleconaryl, Cidofovir, Acyclovir, Pencyclovir, Gancyclovir, Valacyclovir, Famciclovir, Foscarnet, Vidarabine, Amantadine, Zanamivir, Oseltamivir, Resquimod, antiproteases, PEGylated interferon (Pegasys™), anti HIV proteases (e.g. lopinivir, saquinivir, amprenavir, HIV fusion inhibitors, nucleotide HIV RT inhibitors (e.g., AZT, Lamivudine, Abacavir), non-nucleotide HIV RT inhibitors, Doconosol, interferons, butylated hydroxytoluene ("BHT"), and Hypericin.

Examples of biologically-derived antifungal agents include polyenes (e.g., amphotericin B, candicidin, dermostatin, filipin, fungichromin, hachimycin, hamycin, lucensomycin, mepartricin, natamycin, nystatin, pecilocin, perimycin), azaserine, griseofulvin, oligomycins, neomycin undecylenate, pyrrolnitrin, siccanin, tubercidin, and viridin.

Examples of synthetic antifungal agents include allylamines (e.g., butenafine, naftifine, terbinafine), imidazoles (e.g., bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (e.g., tolciclate, tolindate, tolnaftate), triazoles (e.g., fluconazole, itraconazole, saperconazole, terconazole), acrisorcin, amorolfine, biphenamine, bromosalicylchloranilide, buclosamide, calcium propionate, chlorphenesin, ciclopirox, cloxyquin, coparaffinate, diamthazole dihydrochloride, exalamide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, and zinc propionate.

Examples of antiprotozoal agents include polymycin B sulfate, bacitracin zinc, neomycine sulfate (e.g., Neosporin), imidazoles (e.g., clotrimazole, miconazole, ketoconazole), aromatic diamidines (e.g., propamidine isethionate, Brolene), polyhexamethylene biguanide ("PHMB"), chlorhexidine, pyrimethamine (Daraprim®), sulfadiazine, folinic acid (leucovorin), clindamycin, and trimethoprim-sulfamethoxazole.

In one aspect, the anti-infective agent is selected from the group consisting of bacitracin zinc, chloramphenicol,

ciprofloxacin hydrochloride, erythromycin, gatifloxacin, gentamycin sulfate, levofloxacin, moxifloxacin, ofloxacin, sulfacetamide sodium, polymyxin B, tobramycin sulfate, trifluridine, vidarabine, acyclovir, valacyclovir, famcyclovir, foscarnet, ganciclovir, formivirsen, cidofovir, amphotericin B, natamycin, fluconazole, itraconazole, ketoconazole, miconazole, polymyxin B sulfate, neomycin sulfate, clotrimazole, propamidine isethionate, polyhexamethylene biguanide, chlorhexidine, pyrimethamine, sulfadiazine,folinic acid (leucovorin), clindamycin, trimethoprim-sulfamethoxazole, and combinations thereof.

In another aspect, a composition of the present invention can further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108) ), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp. 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006). The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent).

In addition, a composition of the present invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01 % to about 2% by weight). Suitable water-soluble buffering agents that may be employed are sodium carbonate, sodium borate, sodium phosphate, sodium acetate, sodium bicarbonate, etc., as approved by the United States Food and Drug Administration ("US FDA") for the desired route of administration. These agents may be present in amounts sufficient to maintain a pH of the system of between about 2 and about 11. As such, the buffering agent may be as much as about 5% on a weight to weight basis of the total composition. Electrolytes such as, but not limited to, sodium chloride and potassium chloride may also be included in the formulation.

In one aspect, the pH of the composition is in the range from about 4 to about 11. Alternatively, the pH of the composition is in the range from about 5 to about 9, from about 6 to about 9, or from about 6.5 to about 8. In another aspect, the composition comprises a buffer having a pH in one of said pH ranges.

In another aspect, the composition has a pH of about 7. Alternatively, the composition has a pH in a range from about 7 to about 7.5.

In still another aspect, the composition has a pH of about 7.4.

In yet another aspect, a composition also can comprise a viscosity-modifying compound designed to facilitate the administration of the composition into the subject or to promote the bioavailability in the subject. In still another aspect, the viscosity-modifying compound may be chosen so that the composition is not readily dispersed after being administered into the vistreous. Such compounds may enhance the viscosity of the composition, and include, but are not limited to: monomeric polyols, such as, glycerol, propylene glycol, ethylene glycol; polymeric polyols, such as, polyethylene glycol; various polymers of the cellulose family, such as hydroxypropylmethyl cellulose ("HPMC"), carboxymethyl cellulose ("CMC") sodium, hydroxypropyl cellulose ("HPC"); polysaccharides, such as hyaluronic acid and its salts, chondroitin sulfate and its salts, dextrans, such as, dextran 70; water soluble proteins, such as gelatin; vinyl polymers, such as, polyvinyl alcohol, polyvinylpyrrolidone, povidone; carbomers, such as carbomer 934P, carbomer 941, carbomer 940, or carbomer 974P; and acrylic acid polymers. In general, a desired viscosity can be in the range from about 1 to about 400 centipoises ("cps").

In still another aspect of the present disclosure, a method for preparing a composition of the present invention comprises combining: (i) at least a DIGRA, a pharmaceutically acceptable salt thereof, or a pharmaceutically acceptable ester thereof; and (ii) a pharmaceutically acceptable carrier.

In yet another aspect of the present disclosure, a method for preparing a composition of the present invention comprises combining: (i) at least a DIGRA, a pharmaceutically acceptable salt thereof, or a pharmaceutically acceptable ester thereof; and (ii) an anti-inflammatory agent other than said DIGRA, and said pharmaceutically acceptable salt thereof; and (iii) a pharmaceutically acceptable carrier. In one embodiment, such a carrier can be a sterile saline solution

or a physiologically acceptable buffer. In another embodiment, such a carrier comprises a hydrophobic medium, such as a pharmaceutically acceptable oil. In still another embodiment, such as carrier comprises an emulsion of a hydrophobic material and water.

Physiologically acceptable buffers include, a phosphate buffer or a Tris-HCl buffer (comprising tris(hydroxymethyl)aminomethane and HCl). For example, a Tris-HCl buffer having pH of 7.4 comprises 3 g/l of tris(hydroxymethyl) aminomethane and 0.76 g/l of HCl. In yet another aspect, the buffer is 10X phosphate buffer saline ("PBS") or 5X PBS solution.

Other buffers also may be found suitable or desirable in some circumstances, such as buffers based on HEPES (N-{2-hydroxyethyl}peperazine-N'-{2-ethanesulfonic acid}) having $pK_a$ of 7.5 at 25 °C and pH in the range of about 6.8-8.2; BES (N,N-bis{2-hydroxyethyl}2-aminoethanesulfonic acid) having $pK_a$ of 7.1 at 25°C and pH in the range of about 6.4-7.8; MOPS (3-{N-morpholino}propanesulfonic acid) having $pK_a$ of 7.2 at 25°C and pH in the range of about 6.5-7.9; TES (N-tris{hydroxymethyl}-methyl-2-aminoethanesulfonic acid) having $pK_a$ of 7.4 at 25°C and pH in the range of about 6.8-8.2; MOBS (4-{N-morpholino}butanesulfonic acid) having $pK_a$ of 7.6 at 25°C and pH in the range of about 6.9-8.3; DIPSO (3-(N,N-bis{2-hydroxyethyl}amino)-2-hydroxypropane)) having $pK_a$ of 7.52 at 25°C and pH in the range of about 7-8.2; TAPSO (2-hydroxy-3 {tris(hydroxymethyl)methylamino}-1-propanesulfonic acid)) having $pK_a$ of 7.61 at 25°C and pH in the range of about 7-8.2; TAPS ({(2-hydroxy-1,1-bis(hydroxymethyl)ethyl)amino}-1-propanesulfonic acid)) having $pK_a$ of 8.4 at 25°C and pH in the range of about 7.7-9.1; TABS (N-tris(hydroxymethyl)methyl-4-aminobutanesulfonic acid) having $pK_a$ of 8.9 at 25°C and pH in the range of about 8.2-9.6; AMPSO (N-(1,1-dimethyl-2-hydroxyethyl)-3-amino-2-hydroxypropanesulfonic acid)) having $pK_a$ of 9.0 at 25°C and pH in the range of about 8.3-9.7; CHES (2-cyclohexylamino)ethanesulfonic acid) having $pK_a$ of 9.5 at 25°C and pH in the range of about 8.6-10.0; CAPSO (3-(cyclohexylamino)-2-hydroxy-1-propanesulfonic acid) having $pK_a$ of 9.6 at 25°C and pH in the range of about 8.9-10.3; or CAPS (3-(cyclohexylamino)-1-propane sulfonic acid) having $pK_a$ of 10.4 at 25°C and pH in the range of about 9.7-11.1.

In certain embodiments, a composition of the present invention is formulated in a buffer having an acidic pH, such as from about 4 to about 6.8, or alternatively, from about 5 to about 6.8. In such embodiments, the buffer capacity of the composition desirably allows the composition to come rapidly to a physiological pH after being administered into the patient.

It should be understood that the proportions of the various components or mixtures in the following examples may be modified for the appropriate circumstances.

EXAMPLE 1

Two mixtures I and II are made separately by mixing the ingredients listed in Table 1. Five parts (by weight) of mixture I are mixed with one part (by weight) of mixture II for 15 minutes or more. The pH of the combined mixture is adjusted to 6.2-6.4 using 1 N NaOH to yield a composition of the present invention.

Table 1

| Ingredient | Amount |
|---|---|
| Mixture I | |
| Carbopol 934P NF | 0.25 g |
| Purified water | 99.75 g |
| Mixture II | |
| Propylene glycol | 5 g |
| EDTA | 0.1 mg |
| Compound of Formula IV HCl | 0.5 g |

Alternatively, purified water may be substituted with an oil, such as fish-liver oil, peanut oil, sesame oil, coconut oil, sunflower oil, corn oil, or olive oil to produce an oil-based formulation comprising a compound of Formula IV.

EXAMPLE 2

Two mixtures I and II are made separately by mixing the ingredients listed in Table 2. Five parts (by weight) of mixture I are mixed with two parts (by weight) of mixture II for 15 minutes or more. The pH of the combined mixture is adjusted to 6.2-6.4 using 1 N NaOH to yield a composition of the present invention.

Table 2

| Ingredient | Amount |
|---|---|
| Mixture I | |
| Moxifloxacin | 0.2g |
| Diclofenac | 0.3g |
| Carbopol 934P NF | 0.25 g |
| Purified water | 99.25 g |
| Mixture II | |
| Propylene glycol | 5 g |
| EDTA | 0.1 mg |
| Compound of Formula IV | 0.5 g |

Alternatively, purified water may be substituted with an oil, such as fish-liver oil, peanut oil, sesame oil, coconut oil, sunflower oil, corn oil, or olive oil to produce an oil-based formulation comprising a compound of Formula IV.

EXAMPLE 3

Two mixtures I and II are made separately by mixing the ingredients listed in Table 3. Five parts (by weight) of mixture I are mixed with two parts (by weight) of mixture II for 15 minutes or more. The pH of the combined mixture is adjusted to 6.2-6.4 using 1 N NaOH to yield a composition of the present invention.

Table 3

| Ingredient | Amount |
|---|---|
| Mixture I | |
| Gatifloxacin | 0.2g |
| Ciglitazone | 0.2g |
| Carbopol 934P NF | 0.25 g |
| Purified water | 99.35 g |
| Mixture II | |
| Propylene glycol | 3 g |
| Triacetin | 7 g |
| Compound of Formula II | 0.25 g |
| EDTA | 0.1 mg |

EXAMPLE 4:

Two mixtures I and II are made separately by mixing the ingredients listed in Table 4. Five parts (by weight) of mixture I are mixed with one part (by weight) of mixture II for 15 minutes or more. The pH of the combined mixture is adjusted to 6.2-6.4 using 1 N NaOH to yield a composition of the present invention.

Table 4

| Ingredient | Amount |
|---|---|
| Mixture I | |
| Tobramycin sulfate | 0.3g |

(continued)

| Ingredient | Amount |
|---|---|
| Mixture I | |
| Gemfibrozil | 0.3g |
| Carbopol 934P NF | 0.25 g |
| Olive oil | 99.15 g |
| Mixture II | |
| Propylene glycol | 7 g |
| Glycerin | 3 g |
| Compound of Formula III | 1 g |
| Cyclosporine A | 0.5 g |
| HAP (30%) | 0.5 mg |
| Alexidine 2HCl | 1-2 ppm |
| Note: "HAP" denotes hydroxyalkyl phosphonates, such as those known under the trade name Dequest®. | |

EXAMPLE 5:

The ingredients listed in Table 5 are mixed together for at least 15 minutes. The pH of the mixture is adjusted to 6.2-6.4 using 1 N NaOH to yield a composition of the present invention.

Table 5

| Ingredient | Amount (% by weight, except where "ppm" is indicated) |
|---|---|
| Povidone | 1 |
| HAP (30%) | 0.05 |
| Glycerin | 3 |
| Propylene glycol | 3 |
| Compound of Formula IV | 0.5 |
| Trifluridine | 0.1 |
| Tyloxapol | 0.25 |
| BAK | 10-100 ppm |
| Purified water | q.s. to 100 |
| Note: "BAK" denotes benzalkonium chloride. | |

EXAMPLE 6:

The ingredients listed in Table 6 are mixed together for at least 15 minutes. The pH of the mixture is adjusted to 6.2-6.4 using 1 N NaOH to yield a composition of the present invention.

Table 6

| Ingredient | Amount (% by weight, except where "ppm" is indicated) |
|---|---|
| Povidone | 1.5 |
| HAP (30%) | 0.05 |

(continued)

| Ingredient | Amount (% by weight, except where "ppm" is indicated) |
|---|---|
| Glycerin | 3 |
| Propylene glycol | 3 |
| Compound of Formula IV | 0.75 |
| Foscavir | 0.1 |
| Tyloxapol | 0.25 |
| Alexidine 2HCl | 1-2 ppm |
| Purified water | q.s. to 100 |

EXAMPLE 7:

The ingredients listed in Table 7 are mixed together for at least 15 minutes. The pH of the mixture is adjusted to 6.2-6.4 using 1 N NaOH to yield a composition of the present invention.

Table 7

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.25 |
| Amphotericin B | 0.1 |
| Ketorolac | 0.3 |
| Tyloxapol (a surfactant) | 0.25 |
| Alexidine 2HCl | 1-2 ppm |
| Sunflower oil | q.s. to 100 |

EXAMPLE 8:

The ingredients listed in Table 8 are mixed together for at least 15 minutes. The pH of the mixture is adjusted to 6.2-6.4 using 1 N NaOH to yield a composition of the present invention.

Table 8

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.3 |
| Miconazole | 0.2 |
| 15-Deoxy-Δ-12,14-prostaglandin J2 | 0.3 |
| Tyloxapol (a surfactant) | 0.25 |
| Alexidine 2HCl | 1-2 ppm |

(continued)

| Ingredient | Amount (% by weight, except where "ppm" is indicated) |
|---|---|
| Purified water | q.s. to 100 |

EXAMPLE 9:

The ingredients listed in Table 9 are mixed together for at least 15 minutes. The pH of the mixture is adjusted to 6.2-6.4 using 1 N NaOH to yield a composition of the present invention.

Table 9

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.5 |
| Bacitracin zinc | 0.2 |
| Flurbiprofen | 0.2 |
| Levofloxacin | 0.3 |
| Tyloxapol (a surfactant) | 0.25 |
| Alexidine 2HCl | 1-2 ppm |
| Corn oil | q.s. to 100 |

EXAMPLE 10:

The ingredients listed in Table 10 are mixed together for at least 15 minutes. The pH of the mixture is adjusted to 6.2-6.4 using 1 N NaOH to yield a composition of the present invention.

Table 10

| Ingredient | Amount (% by weight, except where "ppm" is indicated) |
|---|---|
| CMC (MV) | 0.5 |
| HAP (30%) | 0.05 |
| Glycerin | 3 |
| Propylene glycol | 3 |
| Compound of Formula IV | 0.75 |
| Moxifloxacin | 0.2 |
| 15-Deoxy-$\Delta$-12,14-prostaglandin J2 | 0.3 |
| Clotrimazole | 0.2 |
| Tyloxapol (a surfactant) | 0.25 |
| Alexidine 2HCl | 1-2 ppm |
| Purified water | q.s. to 100 |

In another aspect of the present disclosure, a DIGRA, a pharmaceutically acceptable salt thereof, or a pharmaceutically acceptable ester thereof, and an anti-inflammatory agent are incorporated into a formulation for topical administration, systemic administration, periocular injection, or intravitreal injection. An injectable intravitreal formulation

can desirably comprise a carrier that provides a sustained-release of the active ingredients, such as for a period longer than about 1 week (or longer than about 1, 2, 3, 4, 5, or 6 months). In certain examples, the sustained-release formulation desirably comprises a carrier that is insoluble or only sparingly soluble in the vitreous. Such a carrier can be an oil-based liquid, emulsion, gel, or semisolid. Examples of oil-based liquids include castor oil, peanut oil, olive oil, coconut oil, sesame oil, cottonseed oil, corn oil, sunflower oil, fish-liver oil, arachis oil, and liquid paraffin.

A compound or composition of the present invention can be injected intravitreally, for example through the pars plana of the ciliary body, to treat or prevent glaucoma or progression thereof using a fine-gauge needle, such as 25-30 gauge. Typically, an amount from about 25 μl to about 100 μl of a composition comprising a DIGRA, a pharmaceutically acceptable salt thereof, or a pharmaceutically acceptable ester thereof is administered into a patient. A concentration of such DIGRA, or pharmaceutically acceptable salt thereof is selected from the ranges disclosed above.

In still another aspect of the present disclosure, a DIGRA, a pharmaceutically acceptable salt thereof, or a pharmaceutically acceptable ester thereof is incorporated into an ophthalmic device or system that comprises a biodegradable material, and the device is injected or implanted into a subject to provide a long-term (e.g., longer than about 1 week, or longer than about 1, 2, 3, 4, 5, or 6 months) treatment or prevention of glaucoma or progression thereof. Such a device system may be injected or implanted by a skilled physician in the subject's ocular or periocular tissue.

In still another aspect of the present disclosure, a method for treating or preventing glaucoma or progression thereof, comprises: (a) providing a composition comprising a DIGRA, a pharmaceutically acceptable salt thereof, or a pharmaceutically acceptable ester thereof; and (b) administering to a subject an effective amount of the composition at a frequency sufficient to treat or prevent said glaucoma or progression thereof.

According to the present invention, such glaucoma results from chronic inflammation.

In another example, the composition for use in any of the foregoing method further comprises an anti-inflammatory agent other than a DIGRA, a pharmaceutically acceptable salt thereof, and a pharmaceutically acceptable ester thereof. Such an anti-inflammatory agent is selected from those disclosed above. The concentrations of the DIGRA, a pharmaceutically acceptable salt thereof, a pharmaceutically acceptable ester thereof, and the anti-inflammatory agent are selected to be in the ranges disclosed above.

In another aspect of the present disclosure, a composition of the present invention is administered intravitreally or periocularly. In still another aspect of the present disclosure, a composition of the present invention is incorporated into an ophthalmic implant system or device, and the implant system or device is surgically implanted in the vitreous cavity or in the back of the eye of the patient for the sustained or long-term release of the active ingredient or ingredients. A typical implant system or device suitable for use in such a method comprises a biodegradable matrix with the active ingredient or ingredients impregnated or dispersed therein. Examples of ophthalmic implant systems or devices for the sustained-release of an active ingredient are disclosed in U.S. Patents 5,378,475; 5,773,019; 5,902,598; 6,001,386; 6,051,576; and 6,726,918.

In yet another aspect of the present disclosure, a composition of the present invention is administered once a day, several (e.g., twice, three, four, or more) times a day, once a week, once a month, once a year, twice a year, four times a year, or at a suitable frequency that is determined to be appropriate for treating or preventing glaucoma or progression thereof.

COMBINATION THERAPY

The composition of the present invention can be used with other therapeutic and adjuvant or prophylactic agents commonly used to reduce, treat, or prevent (a) an increase of intraocular pressure, (b) a loss of retinal ganglion cells, or (c) both, thus providing an enhanced overall treatment or enhancing the effects of the other therapeutic agents, prophylactic agents, and adjunctive agents used to treat and manage the different types of glaucoma. Therapeutic agents used to treat narrow angle or acute congestive glaucoma include, for example, physostigmine salicylate and pilocarpine nitrate. Adjunctive therapy used in the management of narrow angle glaucoma includes, for example, the intravenous administration of a carbonic anhydrase inhibitor such as acetozolamide to reduce the secretion of aqueous humor, or of an osmotic agent such as mannitol or glycerin to induce intraocular dehydration. Therapeutic agents used to manage wide angle or chronic simple glaucoma and secondary glaucoma include, for example, prostaglandin analogs, such as Xalatan® and Lumigan®, β-adrenergic antagonists such as timolol maleate, α-adrenergic agonists, such as brimonidine and apraclonidine, muscarinic cholinergic agents (such as pilocarpine or carbachol), and carbonic anhydrase inhibitors, such as Dorzolamide (Trusopt® or Cosopt®) or brizolamide (Azopt®). Other therapeutic agents used to manage glaucoma include the inhibitors of acetylcholinesterase such as Echothiophate (phospholine iodide).

High doses may be required for some currently used therapeutic agents to achieve levels to effectuate the target response, but may often be associated with a greater frequency of dose-related adverse effects. Thus, combined use of the compounds or compositions of the present invention with agents commonly used to treat glaucoma allows the use of relatively lower doses of such other agents, resulting in a lower frequency of adverse side effects associated with long-term administration of such therapeutic agents. Thus, another indication of the compounds or compositions

in this invention is to reduce adverse side effects of prior-art drugs used to treat glaucoma, such as the development of cataracts with long-acting anticholinesterase agents including demecarium, echothiophate, and isoflurophate.

COMPARISON OF SIDE EFFECTS OF GLUCOCORTICOIDS AND DIGRAS

Side effects of glucocorticoids and DIGRAs may be compared in their use to treat an exemplary inflammation.

In one aspect, a level of at least an adverse side effect is determined in vivo or in vitro. For example, a level of said at least an adverse side effect is determined in vitro by performing a cell culture and determining the level of a biomarker associated with said side effect. Such biomarkers can include proteins (e.g., enzymes), lipids, sugars, and derivatives thereof that participate in, or are the products of, the biochemical cascade resulting in the adverse side effect. Representative in vitro testing methods are further disclosed hereinbelow.

In another embodiment, a level of said at least an adverse side effect is determined in vivo at about one day after said glucocorticoid or DIGRA (or a pharmaceutically acceptable salt thereof, or a pharmaceutically acceptable ester thereof) is first administered to, and are present in, said subject. In another embodiment, a level of said at least an adverse side effect is determined about 14 days after said composition is first administered to, and are present in, said subject. In still another embodiment, a level of said at least an adverse side effect is determined about 30 days after said composition is first administered to, and are present in, said subject. Alternatively, a level of said at least an adverse side effect is determined about 2, 3, 4, 5, or 6 months after said compounds or compositions are first administered to, and are present in, said subject.

In another aspect, said glucocorticoid used to treat said exemplary inflammation is administered to said subject at a dose and a frequency sufficient to produce a beneficial effect on said inflammation equivalent to a compound or composition of the present invention after about the same elapsed time.

One of the most frequent undesirable actions of a glucocorticoid therapy (such as anti-inflammation therapy) is steroid diabetes. The reason for this undesirable condition is the stimulation of gluconeogenesis in the liver by the induction of the transcription of hepatic enzymes involved in gluconeogenesis and metabolism of free amino acids that are produced from the degradation of proteins (catabolic action of glucocorticoids). A key enzyme of the catabolic metabolism in the liver is the tyrosine aminotransferase ("TAT"). The activity of this enzyme can be determined photometrically from cell cultures of treated rat hepatoma cells. Thus, the gluconeogenesis by a glucocorticoid can be compared to that of a DIGRA by measuring the activity of this enzyme. For example, in one procedure, the cells are treated for 24 hours with the test substance (a DIGRA or glucocorticoid), and then the TAT activity is measured. The TAT activities for the selected DIGRA and glucocorticoid are then compared. Other hepatic enzymes can be used in place of TAT, such as phosphoenolpyruvate carboxykinase, glucose-6-phosphatase, or fructose-2,6-biphosphatase. Alternatively, the levels of blood glucose in an animal model may be measured directly and compared for individual subjects that are treated with a glucocorticoid for a selected condition and those that are treated with a DIGRA for the same condition.

Another undesirable result of glucocorticoid therapy is GC-induced cataract. The cataractogenic potential of a compound or composition may be determined by quantifying the effect of the compound or composition on the flux of potassium ions through the membrane of lens cells (such as mammalian lens epithelial cells) in vitro. Such an ion flux may be determined by, for example, electrophysiological techniques or ion-flux imaging techniques (such as with the use of fluorescent dyes). An exemplary in-vitro method for determining the cataractogenic potential of a compound or composition is disclosed in U.S. Patent Application Publication 2004/0219512.

Still another undesirable result of glucocorticoid therapy is hypertension. Blood pressure of similarly matched subjects treated with glucocorticoid and DIGRA for an inflammatory condition may be measured directly and compared.

Yet another undesirable result of glucocorticoid therapy is increased IOP. IOP of similarly matched subjects treated with glucocorticoid and DIGRA for an inflammatory condition may be measured directly and compared.

A glucocorticoid that is used for comparative testing, for example, in the foregoing procedures can be selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortarnate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, combinations thereof, and mixtures thereof. In one embodiment, said glucocorticoid is selected from the group consisting of dexamethasone, prednisone, prednisolone, methylprednisolone, medrysone, triamcinolone, loteprednol etabonate, physiologically acceptable salts thereof, combinations thereof, and mixtures thereof. In another embodiment, said glucocorticoid is acceptable for ophthalmic uses.

TESTING 1: Comparison of the DIGRA Having Formula IV With Two Corticosteroids and One NSAID in Treating Anterior-Segment Inflammation

1. INTRODUCTION

Inflammatory processes are multidimensional in origin, and are characterized by complex cellular and molecular events involving numerous components all of which have not been identified. Prostaglandins are among these mediators and play an important role in certain forms of ocular inflammation. Paracentesis of the anterior chamber in the rabbit eye induces inflammatory reaction due to the disruption of the blood-aqueous barrier ("BAB"), which is mediated, at least in part, by prostaglandin $E_2$ [References 1 -3 below]. Intraocular or topical administration of $PGE_2$ disrupts the BAB. [Reference 4, below] The treatment schedule adopted in this study was similar to the clinical NSAIDs (Ocufen) treatment schedule used by surgeons for patients before cataract surgery. We investigated a dissociated glucocorticoid receptor agonist ("BOL-303242-X", compound having Formula IV above) at different doses on rabbit paracentesis model evaluating aqueous biomarkers levels, and iris-ciliary body MPO activity in comparison with vehicle, dexamethasone, loteprednol and flurbiprofen.

2. METHODS

2.1 Drugs and Materials

2.1.1. Test articles

BOL-303242-X (0.1 %, 0.5% and 1% topical formulations), lot 2676-MLC-107, Bausch & Lomb Incorporated ("B&L") Rochester, USA.

Vehicle (10% PEG 3350; 1% Tween 80; phosphate buffer pH 7.00), lot 2676-MLC-107, B&L Rochester, USA.

Visumetazone® (0.1 % Dexamethasone topical formulation), lot T253, Visufarma, Rome, Italy.

Lotemax® (0.5% Loteprednol topical formulation), lot 078061, B&L IOM, Macherio, Italy.

Ocufen® (0.03% Flurbiprofen topical formulation), lot E45324, Allergan, Westport, Ireland.

2.2 Animals

Species: Rabbit

Breed: New Zealand

Source: Morini (Reggio Emila, Italy)

Sex: Male

Age at Experimental Start: 10 weeks.

Weight Range at Experimental Start: 2.0-2.4 Kg

Total Number of Animals: 28

Identification: Ear tagged with an alphanumeric code (i.e. A1 means test article A and animal 1).

Justification: The rabbit is a standard non-rodent species used in pharmacodynamic studies. The number of animals used in this study is, in judgment of the investigators involved, the minimum number necessary to properly perform this type of study and it is consistent with world wide regulatory guidelines.

Acclimation/Quarantine: Following arrival, a member of the veterinary staff assessed animals as to their general health. Seven days elapsed between animal receipt and the start of experiment in order to acclimate animals to the laboratory environment and to observe them for the development of infection disease.

Animal Husbandry: All the animals were housed in a cleaned and disinfected room, with a constant temperature

(22±1°C), humidity (relative, 30%) and under a constant light-dark cycle (light on between 8.00 and 20.00). Commercial food and tap water were available *ad libitum*. Their body weights were measured just before the experiment (Table T-1). All the animals had a body weight inside the central part of the body weight distribution curve (10%). Four rabbits were replaced with animals of similar age and weight from the same vendor because three of them showed signs of ocular inflammation and one was dead upon arrival.

Animals Welfare Provisions: All experiments were carried out according to the ARVO (Association for Research in Vision and Ophthalmology) guidelines on the use of animals in research. No alternative test system exists which have been adequately validated to permit replacement of the use of live animals in this study. Every effort has been made to obtain the maximum amount of information while reducing to a minimum the number of animals required for this study. To the best of our knowledge, this study is not unnecessary or duplicative. The study protocol was reviewed and approved by the Institutional Animal Care and Use Committee (IACUC) of the University of Catania and complies with the acceptable standards of animal welfare care.

2.3 Experimental Preparations

2.3.1 Study design and randomization

Twenty-eight rabbits were randomly allocated into 7 groups (4 animals/each) as shown in the table below.

Table 8

| Group | No of rabbits | Treatment | | Observations and measurements | Termination and assays |
|---|---|---|---|---|---|
| I | 4 | CTR | 50 μl drops at 180, 120, 90, and 30 min prior to first paracentesis, and at 15, 30, 90 min after the first paracentesis. | Clinical observations and pupillary diameter at 180 and 5 min before the first paracentesis, and at 5 min before the second paracentesis.<br>Paracentesis at 0 and | Termination immediately after the second paracentesis.<br>Aqueous humor collected for $PGE_2$, protein, leukocytes and $LTB_4$ |
| II | 4 | 1% BOL | | | |
| III | 4 | 0.5% BOL | | | |
| IV | 4 | 0.1% BOL | | | |
| V | 4 | 0.5% LE | | | |
| VI | 4 | 0.1% Dex | | 2 hours. | measurements.<br>Iris-ciliary body collected for MPO activity measurement. |
| VII | 4 | 0.03% F | | | |
| CTR=vehicle; BOL=BOL-303242-X; LE=loteprednol etabonate; Dex=dexamethasone; F=flurbiprofen | | | | | |

To each test article was randomly assigned a letter from A to G

A = vehicle (10% PEG3350/1% Tween 80/PB pH 7.00)

B = Ocufen (Flurbiprofen 0.03%)

C = Visumetazone (Dexamethasone 0.1 %)

D = Lotemax (Loteprednol etabonate 0.5%)

E = BOL-303242-X 0.1% (1 mg/g)

F = BOL-303242-X 0.5% (5 mg/g)

G = BOL-303242-X 1% (10 mg/g)

2.3.2 Reagent preparation for MPO assay

2.3.2.1 Phosphate buffer (50mM; pH=6)

3.9g of $NaH_2PO_4$ $2H_2O$ were dissolved in a volumetric flask to 500ml with water. The pH was adjusted to pH=6 with 3N NaOH.

2.3.2.2 Hexa-decyl-trimethyl-ammonium bromide (0.5%)

0.5g of hexa-decyl-trimethyl-ammonium bromide was dissolved in 100ml phosphate buffer.

2.3.2.3 o-dianisidine 2HCl (0.0167%) / $H_2O_2$ (0.0005%) solution

The solution was prepared freshly. Ten microliters of $H_2O_2$ (30 wt.%) were diluted to 1ml with water (solution A). 7.5mg o-dianisidine 2HCl was dissolved in 45ml of phosphate buffer and 75 μl of solution A were added.

2.4 Experimental Protocols

2.4.1 Animals treatment and sample collection

Each rabbit was placed in a restraint device and tagged with the alphanumeric code. The formulations were instilled (50 μl) into the conjunctival sac of both eyes 180, 120, 90 and 30 min before the first paracentesis; then 15, 30, 90 min after the first paracentesis. To perform the first paracentesis the animals were anaesthetized by intravenous injection of 5mg/kg Zoletil® (Virbac; 2.5mg/kg tiletamine HCl and 2.5mg/kg zolazepam HCl) and one drop of local anesthetic (Novesina®, Novartis) was administered to the eye. Anterior chamber paracentesis was performed with a 26 G needle attached to a tuberculin syringe; the needle was introduced into the anterior chamber through the cornea, taking care not to damage the tissues. Two hours after the first paracentesis, the animals were sacrificed with 0.4 ml Tanax® (Intervet International B.V.) and the second paracentesis was performed. About 100 μl of aqueous humor were removed at the second paracentesis. Aqueous humor was immediately split in four aliquots and stored at -80°C until analysis. Then both eyes were enucleated and the iris-ciliary body was carefully excised, placed in polypropylene tubes, and stored at -80 °C until analysis.

2.4.2 Pupillary diameter measurement

The pupillary diameter of both eyes was measured with a Castroviejo caliper 180 min and 5 min before the first paracentesis and 5 min before the second paracentesis.

2.4.3 Clinical evaluation

The clinical evaluation of both eyes was performed by a slit lamp (4179-T; Sbisà, Italy) at 180 min and 5 min before the first paracentesis and 5 min before the second paracentesis. The clinical score was assigned according to the following scheme:

0 = normal
1 = discrete dilatation of iris and conjunctival vessels
2 = moderate dilatation of iris and conjunctival vessels
3 = intense iridal hyperemia with flare in the anterior chamber
4 = intense iridal hyperemia with flare in the anterior chamber and presence of fibrinous exudates.

2.4.4 Prostaglandin $E_2$ ($PGE_2$) measurement

For the quantitative determination of $PGE_2$ in the aqueous humor we used the $PGE_2$ Immunoassay kit (R&D Systems; Cat.No. KGE004; Lot.No. 240010). Eleven microliters or 16μl of aqueous humor were diluted to 110μl or 160μl with the calibrator diluent solution provided with the kit. One hundred microliters of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.5 Protein measurement

For protein concentration determination in the aqueous humor we used the Protein Quantification Kit (Fluka; Cat.No. 77371; Lot.No. 1303129). Five microliters of aqueous humor were diluted to 100μl with water. Twenty microliters of samples and of standards were load into a 96-well plate and recorded in a plate layout. Samples were treated following the assay procedure described in the kit. A microplate reader (GDV, Italy; model DV 990 B/V6) set at 670 nm was used for making the calibration and analyzing the samples.

2.4.6 Leukocytes (PMN) measurement

For the determination of the number of leukocytes we used a haemocytometer (Improved Neubauer Chamber; Bright-line, Hausser Scientific) and a Polyvar 2 microscope (Reichert-Jung).

2.4.7 Leukotriene $B_4$ ($LTB_4$) measurement

For the quantitative determination of $LTB_4$ concentration in the aqueous humor we used the $LTB_4$ Immunoassay kit (R&D Systems; Cat.No. KGE006; Lot.No. 243623). 11μl of aqueous humor were diluted to 110μl with the calibrator diluent solution provided with the kit. 100 μl of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.8 Myeloperoxidase (MPO) measurement

The activity of MPO was measured as previously described by Williams et al.[5] The iris-ciliary bodies were carefully dried, weighed and immersed in 1ml of hexa-decyl-trimethyl-ammonium bromide solution. Then, the samples were sonicated for 10 sec on ice by a ultrasound homogenizer (HD 2070, Bandelin electronic), freeze-thawed three times, sonicated for 10 sec and centrifuged at 14,000 g for 10 min to remove cellular debris. An aliquot of the supernatant (40-200μl) was diluted to 3ml with the *o*-dianisidine 2HCl / $H_2O_2$ solution. The change in absorbance at 460nm was continuously monitored for 5 min by a spectrophotometer (UV/Vis Spectrometer Lambda EZ 201; Perkin Elmer). The slope of the line (Δ/min) was determined for each sample and used to calculate the number of units of MPO in the tissue as follows:

$$MPOunit/g = \frac{(\Delta/\min)\cdot 10^6}{\varepsilon \cdot \mu l \cdot mg}$$

were $\varepsilon = 11.3\ mM^{-1}$.

Values were expressed as units of MPO/g of tissue.

2.5 Data Analysis

Pupillary diameter, $PGE_2$, protein, PMN, and MPO were expressed as mean ± SEM. Statistical analysis was performed using one way ANOVA followed by a Newman-Keuls post hoc test. Clinical score was expressed as % of eyes and the statistical analysis was performed using Kruskal-Wallis followed by a Dunn post hoc test. $P<0.05$ was considered statistically significant in both cases. Prism 4 software (GraphPad Software, Inc.) was used for the analysis and graphs.

3. RESULTS

3.1 Pupillary diameter measurement

The raw data are displayed in Tables T-2 and T-3. No statistical significance was found between the CRT and all the treatments.

3.2 Clinical evaluation

The raw data are displayed in Tables T-4 and T-5. Only the 0.5% LE group showed a significant difference versus CTR ($p<0.05$).

3.3 Prostaglandin $E_2$ ($PGE_2$) measurement

The raw data are displayed in Tables T-6 and T-7. The treatments 0.03% F, 0.5% LE, 0.1% BOL, and 0.5% BOL were statistically significant versus CTR ($p<0.05$).

3.4 Protein measurement

The raw data are displayed in Tables T-8 and T-9. It has been found a statistical significance for the treatments 0.03% F and 1% BOL vs CTR with $p<0.001$, and 0.5% BOL vs CTR with $p<0.05$.

3.5 Leukocytes (PMN) measurement

The raw data are displayed in Tables T-10 and T-11. All the treatments were statistically significant vs CTR ($p<0.001$).

3.6 Leukotriene $B_4$ ($LTB_4$) measurement

All samples were under the limit of quantification (about 0.2 ng/ml) of the assay.

3.7 Myeloperoxidase (MPO) measurement

The raw data are displayed in Tables T-12 and T-13. It has been found a statistical significance for the all the treatments vs CTR with $p<0.01$ for 0.03% F, and $p<0.001$ for 0.1% Dex, 0.5% LE, 0.1% BOL, 0.5% BOL and 1% BOL.

4. DISCUSSION

The preliminary conclusions from the data generated are:

- BOL-303242-X is active in this model.
- There was not a large difference between these concentrations of BOL-303242-X and NSAID and steroid positive controls.

There was not a profound dose-response for BOL-303242-X, perhaps because we are at either maximal efficacy or maximal drug exposure at these doses. However, the results show that BOL-303242-X is as effective an anti-inflammatory drug as some of the commonly accepted prior-art steroids or NSAID. Some other very preliminary data (not shown) suggest that BOL-303242-X does not have some of the side effects of corticosteroids.

5. REFERENCES

1. Eakins KE (1977). Prostaglandin and non prostaglandin-mediated breakdown of the blood-aqueous barrier. Exp. Eye Res., Vol. 25, 483-498.
2. Neufeld AH, Sears ML (1973). The site of action of prostaglandin E2 on the disruption of the blood-aqueous barrier in the rabbit eye. Exp. Eye Res., Vol. 17, 445-448.
3. Unger WG, Cole DP, Hammond B (1975). Disruption of the blood-aqueous barrier following paracentesis in the rabbit. Exp. Eye Res., Vol. 20, 255-270.
4. Stjernschantz J (1984). Autacoids and Neuropeptides. In: Sears, ML (ed.) Pharmacology of the Eye. Springer-Verlag, New York, pp. 311-365.
5. Williams RN, Paterson CA, Eakins KE, Bhattacherjee P (1983) Quantification of ocular inflammation: evaluation of polymorphonuclear leukocyte infiltration by measuring myeloperoxidase activity. Curr. Eye Res., Vol. 2, 465-469.

Table T-1: Rabbit body weight measured just before the experiment

| Rabbit ID | Sex | Body weight (g) |
|---|---|---|
| A1 | M | 2090 |
| A2 | M | 2140 |
| A3 | M | 2100 |
| A4 | M | 2320 |
| B1 | M | 2270 |
| B2 | M | 2190 |
| B3 | M | 2340 |
| B4 | M | 2300 |
| C1 | M | 2160 |
| C2 | M | 2160 |
| C3 | M | 2280 |
| C4 | M | 2400 |
| D1 | M | 2220 |
| D2 | M | 2200 |
| D3 | M | 2180 |
| D4 | M | 2260 |
| E1 | M | 2170 |
| E2 | M | 2330 |
| E3 | M | 2350 |
| E4 | M | 2300 |
| F1 | M | 2190 |
| F2 | M | 2240 |
| F3 | M | 2120 |
| F4 | M | 2200 |
| G1 | M | 2410 |
| G2 | M | 2270 |
| G3 | M | 2310 |
| G4 | M | 2130 |
| Mean ± S.D. | | 2236.8 ± 89.2 |

Table T-2 Raw data of pupillary diameter at -180 min (basal), -5 min (5 min before the first paracentesis) and at +115 min (5 min before the second paracentesis), and calculated difference between the value at +115 min and the value at -180 min.

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| CTR | A1 | DX | 6.0 | 5.5 | 4.0 | -2.0 |
| | | SX | 5.5 | 5.5 | 4.0 | -1.5 |
| | A2 | DX | 6.0 | 6.5 | 4.5 | -1.5 |
| | | SX | 6.0 | 6.5 | 5.0 | -1.0 |
| | A3 | DX | 6.5 | 6.5 | 5.0 | -1.5 |
| | | SX | 6.5 | 6.5 | 5.0 | -1.5 |
| | A4 | DX | 6.0 | 6.5 | 5.0 | -1.0 |
| | | SX | 6.0 | 6.5 | 5.0 | -1.0 |
| | | | | | | |

(continued)

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| 0.03% F | B1 | DX | 5.0 | 6.0 | 4.0 | -1.0 |
| | | SX | 5.0 | 6.0 | 3.5 | -1.5 |
| | B2 | DX | 7.0 | 6.5 | 5.5 | -1.5 |
| | | SX | 6.0 | 7.0 | 5.0 | -1.0 |
| | B3 | DX | 6.0 | 6.5 | 4.5 | -1.5 |
| | | SX | 6.0 | 6.5 | 6.0 | 0.0 |
| | B4 | DX | 5.5 | 6.0 | 5.5 | 0.0 |
| | | SX | 6.0 | 5.5 | 5.0 | -1.0 |
| 0.1% Dex | C1 | DX | 6.0 | 5.5 | 5.5 | -0.5 |
| | | SX | 7.0 | 6.5 | 5.5 | -1.5 |
| | C2 | DX | 5.5 | 6.5 | 6.0 | 0.5 |
| | | SX | 5.5 | 6.0 | 5.5 | 0.0 |
| | C3 | DX | 6.5 | 6.0 | 4.5 | -2.0 |
| | | SX | 6.5 | 6.5 | 5.0 | -1.5 |
| | C4 | DX | 6.5 | 7.0 | 6.0 | -0.5 |
| | | SX | 7.0 | 7.5 | 6.5 | -0.5 |
| | | | | | | |
| 0.5% LE | D1 | DX | 6.0 | 6.0 | 4.5 | -1.5 |
| | | SX | 6.0 | 6.0 | 5.0 | -1.0 |
| | D2 | DX | 6.5 | 6.5 | 5.5 | -1.0 |
| | | SX | 6.5 | 6.5 | 5.5 | -1.0 |
| | D3 | DX | 6.0 | 6.0 | 6.0 | 0.0 |
| | | SX | 6.5 | 6.5 | 6.0 | -0.5 |
| | D4 | DX | 6.5 | 6.5 | 6.0 | -0.5 |
| | | SX | 6.5 | 6.5 | 5.0 | -1.5 |
| | | | | | | |
| 0.1% BOL | E1 | DX | 6.5 | 6.5 | 5.0 | -1.5 |
| | | SX | 6.5 | 6.5 | 6.0 | -0.5 |
| | E2 | DX | 6.5 | 7.0 | 5.0 | -1.5 |
| | | SX | 6.5 | 7.0 | 6.0 | -0.5 |
| | E3 | DX | 7.0 | 7.0 | 6.0 | -1.0 |
| | | SX | 7.5 | 7.5 | 6.5 | -1.0 |
| | E4 | DX | 7.0 | 6.5 | 5.5 | -1.5 |
| | | SX | 7.0 | 7.0 | 5.5 | -1.5 |
| | | | | | | |

(continued)

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| 0.5% BOL | F1 | DX | 8.0 | 8.0 | 6.5 | -1.5 |
| | | SX | 8.0 | 8.0 | 6.5 | -1.5 |
| | F2 | DX | 7.0 | 7.0 | 6.5 | -0.5 |
| | | SX | 7.0 | 7.0 | 6.0 | -1.0 |
| | F3 | DX | 7.5 | 7.5 | 7.0 | -0.5 |
| | | SX | 8.0 | 8.0 | 7.0 | -1.0 |
| | F4 | DX | 7.0 | 7.0 | 6.0 | -1.0 |
| | | SX | 7.5 | 7.0 | 6.5 | -1.0 |
| | | | | | | |
| 1 % BOL | G1 | DX | 6.0 | 6.0 | 5.5 | -0.5 |
| | | SX | 6.5 | 6.5 | 5.0 | -1.5 |
| | G2 | DX | 6.0 | 6.5 | 5.0 | -1.0 |
| | | SX | 6.0 | 6.5 | 5.0 | -1.0 |
| | G3 | DX | 6.5 | 7.0 | 5.5 | -1.0 |
| | | SX | 6.5 | 7.0 | 5.0 | -1.5 |
| | G4 | DX | 6.5 | 6.5 | 6.0 | -0.5 |
| | | SX | 6.5 | 6.0 | 6.0 | -0.5 |

Table T-3 Difference between the value of pupillary diameter at T3=+115 min (5 min before the second paracentesis) and the value at T1=-180 min (basal) (Mean ± SEM).

| Treatment | Rabbit Group ID | Mean (mm) Δ(T3 - T1) | SEM | n |
|---|---|---|---|---|
| CTR | A | -1.4 | 0.12 | 8 |
| 0.03% F | B | -0.9 | 0.22 | 8 |
| 0.1% Dex | C | -0.8 | 0.30 | 8 |
| 0.5% LE | D | -0.9 | 0.18 | 8 |
| 0.1% BOL | E | -1.1 | 0.16 | 8 |
| 0.5% BOL | F | -1.0 | 0.13 | 8 |
| 1 % BOL | G | -0.9 | 0.15 | 8 |

Table T-4 Raw data of clinical score at -180 min (basal), -5 min (5 min before the first paracentesis) and at +115 min (5 min before the second paracentesis).

| Treatment | Rabbit ID | Eye | Clinical Score | | |
|---|---|---|---|---|---|
| | | | -180 min | -5 min | +115 min |
| CTR | A1 | DX | 0 | 1 | 3 |
| | | SX | 0 | 1 | 3 |

(continued)

| Treatment | Rabbit ID | Eye | Clinical Score | | |
|---|---|---|---|---|---|
| | | | -180 min | -5 min | +115 min |
| | A2 | DX | 0 | 0 | 2 |
| | | SX | 0 | 0 | 2 |
| | A3 | DX | 0 | 0 | 3 |
| | | SX | 0 | 0 | 3 |
| | A4 | DX | 0 | 0 | 3 |
| | | SX | 0 | 0 | 3 |
| | | | | | |
| 0.03% F | B1 | DX | 0 | 0 | 2 |
| | | SX | 0 | 0 | 2 |
| | B2 | DX | 0 | 0 | 2 |
| | | SX | 0 | 0 | 2 |
| | B3 | DX | 0 | 0 | 2 |
| | | SX | 0 | 0 | 2 |
| | B4 | DX | 0 | 0 | 2 |
| | | SX | 0 | 0 | 2 |
| 0.1% Dex | C1 | DX | 0 | 0 | 1 |
| | | SX | 0 | 0 | 1 |
| | C2 | DX | 0 | 0 | 1 |
| | | SX | 0 | 0 | 1 |
| | C3 | DX | 0 | 1 | 3 |
| | | SX | 0 | 1 | 3 |
| | C4 | DX | 0 | 0 | 1 |
| | | SX | 0 | 0 | 1 |
| | | | | | |
| 0.5% LE | D1 | DX | 0 | 0 | 2 |
| | | SX | 0 | 0 | 2 |
| | D2 | DX | 0 | 0 | 1 |
| | | SX | 0 | 0 | 1 |
| | D3 | DX | 0 | 0 | 1 |
| | | SX | 0 | 0 | 1 |
| | D4 | DX | 0 | 0 | 1 |
| | | SX | 0 | 0 | 1 |
| | | | | | |
| 0.1% BOL | E1 | DX | 0 | 0 | 2 |
| | | SX | 0 | 0 | 2 |
| | E2 | DX | 0 | 0 | 2 |
| | | SX | 0 | 0 | 2 |

(continued)

| Treatment | Rabbit ID | Eye | Clinical Score | | |
|---|---|---|---|---|---|
| | | | -180 min | -5 min | +115 min |
| | E3 | DX | 0 | 0 | 2 |
| | | SX | 0 | 0 | 2 |
| | E4 | DX | 0 | 0 | 3 |
| | | SX | 0 | 0 | 3 |
| | | | | | |
| 0.5% BOL | F1 | DX | 0 | 0 | 2 |
| | | SX | 0 | 0 | 2 |
| | F2 | DX | 0 | 0 | 1 |
| | | SX | 0 | 0 | 2 |
| | F3 | DX | 0 | 0 | 1 |
| | | SX | 0 | 0 | 1 |
| | F4 | DX | 0 | 0 | 2 |
| | | SX | 0 | 0 | 2 |
| | | | | | |
| 1% BOL | G1 | DX | 0 | 0 | 2 |
| | | SX | 0 | 0 | 2 |
| | G2 | DX | 0 | 0 | 2 |
| | | SX | 0 | 0 | 2 |
| | G3 | DX | 0 | 0 | 2 |
| | | SX | 0 | 0 | 2 |
| | G4 | DX | 0 | 0 | 2 |
| | | SX | 0 | 0 | 2 |

Table T-5 Clinical score expressed as percentage of eyes at -180 min (basal), -5 min (5 min before the first paracentesis) and at +115 min (5 min before the second paracentesis).

| Treatment | Rabbit Group ID | N (eyes) | Score (%) | | | | |
|---|---|---|---|---|---|---|---|
| | | | 0 | -- | 2 | 3 | 4 |
| | | | | | -180 min | | |
| CTR | A | 8 | 100 | -- | -- | -- | -- |
| 0.03% F | B | 8 | 100 | -- | -- | -- | -- |
| 0.1% Dex | C | 8 | 100 | -- | -- | -- | -- |
| 0.5% LE | D | 8 | 100 | -- | -- | -- | -- |
| 0.1% BOL | E | 8 | 100 | -- | -- | -- | -- |
| 0.5% BOL | F | 8 | 100 | -- | -- | -- | -- |
| 1% BOL | G | 8 | 100 | -- | -- | -- | -- |

(continued)

| | | | | | | | |
|---|---|---|---|---|---|---|---|
| | | | | -5 min | | | |
| CTR | A | 8 | 75 | 25 | -- | -- | -- |
| 0.03% F | B | 8 | 100 | -- | -- | -- | -- |
| 0.1% Dex | C | 8 | 75 | -- | -- | -- | -- |
| 0.5% LE | D | 8 | 100 | -- | -- | -- | -- |
| 0.1% BOL | E | 8 | 100 | -- | -- | -- | -- |
| 0.5% BOL | F | 8 | 100 | -- | -- | -- | -- |
| 1% BOL | G | 8 | 100 | -- | -- | -- | -- |
| | | | | +115 min | | | |
| CTR | A | 8 | -- | -- | 25 | 75 | -- |
| 0.03% F | B | 8 | -- | -- | 100 | -- | -- |
| 0.1% Dex | C | 8 | -- | 75 | -- | 25 | -- |
| 0.5% LE | D | 8 | -- | 75 | 25 | -- | -- |
| 0.1% BOL | E | 8 | -- | -- | 75 | 25 | -- |
| 0.5% BOL | F | 8 | -- | 37.5 | 62.5 | -- | -- |
| 1% BOL | G | 8 | -- | -- | 100 | -- | -- |

Table T-6 Raw data of $PGE_2$ levels in aqueous humor samples collected at the second paracentesis

| Treatment | Sample | $PGE_2$ (ng/ml) |
|---|---|---|
| CTR | 2-A1-DX | 3.81 |
| | 2-A1-SX | 2.91 |
| | 2-A2-DX | 4.77 |
| | 2-A2-SX | [1]N/A |
| | 2-A3-DX | 1.46 |
| | 2-A3-SX | 3.00 |
| | 2-A4-DX | 1.87 |
| | 2-A4-SX | 1.88 |
| | | |
| 0.03% F | 2-B1-DX | 1.04 |
| | 2-B1-SX | 0.75 |
| | 2-B2-DX | 0.85 |
| | 2-B2-SX | 1.11 |
| | 2-B3-DX | 2.11 |
| | 2-B3-SX | 0.93 |
| | 2-B4-DX | 0.61 |
| | 2-B4-SX | 2.11 |
| | | |
| 0.1% Dex | 2-C1-DX | 2.51 |

(continued)

| Treatment | Sample | $PGE_2$ (ng/ml) |
|---|---|---|
| | 2-C1-SX | N/A |
| | 2-C2-DX | 2.32 |
| | 2-C2-SX | N/A |
| | 2-C3-DX | 2.10 |
| | 2-C3-SX | 3.03 |
| | 2-C4-DX | 2.32 |
| | 2-C4-SX | 1.30 |
| | | |
| 0.5% LE | 2-D1-DX | [2]N/D |
| | 2-D1-SX | N/D |
| | 2-D2-DX | N/D |
| | 2-D2-SX | 0.23 |
| | 2-D3-DX | N/D |
| | 2-D3-SX | 0.68 |
| | 2-D4-DX | N/D |
| | 2-D4-SX | 1.10 |
| | | |
| 0.1% BOL | 2-E1-DX | 1.62 |
| | 2-E1-SX | 1.88 |
| | 2-E2-DX | 2.15 |
| | 2-E2-SX | 0.70 |
| | 2-E3-DX | 1.34 |
| | 2-E3-SX | 1.03 |
| | 2-E4-DX | N/D |
| | 2-E4-SX | N/D |
| | | |
| 0.5% BOL | 2-F1-DX | 2.31 |
| | 2-F1-SX | 2.59 |
| | 2-F2-DX | N/D |
| | 2-F2-SX | 0.53 |
| | 2-F3-DX | 0.75 |
| | 2-F3-SX | 0.80 |
| | 2-F4-DX | 1.62 |
| | 2-F4-SX | 1.09 |
| | | |
| 1% BOL | 2-G1-DX | 0.50 |
| | 2-G1-SX | 1.87 |
| | 2-G2-DX | 1.71 |

(continued)

| Treatment | Sample | $PGE_2$ (ng/ml) |
|---|---|---|
| | 2-G2-SX | 4.04 |
| | 2-G3-DX | 1.11 |
| | 2-G3-SX | 3.78 |
| | 2-G4-DX | N/D |
| | 2-G4-SX | N/D |
| [1]N/A = not available<br>[2]N/D = not detectable, under the limit of quantification | | |

Table T-7 Levels of $PGE_2$ in aqueous humor samples collected at the second paracentesis (Mean ± SEM).

| Treatment | Sample Group | Mean (ng/ml) | SEM | n |
|---|---|---|---|---|
| CTR | A | 2.815 | 0.449 | 7 |
| 0.03% F | B | 1.189 | 0.209 | 8 |
| 0.1% Dex | C | 2.263 | 0.232 | 6 |
| 0.5% LE | D | 0.672 | 0.250 | 3 |
| 0.1% BOL | E | 1.452 | 0.221 | 6 |
| 0.5% BOL | F | 1.384 | 0.306 | 7 |
| 1 % BOL | G | 2.168 | 0.586 | 6 |

Table T-8 Raw data of protein levels in aqueous humor samples collected at the second paracentesis

| Treatment | Sample | Protein (mg/ml) |
|---|---|---|
| CTR | 2-A1-DX | 50.24 |
| | 2-A1-SX | 53.51 |
| | 2-A2-DX | 28.73 |
| | 2-A2-SX | [1]N/A |
| | 2-A3-DX | 40.09 |
| | 2-A3-SX | 30.84 |
| | 2-A4-DX | 41.79 |
| | 2-A4-SX | 30.35 |
| | | |
| 0.03% F | 2-B1-DX | 20.78 |
| | 2-B1-SX | 28.80 |
| | 2-B2-DX | N/A |
| | 2-B2-SX | 23.41 |
| | 2-B3-DX | 20.21 |
| | 2-B3-SX | 17.53 |
| | 2-B4-DX | 15.12 |
| | 2-B4-SX | 20.52 |

(continued)

| Treatment | Sample | Protein (mg/ml) |
|---|---|---|
| | | |
| 0.1% Dex | 2-C1-DX | 31.31 |
| | 2-C1-SX | N/A |
| | 2-C2-DX | 31.81 |
| | 2-C2-SX | N/A |
| | 2-C3-DX | 35.95 |
| | 2-C3-SX | 37.15 |
| | 2-C4-DX | 32.12 |
| | 2-C4-SX | 32.40 |
| | | |
| 0.5% LE | 2-D1-DX | 36.14 |
| | 2-D1-SX | 39.10 |
| | 2-D2-DX | 34.69 |
| | 2-D2-SX | 26.10 |
| | 2-D3-DX | 26.30 |
| | 2-D3-SX | 28.16 |
| | 2-D4-DX | 40.90 |
| | 2-D4-SX | 39.85 |
| 0.1% BOL | 2-E1-DX | 34.87 |
| | 2-E1-SX | 34.41 |
| | 2-E2-DX | 31.14 |
| | 2-E2-SX | 22.82 |
| | 2-E3-DX | 29.46 |
| | 2-E3-SX | 31.69 |
| | 2-E4-DX | 35.70 |
| | 2-E4-SX | 49.25 |
| | | |
| 0.5% BOL | 2-F1-DX | 33.98 |
| | 2-F1-SX | 33.65 |
| | 2-F2-DX | 19.99 |
| | 2-F2-SX | 27.11 |
| | 2-F3-DX | 19.72 |
| | 2-F3-SX | 36.35 |
| | 2-F4-DX | 27.71 |
| | 2-F4-SX | 32.24 |
| | | |
| 1 % BOL | 2-G1-DX | 20.99 |
| | 2-G1-SX | 21.48 |

(continued)

| Treatment | Sample | Protein (mg/ml) |
|---|---|---|
| | 2-G2-DX | 15.11 |
| | 2-G2-SX | 20.28 |
| | 2-G3-DX | 20.94 |
| | 2-G3-SX | 21.89 |
| | 2-G4-DX | 20.03 |
| | 2-G4-SX | 30.76 |
| [1]N/A = not available | | |

Table T-9 Protein levels in aqueous humor samples collected at the second paracentesis (Mean ± SEM).

| Treatment | Sample Group | Mean (mg/ml) | SEM | n |
|---|---|---|---|---|
| CTR | A | 39.364 | 3.754 | 7 |
| 0.03% F | B | 20.910 | 1.648 | 7 |
| 0.1% Dex | C | 33.457 | 1.001 | 6 |
| 0.5% LE | D | 33.905 | 2.190 | 8 |
| 0.1% BOL | E | 33.667 | 2.655 | 8 |
| 0.5% BOL | F | 28.844 | 2.249 | 8 |
| 1% BOL | G | 21.435 | 1.529 | 8 |

Table T-10 Raw data of PMN numbers in aqueous humor samples collected at the second paracentesis

| Treatment | Sample | PMN (number/μl) |
|---|---|---|
| CTR | 2-A1-DX | 90 |
| | 2-A1-SX | 80 |
| | 2-A2-DX | 70 |
| | 2-A2-SX | [1]N/A |
| | 2-A3-DX | 70 |
| | 2-A3-SX | 80 |
| | 2-A4-DX | 50 |
| | 2-A4-SX | 40 |
| | | |
| 0.03% F | 2-B1-DX | 50 |
| | 2-B1-SX | 40 |
| | 2-B2-DX | N/A |
| | 2-B2-SX | 20 |
| | 2-B3-DX | 10 |
| | 2-B3-SX | 40 |
| | 2-B4-DX | 30 |
| | 2-B4-SX | 20 |

(continued)

| Treatment | Sample | PMN (number/μl) |
|---|---|---|
| | | |
| 0.1% Dex | 2-C1-DX | 20 |
| | 2-C1-SX | N/A |
| | 2-C2-DX | 20 |
| | 2-C2-SX | N/A |
| | 2-C3-DX | 50 |
| | 2-C3-SX | 40 |
| | 2-C4-DX | 20 |
| | 2-C4-SX | 30 |
| | | |
| 0.5% LE | 2-D1-DX | N/A |
| | 2-D1-SX | N/A |
| | 2-D2-DX | 40 |
| | 2-D2-SX | 20 |
| | 2-D3-DX | 20 |
| | 2-D3-SX | 30 |
| | 2-D4-DX | 40 |
| | 2-D4-SX | 20 |
| 0.1% BOL | 2-E1-DX | N/A |
| | 2-E1-SX | 20 |
| | 2-E2-DX | 40 |
| | 2-E2-SX | 50 |
| | 2-E3-DX | 20 |
| | 2-E3-SX | 20 |
| | 2-E4-DX | 20 |
| | 2-E4-SX | N/A |
| | | |
| 0.5% BOL | 2-F1-DX | 40 |
| | 2-F1-SX | 20 |
| | 2-F2-DX | 20 |
| | 2-F2-SX | 10 |
| | 2-F3-DX | 10 |
| | 2-F3-SX | 10 |
| | 2-F4-DX | 20 |
| | 2-F4-SX | 40 |
| | | |
| 1% BOL | 2-G1-DX | 30 |
| | 2-G1-SX | 20 |

(continued)

| Treatment | Sample | PMN (number/μl) |
|---|---|---|
| | 2-G2-DX | 30 |
| | 2-G2-SX | 40 |
| | 2-G3-DX | 20 |
| | 2-G3-SX | 30 |
| | 2-G4-DX | 40 |
| | 2-G4-SX | 20 |
| [1]N/A = not available | | |

Table T-11 PMN numbers in aqueous humor samples collected at the second paracentesis (Mean ± SEM).

| Treatment | Sample Group | Mean (number/μl) | SEM | n |
|---|---|---|---|---|
| CTR | A | 68.571 | 6.701 | 7 |
| 0.03% F | B | 30.000 | 5.345 | 7 |
| 0.1% Dex | C | 30.000 | 5.164 | 6 |
| 0.5% LE | D | 28.333 | 4.014 | 6 |
| 0.1% BOL | E | 28.333 | 5.426 | 6 |
| 0.5% BOL | F | 21.250 | 4.407 | 8 |
| 1% BOL | G | 28.750 | 2.950 | 8 |

Table T-12 Raw data of MPO activity in iris-ciliary body samples collected after the second paracentesis.

| Treatment | Sample | Iris-ciliary body weight (mg) | [1]Volume (μl) | [2]Δ/min | MPO Unit/g |
|---|---|---|---|---|---|
| CTR | A1-DX | 41.7 | 40 | 0.021 | 1.11 |
| | A1-SX | 42.3 | 40 | 0.024 | 1.26 |
| | A2-DX | 46.6 | 40 | 0.039 | 1.85 |
| | A2-SX | 40.5 | 40 | 0.037 | 2.02 |
| | A3-DX | 48.9 | 40 | 0.075 | 3.39 |
| | A3-SX | 51.1 | 40 | 0.049 | 2.12 |
| | A4-DX | 36.6 | 40 | 0.013 | 0.79 |
| | A4-SX | 38.8 | 40 | 0.019 | 1.08 |
| | | | | | |
| 0.03% F | B1-DX | 39.5 | 100 | 0.049 | 1.10 |
| | B1-SX | 42.7 | 100 | 0.082 | 1.70 |
| | B2-DX | 34.1 | 100 | 0.013 | 0.34 |
| | B2-SX | 36.6 | 100 | 0.031 | 0.75 |
| | B3-DX | 45.6 | 100 | 0.038 | 0.74 |
| | B3-SX | 38.0 | 100 | 0.027 | 0.63 |
| | B4-DX | 40.1 | 100 | 0.033 | 0.73 |
| | B4-SX | 42.6 | 100 | 0.061 | 1.27 |

(continued)

| Treatment | Sample | Iris-ciliary body weight (mg) | [1]Volume (μl) | [2]Δ/min | MPO Unit/g |
|---|---|---|---|---|---|
| | | | | | |
| 0.1% Dex | C1-DX | 36.4 | 100 | 0.029 | 0.71 |
| | C1-SX | 45.8 | 100 | 0.031 | 0.60 |
| | C2-DX | 42.9 | 100 | 0.064 | 1.32 |
| | C2-SX | 42.7 | 100 | 0.023 | 0.48 |
| | C3-DX | 43.0 | 100 | 0.019 | 0.39 |
| | C3-SX | 46.8 | 100 | 0.024 | 0.45 |
| | C4-DX | 42.3 | 100 | 0.023 | 0.48 |
| | C4-SX | 36.1 | 100 | 0.021 | 0.51 |
| | | | | | |
| 0.5% LE | D1-DX | 38.9 | 200 | 0.026 | 0.30 |
| | D1-SX | 44.7 | 200 | 0.053 | 0.51 |
| | D2-DX | 35.9 | 200 | 0.067 | 0.81 |
| | D2-SX | 40.7 | 200 | 0.055 | 0.60 |
| | D3-DX | 46.3 | 200 | 0.076 | 0.73 |
| | D3-SX | 41.9 | 200 | 0.096 | 1.01 |
| | D4-DX | 46.7 | [3]N/A | N/A | N/A |
| | D4-SX | 32.9 | N/A | N/A | N/A |
| 0.1% BOL | E1-DX | 43.6 | 100 | 0.051 | 1.04 |
| | E1-SX | 37.2 | 100 | 0.042 | 1.00 |
| | E2-DX | 32.6 | 100 | 0.042 | 1.14 |
| | E2-SX | 37.4 | 100 | 0.045 | 1.06 |
| | E3-DX | 36.2 | 100 | 0.050 | 1.22 |
| | E3-SX | 45.1 | 100 | 0.031 | 0.61 |
| | E4-DX | 30.4 | 100 | 0.036 | 1.05 |
| | E4-SX | 42.3 | 100 | 0.031 | 0.65 |
| | | | | | |
| 0.5% BOL | F1-DX | 45.8 | 100 | 0.044 | 0.85 |
| | F1-SX | 38.2 | 100 | 0.040 | 0.93 |
| | F2-DX | 34.9 | 100 | 0.031 | 0.79 |
| | F2-SX | 42.0 | 100 | 0.049 | 1.03 |
| | F3-DX | 39.1 | 100 | 0.033 | 0.75 |
| | F3-SX | 40.6 | 100 | 0.034 | 0.74 |
| | F4-DX | 36.2 | 100 | 0.022 | 0.54 |
| | F4-SX | 39.5 | 100 | 0.026 | 0.58 |
| | | | | | |
| 1 % BOL | G1-DX | 32.4 | 100 | 0.024 | 0.66 |
| | G1-SX | 43.1 | 100 | 0.033 | 0.68 |

(continued)

| Treatment | Sample | Iris-ciliary body weight (mg) | [1]Volume (μl) | [2]Δ/min | MPO Unit/g |
|---|---|---|---|---|---|
| | G2-DX | 30.6 | 100 | 0.017 | 0.49 |
| | G2-SX | 39.9 | 100 | 0.018 | 0.40 |
| | G3-DX | 41.3 | 100 | 0.016 | 0.34 |
| | G3-SX | 44.9 | 100 | 0.052 | 1.02 |
| | G4-DX | 36.6 | 100 | 0.013 | 0.31 |
| | G4-SX | 36.9 | 100 | 0.018 | 0.43 |

[1]Volume = aliquot (μl) of the supernatant diluted to 3ml for the analysis.
[2]Δ/min = mean of the slope of the line recorded every 15 sec for 5 min
[3]N/A = not available

Table T-13 MPO activity in iris-ciliary body samples collected after the second paracentesis (Mean ± SEM).

| Treatment | Sample Group | Mean MPO Unit/g | SEM | n |
|---|---|---|---|---|
| CTR | A | 1.703 | 0.297 | 8 |
| 0.03% F | B | 0.906 | 0.151 | 8 |
| 0.1% Dex | C | 0.618 | 0.106 | 8 |
| 0.5% LE | D | 0.661 | 0.102 | 6 |
| 0.1% BOL | E | 0.971 | 0.079 | 8 |
| 0.5% BOL | F | 0.775 | 0.058 | 8 |
| 1 % BOL | G | 0.542 | 0.083 | 8 |

TESTING 2: Effect of BOL-303242-X on Inhibiting IL-1β-Induced Cytokine Expression in Human Corneal Epithelial Cells

1. BACKGROUND/RATIONALE:

Levels of cytokines associated with immune cells are direct indications of activity of these cells in an inflammatory condition. Reduced levels of these cytokines indicate a positive therapeutic effect on inflammation of a test compound. This study was designed to determine the effect of BOL-303242-X on IL-1ß -induced cytokine production in human corneal epithelial cells ("HCECs").

1. PURPOSE

To determine the effects of BOL-303242-X on IL-1ß-stimulated cytokine expression in primary human corneal epithelial cells using a 30-cytokine Luminex kit. Dexamethasone was used as a control.

3. EXPERIMENTAL DESIGN

Primary HCECs were seeded in 24-well plates. After 24 h, cells were treated with vehicle, IL-1ß, IL-1ß + dexamethasone, or IL-1ß + BOL-303242-X in basic EpiLife medium for 18 h (Table T-14). Each treatment was performed in triplicate. Media were collected and used for determination of cytokine content using a 30-cytokine Luminex kit. Cell viability was determined by alamarBlue assay (LP06013).

| Group* | Day 1 | Day 2: cells were treated with the test agents in basic EpiLife medium for 18 h | Day 3 |
|---|---|---|---|
| 1 | Cells were seeded in 24-well plates (5 X | Control (0.1% DMSO) | Media for Luminex assays; cells for cell viability |
| 2 | | 10 ng/ml IL-1ß | |
| 3 | | 10 ng/ml IL-1ß + 1 nM dexamethasone | |
| 4 | | 10 ng/ml IL-1ß + 10 nM dexamethasone | |
| 5 | | 10 ng/ml IL-1ß + 100 nM dexamethasone | |
| 6 | | 10 ng/ml IL-1ß + 1 μM dexamethasone | |
| 7 | $10^5$/well in 0.5 ml medium) in EpiLife medium | 10 ng/ml IL-1ß + 10 μM dexamethasone | assay |
| 8 | | 10 ng/ml IL-1ß + 1 nM BOL-303242-X | |
| 9 | | 10 ng/ml IL-1ß + 10 nM BOL-303242-X | |
| 10 | | 10 ng/ml IL-1ß + 100 nM BOL-303242-X | |
| 11 | | 10 ng/ml IL-1ß + 1 μM BOL-303242-X | |
| 12 | | 10 ng/ml IL-1ß + 10 μM BOL-303242-X | |
| *triplicate wells per group | | | |

Dexamethasone:

Lot Number: 016K14521
Parent MW: 392.46
Parent:Total MW Ratio = 1.0

BOL-303242-X:

Lot Number: 6286
Parent MW: 462.48
Parent:Total MW Ratio = 1.0

4. DATA ANALYSIS

Median fluorescence intensity (MFI) was used to obtain the concentration of each cytokines in pg/ml based on the standard curve of each cytokine assayed by Luminex. The linear range of the standard curve for each cytokine was used for determination of cytokine concentration. Duplicate values for each sample were averaged. Data were expressed as mean ± SD. Statistical analysis was performed using one-way ANOVA-Dunnett's test, and $P < 0.05$ was considered statistically significant.

5. RESULTS

No statistically significant effect on cellular metabolic activity (as measured by alamarBlue assay) was observed with the various treatments.

Substantial amounts of 16 out of 30 cytokines tested were detected in this study and 13 out of 14 cytokines detected were stimulated by 10 ng/ml IL-1ß (Table T-14). IL-1β was excluded from analysis because it was the stimulus. IL-1ra was excluded because the MFI was not within the standard range.

Dexamethasone and BOL-303242-X significantly inhibited IL-1ß-stimulated cytokine production with comparable potency on 6 cytokines (IL-6, IL-7, MCP-1, TGF-α, TNF-α and VEGF), and a significant inhibitory effect was observed at 1 nM on IL-6 and at 10 nM on MCP-1, TGF-α and TNF-α (Table T-14 and Figures 1A-1F).

BOL-303242-X also significantly inhibited IL-1ß-stimulated G-CSF production with better potency compared to dexamethasone, and a significant inhibitory effect was observed at 10 μg/ml by BOL-303242-X while no significant effect was observed by dexamethasone on this cytokine (Fig. 2).

BOL-303242-X also significantly inhibited IL-1ß-stimulated cytokine production with less potency compared to dexamethasone on 3 cytokines (GM-CSF, IL-8, and RANTES). A significant inhibitory effect was observed at 1 nM by dexamethasone and at 10 nM by BOL-303242-X on GM-CSF. A significant inhibitory effect was observed at 1 μM by dexamethasone on RANTES while no significant effect was observed by BOL-303242-X on this cytokine (Figures 3A-3C).

6. CONCLUSION

BOL-303242-X and dexamethasone have comparable potency for inhibition of IL-1ß-stimulated cytokine production in HCECs for the cases of IL-6, IL-7, TGF-α, TNF-α, VGEF, and MCP-1. BOL-303242-X is more potent than dexamethasone in inhibiting IL-1ß-stimulated production of G-CSF in HCECs. BOL-303242-X is somewhat less potent than dexamethasone in inhibiting IL-1ß-stimulated production of GM-CSF, IL-8, and RANTES in HCECs.

Table T-14

| Inhibition of IL-1β stimulated cytokine production by dexamethasone and BOL-303242-X in primary human corneal epithelial cells | | | | | | | | | | | |
|---|---|---|---|---|---|---|---|---|---|---|---|
| Cytokines detected * | Stimulated by IL-1β (10 ng/ml) | Inhibited by dexamethasone (μM) | | | | | Inhibited by BOL-303242-X (μM) | | | | |
| | | 0.0 01 | 0.0 1 | 0.1 | 1 | 10 | 0.0 01 | 0.0 1 | 0.1 | 1 | 10 |
| G-CSF | X | | | | | | | | | | X |
| GM-CSF | X | X | X | X | X | X | | X | X | | X |
| IL-1α | X | | | | | | | | | | |
| IL-6 | X | X | X | X | X | X | X | X | X | X | X |
| IL-7 | X | | | X | | | | | | | X |
| IL-8 | X | | | X | X | | | X | | | |
| IP-10 | X | | | | | | | | | | |
| MCP-1 | X | | X | X | X | X | | X | X | X | X |
| MIP-1α | | | | | | | | | | | |
| MIP-1β | X | | | | | | | | | | |
| RANTES | X | | | | X | X | | | | | |
| TGF-α | X | | X | X | X | X | | X | X | X | X |
| TNF-α | X | | X | X | X | X | | X | | | X |
| VEGF | X | | | X | X | | | | | X | X |
| Notes: (*) EGF, Eotaxin, Fractalkine, IFNγ, IL-10, IL-12p40, IL-12p70, IL-13, IL15, IL-17, IL-2, IL-4, IL-5, sCD40L were not detected. IL-1β was excluded from analysis because it was the stimulus. IL-1ra was excluded because the MFI was out of range of the standards. | | | | | | | | | | | |

TESTING 3: Evaluation Of The Effect Of Topical Bol-303242-X, Administered Unilaterally Four Times Daily, On The Intraocular Pressure in New Zealand White Rabbits For 33 Days

INTRODUCTION

The objective of this study was to evaluate the effect of topical BOL-303242-X on the intraocular pressure (IOP) in New Zealand White rabbits when administered to right eyes four times daily for 33 days. Dosing was discontinued after 31 days due to high mortality rates and limited supply of test articles. The protocol is attached as Appendix 1.

MATERIALS AND METHODS

Test Articles

Three test articles were identified as follows:

10 mg/g BOL-303242-X Ophthalmic Suspension (Lot No. 2676-MLC-270)

5 mg/g BOL-303242-X Ophthalmic Suspension (Lot No. 2676-MLC-270)

1 mg/g BOL-303242-X Ophthalmic Suspension (Lot No. 2676-MLC-270)

A negative control (balanced salt solution (BSS), B. Braun Medical Inc., Lot No. J6N011, exp. 10/08), and a positive control (0.1% dexamethasone ophthalmic suspension (Maxidex®, Alcon Laboratories, Inc., Lot No. 114619F, exp. 01/09)) were also provided. The formulations were provided in ready-to-use form and stored at room temperature. The suspensions were shaken before dose administrations to re-suspend them.

Test System

Animals

Seventy-five female New Zealand White rabbits were obtained from The Rabbit Source (Ramona, CA). Animals were 6-8 weeks old at the time of IOP-training initiation, and they weighed 1.38-2.05 kg at randomization. The protocol specified that animals would weigh at least 1.5-2.5 kg; this deviation had no effect on the outcome of the study. Animals were identified by ear tags and cage cards.

Animal Husbandry

Upon arrival, animals were examined to ensure that they were healthy and quarantined for 10 days before placement on study. At the end of the quarantine period, animals were again examined for general health parameters and for any anatomical ophthalmic abnormalities. Quarantine was conducted according to internal operating procedure.
Animals were housed in individual, hanging, stainless steel cages. Housing and sanitation were performed according to internal operating procedure.
Animals were provided Teklad Certified Global High Fiber Rabbit Diet. Diet certification and analysis were provided by the vendor, Harlan Teklad. No analyses outside those provided by the manufacturer were performed. Animals were provided tap water *ad libitum.* No contaminants were known to exist in the water and no additional analyses outside those provided by the local water district and as specified in internal operating procedure were performed.
Environmental parameters were monitored according to internal operating procedure. The study room temperature was 65-72°F with 58-77% relative humidity

Pre-Treatment Examinations

Prior to placement on study, each animal underwent a pre-treatment ophthalmic examination (slit lamp and indirect ophthalmoscopy). Observations were scored according to the McDonald Shadduck system and recorded using a standardized data collection sheet. Acceptance criteria for placement on study were as follows: Scores of ≤ 1 for conjunctival congestion and swelling; scores of 0 for all other observation variables.

IOP Conditioning and Pre-Selection

Seventy-five rabbits underwent two weeks of IOP training to condition them for IOP measurement. IOP was determined for both eyes of each animal using a Medtronic Solan, Model 30 classic pneumatonometer. Proparacaine hydrochloride 0.5% (1 drop) was delivered to each eye prior to IOP measurement. A two-point diurnal curve was established: IOP was recorded on Monday, Wednesday, and Friday of each week, at 8 a.m. and 12 p.m., with a ± 1 hour range for each of these times. The time of the measurements was recorded. During the two weeks of IOP conditioning, one rabbit died and two rabbits were euthanized due to poor health.

At the end of the two weeks of conditioning, 50 rabbits were selected for topical dosing based on the consistency of their IOP measurements at each time point. The selected rabbits continued to have their IOPs measured for one additional week.

Randomization

Prior to dosing, 50 animals were weighed and randomly assigned to five treatment groups. Treatment groups are described in Table T3-1. Animals were randomized to treatment groups according to a modified Latin square.

Topical Dosing Procedure

On Days 1-31, animals received daily topical doses of the appropriate test article into the right eye. Animals were dosed four times per day, with doses administered 2 hours apart. Doses were administered using a calibrated 50-μL pipette. The eyelids were held close for 10 seconds immediately following dosing. The time of each dose administration was recorded.

The protocol indicated that animals would be dosed four times daily for 33 days. Per decision of the Sponsor and Study Director, dosing was discontinued after 31 days due to high mortality rates and limited supply of test articles. This deviation had no adverse effect on the outcome of the study.

Mortality/Morbidity

Animals were observed for mortality/morbidity twice daily. Animals determined to be moribund were euthanized with an intravenous injection of commercial euthanasia solution.

Body Weights

Animals were weighed at randomization.

Intraocular Pressure Measurements

Intraocular pressure ("IOP") was determined for both eyes of each animal on Days 3, 5, 10, 12, 16, 18, 22, 24, 26, 30, and 32. IOP was evaluated with a Medtronic Solan, Model 30 classic pneumatonometer. Proparacaine hydrochloride 0.5% (1 drop) was delivered to each eye prior to IOP measurement. IOP was measured on Monday, Wednesday, and Friday of each week. A two-point diurnal curve was established: IOP was recorded at 8 a.m. and 12 p.m. on Day 3, and at 8 a.m. and 2 p.m. on later days, with a ± 1 hour range for each of these times. The time of the measurements was recorded.

Ophthalmic Observations

Ophthalmic examinations (slit lamp) were performed prior to the first dosing on Days 5, 12, 22, 26, and 33. Ocular findings were scored according to the McDonald Shadduck system and recorded using a standardized data collection sheet.

Study Completion

Following completion of final ophthalmic observations (Day 33), remaining animals were returned to the vivarium.

Statistical Analysis

Descriptive statistics were prepared for IOP data of each treatment group (left and right eyes separately) at

each measurement interval. The statistics included the number of observations ("N"), mean, standard deviation ("STD"), and standard error ("SEM"). Statistical analyses were conducted on IOP results using Statistical Analysis Systems (SAS Institute, Inc., Cary, NC, V8.0). Parameters were evaluated using analysis of variance/GLM Procedure followed by Tukey's Standardized Range Test (Tukey, 1985) for post hoc comparisons of group means. The level of significance was set at a probability of $p < 0.05$ for all statistical procedures. Group IOP means were compared at each interval, with left and right eyes compared separately.

IOP data for the following six animals were excluded from group statistics: Group A, Nos. 3081, 3037, 3068, and 3011; Group C, No. 3034; and Group E, No. 3084. The excluded Group A animals showed no IOP response to dexamethasone dosing, and the excluded Group C and Group E animals had outlying IOP data.

Animal Welfare Statement

This study was performed to develop a hypertensive model of intraocular pressure in New Zealand White rabbits. Alternatives to performing this study were explored; however, to properly develop the model, a whole-body test system was required. This study complied with all inyernal animal welfare policies and was approved by the Institutional Animal Care and Use Committee.

RESULTS

Mortality

Mortality data are presented in Table T3-2. Ten rabbits died or were euthanized between Days 11 and 33, as follows: Six of ten rabbits dosed with dexamethasone, one of ten rabbits dosed with 10 mg/g BOL-303242-X (0.5 mg/dose), two of ten rabbits dosed with 5 mg/g BOL-303242-X (0.25 mg/dose), and one of ten rabbits dosed with 1 mg/g BOL-303242-X (0.05 mg/dose). Seven rabbits were noted to have diarrhea, often described as severe and hemorrhagic, prior to death or euthanasia. No signs of poor health were noted for two rabbits that were found dead. Further information on observed mortality is shown in the following table.

| Group | Rabbit No. | Treatment (4 x Daily) | Day of Death(1) | Recorded Notes |
|---|---|---|---|---|
| A | 3011 | 0.1% Dexamethasone (0.05 mg/dose) | 23 | Euthanized due to severe profuse hemorrhagic diarrhea. Noted to be malnourished and anorexic. |
| A | 3016 | 0.1% Dexamethasone (0.05 mg/dose) | 27 | Found dead. No rigor mortis present. |
| A | 3037 | 0.1% Dexamethasone (0.05 mg/dose) | 25 | Euthanized due to severe hemorrhagic diarrhea. Noted to be dehydrated, lethargic, and cachectic. |
| A | 3038 | 0.1% Dexamethasone (0.05 mg/dose) | 13 | Euthanized due to severe hemorrhagic diarrhea. |
| A | 3068 | 0.1% Dexamethasone (0.05 mg/dose) | 25 | Euthanized due to severe hemorrhagic diarrhea. Noted to be dehydrated, lethargic, and cachectic. |
| A | 3086 | 0.1% Dexamethasone (0.05 mg/dose) | 27 | Euthanized. Very sick/poor health; left (untreated) eye protruding. |
| B | 3008 | 10 mg/g BOL-303242-X (0.5 mg/dose) | 11 | Found dead. Noted on Day 9 to have significant diarrhea and a yellowish discharge in the dosed eye. |
| C | 3028 | 5 mg/g BOL-303242-X (0.25 mg/dose) | 17 | Euthanized due to severe diarrhea. |

(continued)

| Group | Rabbit No. | Treatment (4 x Daily) | Day of Death(1) | Recorded Notes |
|---|---|---|---|---|
| C | 3074 | 5 mg/g BOL-303242-X (0.25 mg/ dose) | 33 | Euthanized prior to final ocular examination due to a respiratory infection. Diarrhea noted on Day 26. |
| D | 3010 | 1 mg/g BOL-303242-X (0.05 mg/ dose) | 29 | Found dead. |

(1) Day euthanized or found dead.

Remaining rabbits survived until study completion (Day 33). One surviving rabbit dosed with 10 mg/g BOL-303242-X (0.5 mg/dose) was noted to have diarrhea on Day 18 (Group B, No. 3048).

Ophthalmic Observations

Slit-lamp ophthalmic observations are presented in Table T3-3. A key to the ophthalmic observation scores is presented in Table T3-4. Eyes appeared normal at most observations. Mild conjunctival congestion (score = 1) was seen sporadically, mostly in treated right eyes, with no consistent association with test or control article. The only other findings were a small area of corneal pigmentation in an untreated left eye (Group A, No. 3086), a pinpoint corneal scar in a 10 mg/g BOL-303242-X-dosed right eye (Group B, No. 3083), and a subconjunctival hemorrhage in a 1 mg/g BOL-303242-X-dosed right eye (Group D, No. 3043). The observed corneal lesions might be related to the pneumotonometry procedure.

Intraocular Pressure Measurements

Descriptive statistics for IOP data are presented in Table T3-5 (left eyes, a.m.), Table T3-6 (right eyes, p.m.), Table T3-7 (left eyes, p.m.) and Table T3-8 (right eyes, p.m.).

Mean IOP varied throughout the study for all groups; the variations were similar for left and right eyes within each group. For all groups (including the BSS dose group), mean IOP reached a maximum between Days 5 and 10 for both left and right eyes, a.m. and p.m. readings. Diurnal changes in IOP from a.m. to p.m. were not evident during the study, possibly due to daily feeding of rabbits prior to p.m. measurements.

For the dexamethasone group (Group A), mean IOP of both left and right eyes increased sharply after treatment began. This increase was not seen in the mean IOPs of the BOL-303242-X groups (Groups B-D) at any point of the study. On several days, the mean IOP in one or both eyes of the dexamethasone group (Group A) was significantly higher ($p < 0.05$) than the mean IOP in the corresponding eyes of other groups. This difference was more common in the a.m. than the p.m., and it occurred at more timepoints for the untreated left eyes than the treated right eyes. Mean IOP of BSS-dosed right eyes (Group E) was generally lower than mean IOP of BOL-303242-X-dosed right eyes (Groups B-D) in the a.m. but not in the p.m. No statistically significant ($p < 0.05$) differences in mean IOP were seen between the BSS group and BOL-303242-X groups.

CONCLUSIONS

The objective of this study was to evaluate the effect of topical BOL-303242-X on the intraocular pressure (IOP) in New Zealand White rabbits when administered to right eyes four times daily for 33 days. In conclusion, unilateral topical instillation of BOL-303242-X suspension (0.05, 0.25, or 0.5 mg/dose), dexamethasone suspension (0.05 mg/dose), or balanced salt solution in rabbit eyes four times daily up to 31 days was associated with sporadic mild conjunctival congestion. Dosing with dexamethasone up to 31 days was associated with a higher mortality rate (6 deaths per 10 rabbits) than dosing with BOL-303242-X up to 31 days (per dose level, 1-2 deaths per 10 rabbits). Daily dosing with the BOL-303242-X suspensions did not increase IOP when compared to daily dosing with dexamethasone.

Table T3-1
Treatment Groups

| Group | No. | Treatment (4 x Daily) | Dose Location (Right Eye) | Dose Volume | Drug Dose Level | Scheduled Study Completion(1) |
|---|---|---|---|---|---|---|
| A | 10 | 0.1% Dexamethasone (Maxidex®) | Topical | 50 µL | 0.05 mg/dose | Day 33 |
| B | 10 | 10 mg/g BOL-303242-X | Topical | 50 µL | 0.5 mg/dose | Day 33 |
| C | 10 | 5 mg/g BOL-303242-X | Topical | 50 µL | 0.25 mg/dose | Day 33 |
| D | 10 | 1 mg/g BOL-303242-X | Topical | 50 µL | 0.05 mg/dose | Day 33 |
| E | 10 | Balanced Salt Solution | Topical | 50 µL | N/A | Day 33 |

N/A = Not Applicable.
(1) Dosing was performed daily through Day 31. Final ophthalmic examinations were performed on Day 33.

Table T3-2
Mortality

| Group | No. | Treatment (4 x Daily) | Dose Location (Right Eye) | Dose Volume | Drug Dose Level | Scheduled Study Completion (1) | Mortality(2) |
|---|---|---|---|---|---|---|---|
| A | 10 | 0.1% Dexamethasone (Maxidex®) | Topical | 50 µL | 0.05 mg/ dose | Day 33 | 6/10(3) |
| B | 10 | 10 mg/g BOL-303242-X | Topical | 50 µL | 0.5 mg/ dose | Day 33 | 1/10(4) |
| C | 10 | 5 mg/g BOL-303242-X | Topical | 50 µL | 0.25 mg/ dose | Day 33 | 2/10(5) |
| D | 10 | 1 mg/g BOL-303242-X | Topical | 50 µL | 0.05 mg/ dose | Day 33 | 1/10(6) |
| E | 10 | Balanced Salt Solution | Topical | 50 µL | N/A | Day 33 | 0/10 |

N/A = Not Applicable.
(1) Dosing was performed daily through Day 31. Final ophthalmic examinations were performed on Day 33.
(2) Mortality is expressed as the number of animals found dead or euthanized prior to study completion/number of animals in group.
(3) One Group A rabbit was found dead on Day 27. Five Group A rabbits were euthanized between Days 13 and 27 due to severe diarrhea.
(4) One Group B rabbit was found dead on Day 11; it was observed to have diarrhea on Day 10.
(5) One Group C rabbit was euthanized on Day 17 due to severe diarrhea. The other was euthanized on Day 33 prior to final ophthalmic examinations due to a respiratory infection.
(6) One Group D rabbit was found dead on Day 29.

Table T3-3

Ophthalmic Observations (Slit-Lamp)

| Group | Animal No. | Treatment (4 x Daily) | Eye | Day | Ophthalmic Observation(1) | Score |
|---|---|---|---|---|---|---|
| A | 3016 | Untreated<br>0.1% Dexamethasone | Left<br>Right | 5, 12, 22, 26<br>5 12,22,26 | AN<br>Conjunctival Congestion AN | N/A<br>1 N/A |
| A | 3081 | Untreated<br>0.1% Dexamethasone | Left<br>Right | 5, 12, 22, 26, 33<br>22 5, 12, 26, 33 | AN<br>Conjunctival Congestion AN | N/A<br>1 N/A |
| A | 3086 | Untreated<br>0.1% Dexamethasone | Left<br>Right | 26 5, 12, 22<br>5, 12, 22, 26 | Cornea AN<br>AN | 1(2) N/A<br>N/A |
| A | 3037 | Untreated<br>0.1% Dexamethasone | Left<br>Right | 5, 12, 22<br>5, 12, 22 | AN<br>AN | N/A<br>N/A |
| A | 3006 | Untreated<br>0.1% Dexamethasone | Left<br>Right | 5, 12, 22, 26, 33<br>5, 12, 22, 26, 33 | AN<br>AN | N/A<br>N/A |
| A | 3068 | Untreated<br>0.1% Dexamethasone | Left<br>Right | 5, 12, 22<br>5, 12, 22 | AN<br>AN | N/A<br>N/A |
| A | 3033 | Untreated<br>0.1% Dexamethasone | Left<br>Right | 5, 12, 22, 26, 33<br>5, 12, 22, 26, 33 | AN<br>AN | N/A<br>N/A |
| A | 3029 | Untreated<br>0.1% Dexamethasone | Left<br>Right | 5, 12, 22, 26, 33<br>5, 12, 22, 26, 33 | AN<br>AN | N/A<br>N/A |
| A | 3011 | Untreated<br>0.1% Dexamethasone | Left<br>Right | 5, 12, 22<br>5, 12, 22 | AN<br>AN | N/A<br>N/A |
| A | 3038 | Untreated<br>0.1% Dexamethasone | Left<br>Right | 5, 12<br>5, 12 | AN<br>AN | N/A<br>N/A |

AN = Appeared normal. N/A = Not Applicable. See Table T3-4 for key to ophthalmic observation scores.

(1) Observations were made prior to the first dose of the day.

(2) Small area of pigmentation in center of cornea.

| Group | Animal No. | Topical Treatment | Eye | Day | Ophthalmic Observation(1) | Score |
|---|---|---|---|---|---|---|
| B | 3083 | Untreated<br>10 mg/g BOL-303242-X | Left<br>Right | 5, 12, 22, 26, 33<br>5<br>5 12,22,26,33 | AN<br>Cornea Surface area of cornea involvement AN | N/A<br>1(2) 1 N/A |
| B | 3008 | Untreated<br>10 mg/g BOL-303242-X | Left<br>Right | 5<br>5 | AN<br>AN | N/A<br>N/A |
| B | 3017 | Untreated | Left | 5, 12, 22, 26, 33 | AN | N/A |

(continued)

| Group | Animal No. | Topical Treatment | Eye | Day | Ophthalmic Observation(1) | Score |
|---|---|---|---|---|---|---|
| | | 10 mg/g BOL-303242-X | Right | 5, 12 22,26,33 | Conjunctival Congestion AN | 1 N/A |
| B | 3048 | Untreated | Left | 5, 12,22,26,33 | AN | N/A |
| | | 10 mg/g BOL-303242-X | Right | 5, 12, 22, 26, 33 | AN | N/A |
| B | 3003 | Untreated | Left | 5, 12, 22, 26, 33 | AN | N/A |
| | | 10 mg/g BOL-303242-X | Right | 12 5, 22, 26, 33 | Conjunctival Congestion AN | 1 N/A |
| B | 3042 | Untreated | Left | 5, 12, 22, 26, 33 | AN | N/A |
| | | 10 mg/g BOL-303242-X | Right | 26 5, 12, 22, 33 | Conjunctival Congestion AN | 1 N/A |
| B | 3023 | Untreated | Left | 5, 12, 22, 26, 33 | AN | N/A |
| | | 10 mg/g BOL-303242-X | Right | 5, 12, 22, 26, 33 | AN | N/A |
| B | 3004 | Untreated | Left | 5, 12, 22, 26, 33 | AN | N/A |
| | | 10 mg/g BOL-303242-X | Right | 5, 12, 22, 26, 33 | AN | N/A |
| B | 3049 | Untreated | Left | 5, 12, 22, 26, 33 | AN | N/A |
| | | 10 mg/g BOL-303242-X | Right | 5, 12, 22, 26, 33 | AN | N/A |
| B | 3026 | Untreated | Left | 5, 12, 22, 26, 33 | AN | N/A |
| | | 10 mg/g BOL-303242-X | Right | 5, 12, 22, 26, 33 | AN | N/A |
| AN = Appeared normal. N/A = Not Applicable. See Table T3-4 for key to ophthalmic observation scores. (1) Observations were made prior to the first dose of the day. (2) Pinpoint corneal scar. | | | | | | |
| C | 3028 | Untreated | Left | 5, 12 | AN | N/A |
| | | 5 mg/g BOL-303242-X | Right | 5, 12 | AN | N/A |
| C | 3064 | Untreated | Left | 5, 12, 22, 26, 33 | AN | N/A |
| | | 5 mg/g BOL-303242-X | Right | 5 12,22,26,33 | Conjunctival congestion AN | I N/A |
| C | 3031 | Untreated | Left | 5, 12, 22, 26, 33 | AN | N/A |
| | | 5 mg/g BOL-303242-X | Right | 22 5, 12, 26, 33 | Conjunctival congestion AN | I N/A |
| C | 3032 | Untreated | Left | 5, 12, 22, 26, 33 | AN | N/A |
| | | 5 mg/g BOL-303242-X | Right | 5, 12, 22, 26, 33 | AN | N/A |
| C | 3041 | Untreated | Left | 5, 12, 22, 26, 33 | AN | N/A |
| | | 5 mg/g BOL-303242-X | Right | 5, 12, 22, 26, 33 | AN | N/A |
| C | 3034 | Untreated | Left | 5, 12, 22, 26, 33 | AN | N/A |
| | | 5 mg/g BOL-303242-X | Right | 5, 12, 22, 26, 33 | AN | N/A |
| C | 3035 | Untreated | Left | 5, 12, 22, 26, 33 | AN | N/A |

(continued)

| Group | Animal No. | Topical Treatment | Eye | Day | Ophthalmic Observation(1) | Score |
|---|---|---|---|---|---|---|
| | | 5 mg/g BOL-303242-X | Right | 22, 26 5, 12, 33 | Conjunctival congestion AN | 1 N/A |
| C | 3046 | Untreated | Left | 5, 12, 22, 26, 33 | AN | N/A |
| | | 5 mg/g BOL-303242-X | Right | 5, 12, 22, 26, 33 | AN | N/A |
| C | 3058 | Untreated | Left | 5, 12, 22, 26, 33 | AN | N/A |
| | | 5 mg/g BOL-303242-X | Right | 5, 12, 22, 26, 33 | AN | N/A |
| C | 3074 | Untreated | Left | 5, 12, 22, 26 | AN | N/A |
| | | 5 mg/g BOL-303242-X | Right | 26 5, 12, 22 | Conjunctival congestion AN | 1 N/A |

AN = Appeared normal. N/A = Not Applicable. See Table T3-4 for key to ophthalmic observation scores.
(1) Observations were made prior to the first dose of the day.

| Group | Animal No. | Topical Treatment | Eye | Day | Ophthalmic Observation | Score |
|---|---|---|---|---|---|---|
| D | 3010 | Untreated | Left | 5, 12, 22, 26 | AN | N/A |
| | | 1 mg/g BOL-303242-X | Right | 5, 12,22,26 | AN | N/A |
| D | 3039 | Untreated | Left | 5, 12, 22, 26, 33 | AN | N/A |
| | | 1 mg/g BOL-303242-X | Right | 5, 12, 22, 26, 33 | AN | N/A |
| D | 3043 | Untreated | Left | 5, 12, 22, 26, 33 | AN(2) | N/A |
| | | 1 mg/g BOL-303242-X | Right | 5, 12, 22, 26, 33 | AN | N/A |
| D | 3044 | Untreated | Left | 5, 12, 22, 26, 33 | AN | N/A |
| | | 1 mg/g BOL-303242-X | Right | 5, 12, 22, 26, 33 | AN | N/A |
| D | 3027 | Untreated | Left | 5, 12, 22, 26, 33 | AN | N/A |
| | | l mg/g BOL-303242-X | Right | 5, 12, 22, 26, 33 | AN | N/A |
| D | 3072 | Untreated | Left | 5, 12, 22, 26, 33 | AN | N/A |
| | | 1 mg/g BOL-303242-X | Right | 5, 12,22,26,33 | AN | N/A |
| D | 3040 | Untreated | Left | 5, 12, 22, 26, 33 | AN | N/A |
| | | 1 mg/g BOL-303242-X | Right | 22 5, 12, 26, 33 | Conjunctival congestion AN | 1 N/A |
| D | 3020 | Untreated | Left | 5, 12, 22, 26, 33 | AN | N/A |
| | | 1 mg/g BOL-303242-X | Right | 5, 12, 22, 26, 33 | AN | N/A |
| D | 3063 | Untreated | Left | 5, 12, 22, 26, 33 | AN | N/A |
| | | 1 mg/g BOL-303242-X | Right | 5, 12, 22, 26, 33 | AN | N/A |
| D | 3077 | Untreated | Left | 5, 12, 22, 26, 33 | AN | N/A |
| | | 1 mg/g BOL-303242-X | Right | 5, 12, 22, 26, 33 | AN | N/A |

AN = Appeared normal. N/A = Not Applicable. See Table T3-4 for key to ophthalmic observation scores.
(1) Observations were made prior to the first dose of the day.
(2) Day 12: Subconjunctival hemorrhage observed.

(continued)

| Group | Animal No. | Topical Treatment | Eye | Day | Ophthalmic Observation(1) | Score |
|---|---|---|---|---|---|---|
| E | 3002 | Untreated | Left | 5, 12, 22, 26, 33 | AN | N/A |
| | | Balanced Salt Solution | Right | 5, 12, 22, 26, 33 | AN | N/A |
| E | 3084 | Untreated | Left | 5,12,22,26,33 | AN | N/A |
| | | Balanced Salt Solution | Right | 5, 12, 22, 26, 33 | AN | N/A |
| E | 3057 | Untreated | Left | 5, 12, 22, 26, 33 | AN | N/A |
| | | Balanced Salt Solution | Right | 12,22,26 5, 33 | Conjunctival Congestion AN | 1 N/A |
| E | 3087 | Untreated | Left | 5, 12, 22, 26, 33 | AN | N/A |
| | | Balanced Salt Solution | Right | 5, 12, 22, 26, 33 | AN | N/A |
| E | 3018 | Untreated | Left | 5, 12, 22, 26, 33 | AN | N/A |
| | | Balanced Salt Solution | Right | 26 5, 12, 22, 33 | Conjunctival Congestion AN | 1 N/A |
| E | 3090 | Untreated | Left | 5, 12, 22, 26, 33 | AN | N/A |
| | | Balanced Salt Solution | Right | 5, 12,22,26,33 | AN | N/A |
| E | 3047 | Untreated | Left | 5, 12, 22, 26, 33 | AN | N/A |
| | | Balanced Salt Solution | Right | 5, 12, 22, 26, 33 | AN | N/A |
| E | 3070 | Untreated | Left | 26 5, 12, 22, 33 | Conjunctival Congestion AN | 1 N/A |
| | | Balanced Salt Solution | Right | 5, 12, 22, 26, 33 | AN | N/A |
| E | 3019 | Untreated | Left | 5, 12, 22, 26, 33 | AN | N/A |
| | | Balanced Salt Solution | Right | 5, 12, 22, 26, 33 | AN | N/A |
| E | 3007 | Untreated | Left | 5, 12, 22, 26, 33 | AN | N/A |
| | | Balanced Salt Solution | Right | 5, 12, 22, 26, 33 | AN | N/A |

AN = Appeared normal. N/A = Not Applicable. See Table T3-4 for key to ophthalmic observation scores.

(1) Observations were made prior to the first dose of the day.

Table T3-4

Key to Ophthalmic Observation Scoring System

CONJUNCTIVAL CONGESTION

1 = A flushed, reddish color predominantly confined to the palpebral conjunctiva with some perilimbal injection but primarily confined to the lower and upper parts of the eye from the 4:00 to 7:00 and 11:00 to 1:00 positions.

CORNEA

1 = Some loss of transparency. Only the epithelium and/or the anterior half of the stoma are involved. The underlying structures are clearly visible although some cloudiness may be readily apparent.

SURFACE AREA OF CORNEA INVOLVEMENT

1 = 1-25% area of stromal cloudiness.

Table T3-5
Descriptive Statistics for Intraocular Pressure in Untreated Left Eyes (A.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study (5/9/07) | MEAN | 24.4 | 23.8 | 24.2 | 23.9 | 23.4 |
| | SEM | 0.7 | 0.6 | 0.4 | 0.4 | 0.5 |
| | STD | 2.1 | 1.8 | 1.2 | 1.3 | 1.5 |
| | N | 10 | 10 | 10 | 10 | 10 |
| 3 | MEAN | 24.3 | 23.3 | 23.8 | 23.5 | 22.7 |
| | SEM | 0.5 | 0.4 | 0.4 | 0.6 | 0.4 |
| | STD | 1.2 | 1.2 | 1.1 | 1.8 | 1.3 |
| | N | 6 | 10 | 9 | 10 | 9 |
| 5 | MEAN | 24.3 | 23.4 | 24.4 | 24.4 | 24.1 |
| | SEM | 0.8 | 0.6 | 0.6 | 0.5 | 0.4 |
| | STD | 2.0 | 1.9 | 1.7 | 1.5 | 1.3 |
| | N | 6 | 10 | 9 | 10 | 9 |
| 10 | MEAN | 26.9 | 24.0 | 24.6 | 24.5 | 25.4 |
| | SEM | 0.5 | 0.8 | 0.6 | 0.4 | 0.7 |
| | STD | 1.2 | 2.4 | 1.9 | 1.2 | 2.1 |
| | N | 6 | 10 | 9 | 10 | 9 |
| 12 | MEAN | 26.2 | 23.8 | 23.8 | 22.2 | 23.7 |
| | SEM | 0.6 | 0.7 | 0.7 | 0.7 | 0.7 |
| | STD | 1.5 | 2.0 | 2.2 | 2.3 | 2.0 |
| | N | 6 | 9 | 9 | 10 | 9 |
| 16 | MEAN | 25.0 | 22.9 | 23.4 | 21.6 | 20.3 |
| | SEM | 1.0 | 0.7 | 0.6 | 1.1 | 0.6 |
| | STD | 2.2 | 2.1 | 1.7 | 3.4 | 1.9 |
| | N | 5 | 9 | 9 | 10 | 9 |
| 18 | MEAN | 24.2 | 21.2 | 21.9 | 23.3 | 22.3 |
| | SEM | 0.4 | 0.5 | 0.6 | 0.4 | 0.6 |
| | STD | 1.0 | 1.6 | 1.7 | 1.4 | 1.9 |
| | N | 5 | 9 | 8 | 10 | 9 |
| 22 | MEAN | 25.0 | 21.8 | 21.6 | 22.4 | 22.0 |
| | SEM | 0.5 | 0.6 | 1.1 | 0.3 | 0.5 |
| | STD | 1.2 | 1.8 | 3.0 | 1.0 | 1.6 |
| | N | 5 | 9 | 8 | 10 | 9 |
| 24 | MEAN | 23.6 | 20.2 | 22.1 | 22.4 | 20.8 |
| | SEM | 0.9 | 0.6 | 0.6 | 0.8 | 0.7 |
| | STD | 2.1 | 1.8 | 1.7 | 2.5 | 2.1 |
| | N | 5 | 9 | 8 | 10 | 9 |

(continued)

Descriptive Statistics for Intraocular Pressure in Untreated Left Eyes (A.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| 26 | MEAN | 23.7 | 21.7 | 21.7 | 22.9 | 20.5 |
| | SEM | 1.0 | 0.7 | 1.1 | 0.6 | 0.6 |
| | STD | 2.2 | 2.0 | 3.0 | 2.0 | 1.7 |
| | N | 5 | 9 | 8 | 10 | 9 |
| 30 | MEAN | 24.0 | 22.7 | 22.6 | 23.4 | 22.7 |
| | SEM | 1.0 | 0.6 | 1.2 | 0.8 | 0.5 |
| | STD | 1.7 | 1.7 | 3.4 | 2.4 | 1.5 |
| | N | 3 | 9 | 8 | 9 | 9 |
| 32 | MEAN | 25.5 | 22.9 | 23.1 | 24.1 | 22.3 |
| | SEM | 0.8 | 0.5 | 0.7 | 0.6 | 0.5 |
| | STD | 1.3 | 1.6 | 2.1 | 1.8 | 1.5 |
| | N | 3 | 9 | 8 | 9 | 9 |

NOTE: Differences between means with a same superscript in the same row are statistically significant ($p < 0.05$).

Table T3-6

Descriptive Statistics for Intraocular Pressure in Treated Right Eyes (A.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study (5/9/07) | MEAN | 24.1 | 24.0 | 24.8 | 24.4 | 24.1 |
| | SEM | 0.7 | 0.5 | 0.5 | 0.6 | 0.5 |
| | STD | 2.2 | 1.7 | 1.6 | 1.9 | 1.6 |
| | N | 10 | 10 | 10 | 10 | 10 |
| 3 | MEAN | 24.3 | 22.7 | 23.7 | 23.0 | 22.1 |
| | SEM | 0.8 | 0.5 | 0.4 | 0.6 | 0.4 |
| | STD | 2.0 | 1.5 | 1.3 | 2.0 | 1.3 |
| | N | 6 | 10 | 9 | 10 | 9 |
| 5 | MEAN | 24.7 | 23.8 | 24.7 | 24.7 | 24.0 |
| | SEM | 0.8 | 0.7 | 0.7 | 0.5 | 0.5 |
| | STD | 1.9 | 2.3 | 2.1 | 1.5 | 1.5 |
| | N | 6 | 10 | 9 | 10 | 9 |
| 10 | MEAN | 26.9 | 24.5 | 25.2 | 24.8 | 25.3 |
| | SEM | 0.3 | 0.6 | 0.6 | 0.5 | 0.6 |
| | STD | 0.7 | 2.0 | 1.7 | 1.4 | 1.8 |
| | N | 6 | 10 | 9 | 10 | 9 |
| 12 | MEAN | 26.7 | 23.9 | 25.0 | 23.4 | 23.2 |
| | SEM | 0.8 | 1.1 | 0.8 | 0.8 | 0.5 |

(continued)

Descriptive Statistics for Intraocular Pressure in Treated Right Eyes (A.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| | STD | 1.9 | 3.4 | 2.3 | 2.6 | 1.6 |
| | N | 6 | 9 | 9 | 10 | 9 |
| 16 | MEAN | 25.8 | 23.4 | 24.3 | 22.1 | 20.7 |
| | SEM | 1.4 | 0.7 | 0.6 | 1.0 | 0.9 |
| | STD | 3.2 | 2.1 | 1.7 | 3.0 | 2.8 |
| | N | 5 | 9 | 9 | 10 | 9 |
| 18 | MEAN | 24.1 | 22.3 | 23.9 | 23.7 | 21.9 |
| | SEM | 0.7 | 0.8 | 0.7 | 0.5 | 0.8 |
| | STD | 1.6 | 2.3 | 1.9 | 1.7 | 2.4 |
| | N | 5 | 9 | 8 | 10 | 9 |
| 22 | MEAN | 25.4 | 22.4 | 22.4 | 23.2 | 21.4 |
| | SEM | 0.4 | 0.6 | 0.7 | 0.4 | 0.6 |
| | STD | 0.8 | 1.9 | 1.9 | 1.4 | 1.8 |
| | N | 5 | 9 | 8 | 10 | 9 |
| 24 | MEAN | 24.3 | 21.2 | 23.8 | 22.1 | 21.1 |
| | SEM | 0.8 | 0.7 | 0.6 | 0.7 | 0.9 |
| | STD | 1.8 | 2.2 | 1.7 | 2.2 | 2.6 |
| | N | 5 | 9 | 8 | 10 | 9 |
| 26 | MEAN | 23.1 | 21.8 | 22.1 | 23.1 | 20.4 |
| | SEM | 0.9 | 1.0 | 1.3 | 0.8 | 0.5 |
| | STD | 1.9 | 3.0 | 3.7 | 2.4 | 1.4 |
| | N | 5 | 9 | 8 | 10 | 9 |
| 30 | MEAN | 23.5 | 22.7 | 22.9 | 24.2 | 22.1 |
| | SEM | 1.0 | 0.6 | 1.3 | 0.8 | 0.5 |
| | STD | 1.8 | 1.8 | 3.5 | 2.4 | 1.4 |
| | N | 3 | 9 | 8 | 9 | 9 |
| 32 | MEAN | 25.5 | 23.9 | 23.4 | 24.9 | 23.1 |
| | SEM | 0.6 | 0.4 | 0.9 | 0.6 | 0.5 |
| | STD | 1.0 | 1.2 | 2.5 | 1.9 | 1.4 |
| | N | 3 | 9 | 8 | 9 | 9 |

NOTE: Differences between means with a same superscript in the same row are statistically significant ($p < 0.05$).

Table T3-7
Descriptive Statistics for Intraocular Pressure in Untreated Left Eyes (P.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study (5/9/07) | MEAN | 24.2 | 23.9 | 24.4 | 24.2 | 24.2 |
| | SEM | 0.5 | 0.4 | 0.3 | 0.5 | 0.4 |
| | STD | 1.5 | 1.1 | 1.1 | 1.7 | 1.3 |
| | N | 10 | 10 | 10 | 10 | 10 |
| 3 | MEAN | 24.3 | 23.3 | 23.9 | 25.0 | 23.5 |
| | SEM | 0.7 | 0.4 | 0.5 | 0.4 | 0.4 |
| | STD | 1.7 | 1.2 | 1.4 | 1.3 | 1.2 |
| | N | 6 | 10 | 9 | 10 | 9 |
| 5 | MEAN | 25.6 | 25.2 | 24.8 | 24.7 | 25.1 |
| | SEM | 0.6 | 0.6 | 0.7 | 0.4 | 0.4 |
| | STD | 1.4 | 2.0 | 2.0 | 1.3 | 1.2 |
| | N | 6 | 10 | 9 | 10 | 9 |
| 10 | MEAN | 26.6 | 23.5 | 24.6 | 24.9 | 24.9 |
| | SEM | 0.6 | 1.5 | 0.4 | 0.5 | 0.4 |
| | STD | 1.4 | 4.9 | 1.1 | 1.6 | 1.3 |
| | N | 6 | 10 | 9 | 10 | 9 |
| 12 | MEAN | 22.8 | 24.1 | 23.3 | 23.7 | 24.4 |
| | SEM | 0.9 | 0.9 | 0.5 | 0.4 | 0.7 |
| | STD | 2.2 | 2.8 | 1.5 | 1.4 | 2.0 |
| | N | 6 | 9 | 9 | 10 | 9 |
| 16 | MEAN | 22.6 | 21.4 | 20.4 | 21.9 | 21.3 |
| | SEM | 0.6 | 0.4 | 0.6 | 0.4 | 0.5 |
| | STD | 1.4 | 1.2 | 1.8 | 1.3 | 1.5 |
| | N | 5 | 9 | 9 | 10 | 9 |
| 18 | MEAN | 23.6 | 22.1 | 21.9 | 22.7 | 22.0 |
| | SEM | 0.7 | 0.6 | 0.8 | 0.4 | 0.5 |
| | STD | 1.6 | 1.9 | 2.2 | 1.3 | 1.5 |
| | N | 5 | 9 | 8 | 10 | 9 |
| 22 | MEAN | 23.6 | 22.6 | 22.1 | 22.1 | 21.1 |
| | SEM | 0.4 | 0.5 | 0.8 | 0.7 | 0.8 |
| | STD | 1.0 | 1.5 | 2.2 | 2.1 | 2.4 |
| | N | 5 | 9 | 8 | 10 | 9 |
| 24 | MEAN | 25.3 | 22.8 | 22.2 | 22.9 | 22.1 |
| | SEM | 0.7 | 0.8 | 0.8 | 0.5 | 0.4 |
| | STD | 1.5 | 2.3 | 2.4 | 1.6 | 1.2 |
| | N | 5 | 9 | 8 | 10 | 9 |
| 26 | MEAN | 21.9 | 21.4 | 22.3 | 22.1 | 20.9 |
| | SEM | 1.2 | 0.9 | 1.1 | 1.0 | 0.7 |
| | STD | 2.7 | 2.6 | 3.2 | 3.2 | 2.0 |

(continued)

Descriptive Statistics for Intraocular Pressure in Untreated Left Eyes (P.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| | N | 5 | 9 | 8 | 10 | 9 |
| 30 | MEAN | 23.3 | 21.7 | 20.9 | 21.3 | 22.9 |
| | SEM | 1.1 | 0.8 | 1.1 | 0.4 | 0.7 |
| | STD | 1.9 | 2.4 | 3.0 | 1.1 | 2.0 |
| | N | 3 | 9 | 8 | 9 | 9 |
| 32 | MEAN | 25.2 | 22.6 | 21.5 | 21.9 | 22.2 |
| | SEM | 0.3 | 1.2 | 1.3 | 0.3 | 0.6 |
| | STD | 0.6 | 3.5 | 3.5 | 1.0 | 1.7 |
| | N | 3 | 9 | 8 | 9 | 9 |

NOTE: Differences between means with a same superscript in the same row are statistically significant ($p < 0.05$).

Table T3-8

Descriptive Statistics for Intraocular Pressure in Treated Right Eyes (P.M. Readings)

| Day | Statistic | Intraocular Pressure (mmHg) | | | | |
|---|---|---|---|---|---|---|
| | | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| Pre-Study (5/9/07) | MEAN | 23.4 | 24.0 | 24.5 | 24.2 | 24.2 |
| | SEM | 0.6 | 0.4 | 0.3 | 0.5 | 0.5 |
| | STD | 1.8 | 1.2 | 0.9 | 1.7 | 1.6 |
| | N | 10 | 10 | 10 | 10 | 10 |
| 3 | MEAN | 24.1 | 23.1 | 23.6 | 24.7 | 23.2 |
| | SEM | 0.6 | 0.3 | 0.5 | 0.4 | 0.6 |
| | STD | 1.4 | 0.8 | 1.6 | 1.2 | 1.7 |
| | N | 6 | 10 | 9 | 10 | 9 |
| 5 | MEAN | 26.3 | 25.7 | 24.8 | 25.5 | 25.6 |
| | SEM | 0.5 | 0.5 | 0.6 | 0.5 | 0.6 |
| | STD | 1.2 | 1.7 | 1.9 | 1.6 | 1.8 |
| | N | 6 | 10 | 9 | 10 | 9 |
| 10 | MEAN | 26.8 | 24.3 | 25.6 | 25.3 | 24.9 |
| | SEM | 0.4 | 1.5 | 0.5 | 0.6 | 0.6 |
| | STD | 1.0 | 4.6 | 1.6 | 2.0 | 1.7 |
| | N | 6 | 10 | 9 | 10 | 9 |
| 12 | MEAN | 23.4 | 23.8 | 23.4 | 24.0 | 25.3 |
| | SEM | 0.5 | 0.8 | 0.6 | 0.5 | 0.5 |
| | STD | 1.3 | 2.5 | 1.7 | 1.5 | 1.4 |
| | N | 6 | 9 | 9 | 10 | 9 |
| 16 | MEAN | 21.5 | 21.6 | 21.4 | 22.0 | 21.3 |

(continued)

Descriptive Statistics for Intraocular Pressure in Treated Right Eyes (P.M. Readings)

| | | Intraocular Pressure (mmHg) | | | | |
|---|---|---|---|---|---|---|
| Day | Statistic | 0.1% Dexamethasone (Group A) | 10 mg/g BOL-303242-X (Group B) | 5 mg/g BOL-303242-X (Group C) | 1 mg/g BOL-303242-X (Group D) | Balanced Salt Solution (Group E) |
| | SEM | 0.9 | 0.6 | 0.7 | 0.5 | 0.4 |
| | STD | 2.1 | 1.9 | 2.1 | 1.6 | 1.1 |
| | N | 5 | 9 | 9 | 10 | 9 |

| Day | Statistic | 0.1% Dexamethasone (Group A) | 10 mg/g BOL-303242-X (Group B) | 5 mg/g BOL-303242-X (Group C) | I mg/g BOL-303242-X (Group D) | Balanced Salt Solution (Group E) |
|---|---|---|---|---|---|---|
| 18 | MEAN | 23.6 | 22.5 | 21.6 | 23.1 | 21.9 |
| | SEM | 0.8 | 0.9 | 0.9 | 0.3 | 0.5 |
| | STD | 1.8 | 2.6 | 2.6 | 0.9 | 1.5 |
| | N | 5 | 9 | 8 | 10 | 9 |
| 22 | MEAN | 23.1 | 23.1 | 22.8 | 22.5 | 21.2 |
| | SEM | 1.4 | 0.5 | 1.1 | 0.4 | 0.8 |
| | STD | 3.2 | 1.6 | 3.0 | 1.4 | 2.3 |
| | N | 5 | 9 | 8 | 10 | 9 |
| 24 | MEAN | 25.4 | 22.8 | 23.4 | 23.6 | 22.8 |
| | SEM | 0.3 | 0.8 | 0.9 | 0.6 | 0.6 |
| | STD | 0.7 | 2.5 | 2.5 | 2.0 | 1.8 |
| | N | 5 | 9 | 8 | 10 | 9 |
| 26 | MEAN | 21.2 | 20.9 | 22.2 | 22.6 | 20.8 |
| | SEM | 1.1 | 0.9 | 1.3 | 0.7 | 0.5 |
| | STD | 2.6 | 2.6 | 3.8 | 2.1 | 1.5 |
| | N | 5 | 9 | 8 | 10 | 9 |
| 30 | MEAN | 22.3 | 22.4 | 22.4 | 21.8 | 23.5 |
| | SEM | 1.1 | 1.1 | 1.0 | 0.3 | 0.5 |
| | STD | 1.9 | 3.3 | 2.7 | 1.0 | 1.5 |
| | N | 3 | 9 | 8 | 9 | 9 |
| 32 | MEAN | 24.2 | 23.3 | 22.7 | 22.9 | 22.5 |
| | SEM | 1.4 | 1.1 | 1.2 | 0.5 | 0.6 |
| | STD | 2.4 | 3.4 | 3.4 | 1.5 | 1.8 |
| | N | 3 | 9 | 8 | 9 | 9 |

## Claims

1. A composition comprising a dissociated glucocorticoid receptor agonist ("DIGRA"), a pharmaceutically acceptable salt thereof, or a pharmaceutically acceptable ester thereof for use for treating or preventing glaucoma or progression thereof in a subject, wherein the DIGRA has Formula I

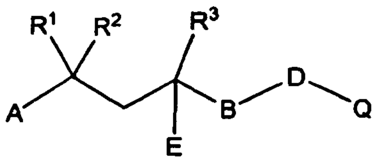

(I)

wherein A is a dihydrobenzofuranyl group substituted with a halogen atom; Q is a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; E is the hydroxy group, $R^3$ is a trifluoromethyl group, and wherein said glaucoma results from chronic inflammation.

2. The composition for use as in claim 1, wherein said glaucoma is selected from the group consisting of primary open-angle glaucoma, primary angle-closure glaucoma, secondary open-angle glaucoma, secondary angle-closure glaucoma, pigmentary glaucoma, neovascular glaucoma, pseudophakic glaucoma, malignant glaucoma, uveitic glaucoma, glaucoma due to peripheral anterior synechia, and combinations thereof.

3. The composition for use as in claim 1 or 2, wherein the composition causes a lower level of at least an adverse side effect in a subject than another composition comprising at least a glucocorticoid, wherein both said compositions are used to treat, control, reduce, ameliorate, or alleviate an inflammatory condition.

4. The composition for use as in claim 3, wherein said level of said at least an adverse side effect is determined by in vitro testing.

5. The composition for use as in claim 3, wherein said level of said at least an adverse side effect is determined in vivo.

6. The composition for use as in claim 3, wherein said at least a glucocorticoid is selected from the group consisting of dexamethasone, prednisone, prednisolone, methylprednisolone, medrysone, triamcinolone, triamcinolone acetonide, fluorometholone, loteprednol etabonate, physiologically acceptable salts thereof, combinations thereof, and mixtures thereof.

7. The composition for use as in claim 3, wherein said at least an adverse side effect is selected from the group consisting of glaucoma, cataract, hypertension, hyperglycemia, hyperlipidemia, and hypercholesterolemia.

8. The composition for use as in claim 3, wherein the level of said at least an adverse side effect is determined at a time selected from the group consisting of about 14 days, about 30 days, about 2 months, about 3 months, about 4 months, about 5 months, and about 6 months, after the composition is first administered to, and is present in, a subject.

9. The composition for use as in claim 1 or 2, wherein the DIGRA has Formula I

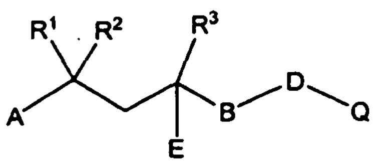

(I)

wherein A is a dihydrobenzofuranyl group substituted with a fluorine atom; Q is a quinolinyl or isoquinolinyl group substituted with a methyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; $R_3$ is a trifluoromethyl group;

10. A composition comprising a dissociated glucocorticoid receptor agonist ("DIGRA"), a pharmaceuticall acceptable salt thereof, or a pharmaceutically acceptable ester thereof for use for treating or preventing glaucoma or progression thereof in a subject, wherein the DIGRA has Formula II

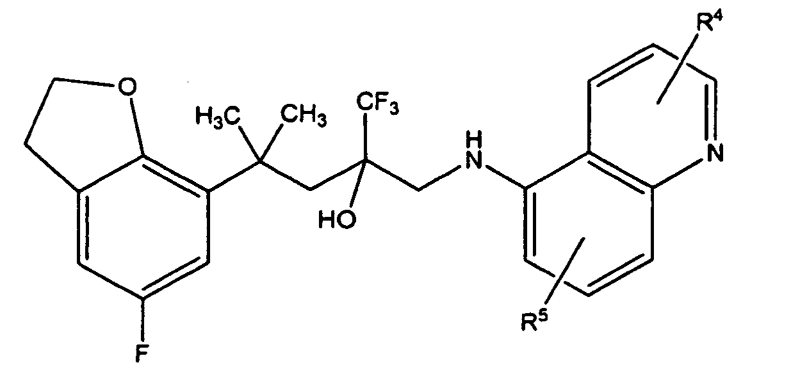

(II)

or Formula III

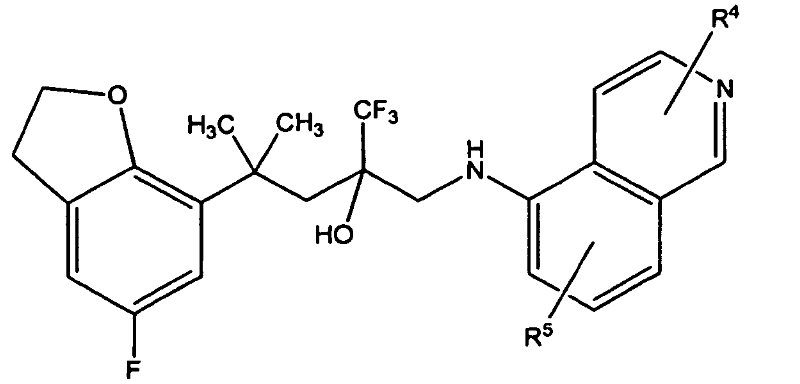

(III)

wherein $R^4$ and $R^5$ are independently selected from the group consisting of hydrogen, halogen, cyano, hydroxy, $C_1$-$C_{10}$ alkoxy groups, unsubstituted $C_1$-$C_{10}$ linear or branched alkyl groups, substituted $C_1$-$C_{10}$ linear or branched alkyl groups, unsubstituted $C_3$-$C_{10}$ cyclic alkyl groups, and substituted $C_3$-$C_{10}$ cyclic alkyl groups, wherein said glaucoma results from chronic inflammation.

**11.** The composition for use as in claim 1 or 2, wherein the DIGRA has Formula IV

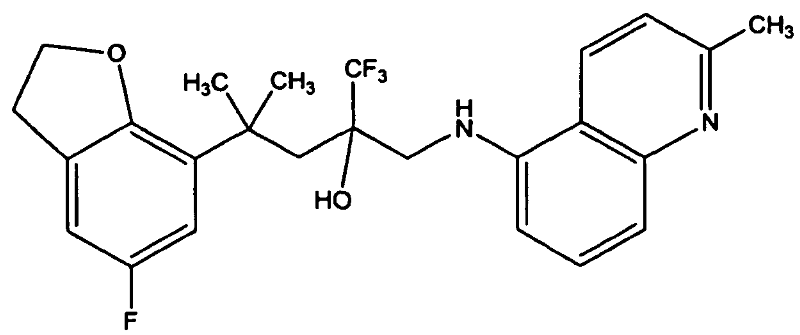

(IV)

**12.** The composition for use as in claim 11, further comprising an additional anti-inflammatory agent selected from the group consisting of non-steroidal anti-inflammatory drugs ("NSAIDs"), peroxisome proliferator-activated receptor ("PPAR") ligands, anti-histaminic drugs, antagonists to proinflammatory cytokines, inhibitors of proinflammatory cytokines, nitric oxide synthase inhibitors, combinations thereof, and mixtures thereof.

**13.** The composition for use as in claim 1 or 2, further comprising an additional anti-inflammatory agent selected from the group consisting of NSAIDs, PPAR ligands, antihistaminic drugs, antagonists to proinflammatory cytokines, inhibitors of proinflammatory cytokines, nitric oxide synthase inhibitors, combinations thereof, and mixtures thereof.

**14.** The composition for use as in claim 6, further comprising an additional anti-inflammatory agent selected from the

group consisting of NSAIDs, PPAR ligands, antihistaminic drugs, antagonists to proinflammatory cytokines, inhibitors of proinflammatory cytokines, nitric oxide synthase inhibitors, combinations thereof, and mixtures thereof.

**15.** The composition for use as in claim 14, wherein said additional anti-inflammatory agent is selected from the group consisting of PPAR ligands, inhibitors of proinflammatory cytokines, and combinations thereof.

**16.** The composition for use as in claim 9, further comprising an additional anti-inflammatory agent selected from the group consisting of NSAIDs, PPAR ligands, antihistaminic drugs, antagonists to proinflammatory cytokines, inhibitors of proinflammatory cytokines, nitric oxide synthase inhibitors, combinations thereof, and mixtures thereof.

**17.** The composition for use as in claim 10 or 11, further comprising an additional anti-inflammatory agent selected from the group consisting of NSAIDs, PPAR ligands, antihistaminic drugs, antagonists to proinflammatory cytokines, inhibitors of proinflammatory cytokines, nitric oxide synthase inhibitors, combinations thereof, and mixtures thereof.

**18.** The composition for use as in claim 16, wherein said additional anti-inflammatory agent comprises a nitric oxide synthase inhibitor.

**19.** The composition for use as in claim 17, wherein said additional anti-inflammatory agent comprises a nitric oxide synthase inhibitor.

**20.** Use of a DIGRA, a pharmaceutically acceptable salt thereof, or a pharmaceutically acceptable ester thereof for the preparation of a medicament for treating or preventing glaucoma or progression thereof, wherein the DIGRA has Formula I

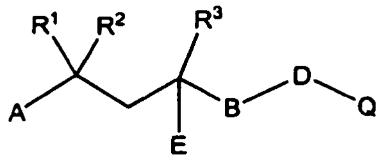

(I)

wherein A is a dihydrobenzofuranyl group substituted with a halogen atom; Q is a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH-group; and E is the hydroxy group, $R^3$ is a trifluoromethyl group, and wherein said glaucoma results from chronic inflammation.

**21.** The use of claim 20, further including using an additional anti-inflammatory agent for the preparation.

**22.** The use of claim 21, wherein said additional anti-inflammatory agent is selected from the group consisting of NSAIDs, PPAR ligands, combinations thereof, and mixtures thereof.

**23.** The use of claim 20, wherein said DIGRA, pharmaceutically acceptable salt thereof, or a pharmaceutically acceptable ester thereof is included in a composition of any one of claims 3, 4, 7 and 8.

**24.** The use of claim 20, further including using another therapeutic or prophylactic agent for treating, reducing, or preventing increased intraocular pressure, loss of retinal ganglion cells, or both, in said preparation.

**25.** The use of claim 24, wherein said another therapeutic or prophylactic agent is selected from the group consisting of acetylcholinesterase inhibitors, muscarinic cholinergic agonists, carbonic anhydrase inhibitors, prostaglandin analogs, β-adrenergic antagonists, α-adrenergic agonists, osmotic agents, and combinations thereof.

**Patentansprüche**

**1.** Eine Zusammensetzung umfassend einen dissoziierten Glucocorticoid-Rezeptor-Agonisten ("DIGRA"), ein pharmazeutisch zulässiges Salz davon oder einen pharmazeutisch zulässigen Ester davon zur Verwendung bei der Behandlung oder Verhütung von Glaukom oder dem Fortschreiten davon in einem Subjekt, wobei das DIGRA die Formel I

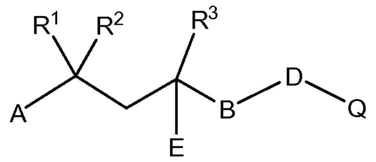

(I)

aufweist, wobei A eine mit einem Halogenatom substituierte Dihydrobenzofuranyl-Gruppe ist; Q eine mit einer $C_1$-$C_{10}$-ALkyl-Gruppe substituierte Quinolinyl- oder Isoquinolinyl-Gruppe ist; $R^1$ und $R^2$ jeweils unabhängig voneinander ausgewählt sind aus der Gruppe bestehend aus unsubstituierten und substituierten $C_1$-$C_5$-Alkyl-Gruppen; B eine $C_1$-$C_3$-Alkylen-Gruppe ist; D eine -NH-Gruppe ist; E eine Hydroxy-Gruppe ist und $R^3$ eine Trifluormethylgruppe ist und wobei besagtes Glaukom die Folge einer chronischen Entzündung ist.

2. Die Zusammensetzung zur Verwendung gemäß Anspruch 1, wobei besagtes Glaukom ausgewählt ist aus der Gruppe bestehend aus primärem Offenwinkelglaukom, primärem Engwinkelglaukom, sekundärem Offenwinkelglaukon, sekundärem Engwinkelglaukom, Pigmentglaukom, neovaskulärem Glaukom, pseudophaken Glaukom, malignem Glaukom, uveitischem Glaukom, Glaukom aufgrund peripherer vorderer Synechie und Kombinationen davon.

3. Die Zusammensetzung zur Verwendung gemäß Anspruch 1 oder 2, wobei die Zusammensetzung einen geringeren Grad wenigstens einer unerwünschten Nebenwirkung in einem Subjekt verursacht als eine andere Zusammensetzung, die wenigstens ein Glucocorticoid umfasst, wobei beide besagte Zusammensetzungen zur Behandlung, Kontrolle, Reduzierung, Verbesserung oder Linderung eines entzündlichen Zustands verwendet werden.

4. Die Zusammensetzung zur Verwendung gemäß Anspruch 3, wobei besagter Grad besagter wenigstens einer unerwünschten Nebenwirkung durch in vitro-Tests ermittelt wird.

5. Die Zusammensetzung zur Verwendung gemäß Anspruch 3, wobei besagter Grad besagter wenigstens einer unerwünschten Nebenwirkung in vivo ermittelt wird.

6. Die Zusammensetzung zur Verwendung gemäß Anspruch 3, wobei besagtes wenigstens eine Glucocorticoid ausgewählt ist aus der Gruppe bestehend aus Dexamethason, Prednison, Prednisolon, Methylprednisolon, Medryson, Triamcinolon, Triamcinolonacetonid, Fluorometholon, Loteprednoletabonat, physiologisch zulässigen Salzen davon, Kombinationen davon und Mischungen davon.

7. Die Zusammensetzung zur Verwendung gemäß Anspruch 3, wobei besagte wenigstens eine unerwünschte Nebenwirkung ausgewählt ist aus der Gruppe bestehend aus Glaukom, Katarakt, Hypertonie, Hyperglykämie, Hyperlipoproteinämie und Hypercholesterinämie.

8. Die Zusammensetzung zur Verwendung gemäß Anspruch 3, wobei der Grad besagter wenigstens einer unerwünschten Nebenwirkung zu einer Zeit, ausgewählt aus der Gruppe bestehend aus etwa 14 Tagen, etwa 30 Tagen, etwa 2 Monaten, etwa 3 Monaten, etwa 4 Monaten, etwa 5 Monaten und etwa 6 Monaten, ermittelt wird, nachdem die Zusammensetzung das erste Mal einem Subjekt verabreicht wird oder in einem Subjekt vorhanden ist.

9. Die Zusammensetzung zur Verwendung gemäß Anspruch 1 oder 2, wobei das DIGRA die Formel I

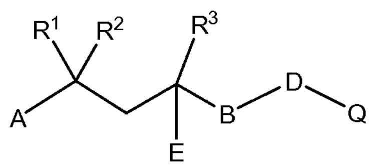

(I)

aufweist, wobei A eine mit einem Fluoratom substituierte Dihydrobenzofuranyl-Gruppe ist; Q eine mit einer Methylgruppe substituierte Quinolinyl- oder Isoquinolinyl-Gruppe ist; $R^1$ und $R^2$ jeweils unabhängig voneinander ausgewählt sind aus der Gruppe bestehend aus unsubstituierten und substituierten $C_1$-$C_5$-Alkyl-Gruppen; B eine $C_1$-$C_3$-Alkylen-Gruppe ist; D eine -NH-Gruppe ist; E eine Hydroxy-Gruppe ist; $R^3$ eine Trifluormethylgruppe ist.

**10.** Eine Zusammensetzung umfassend einen dissoziierten Glucocorticoid-Rezeptor-Agonisten ("DIGRA"), ein pharmazeutisch zulässiges Salz davon oder einen pharmazeutisch zulässigen Ester davon zur Verwendung bei der Behandlung oder Verhütung von Glaukom oder dem Fortschreiten davon in einem Subjekt, wobei das DIGRA die Formel II

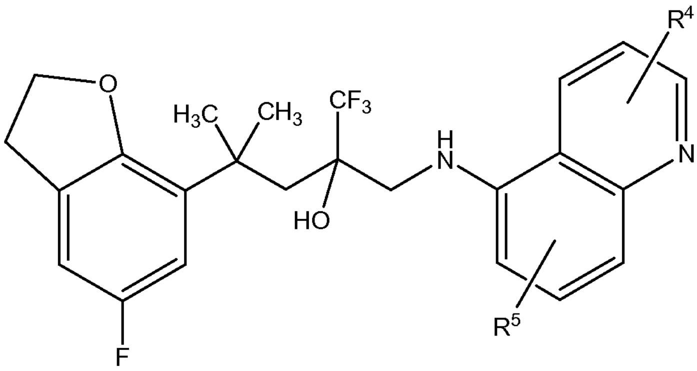

(II)

oder Formel III

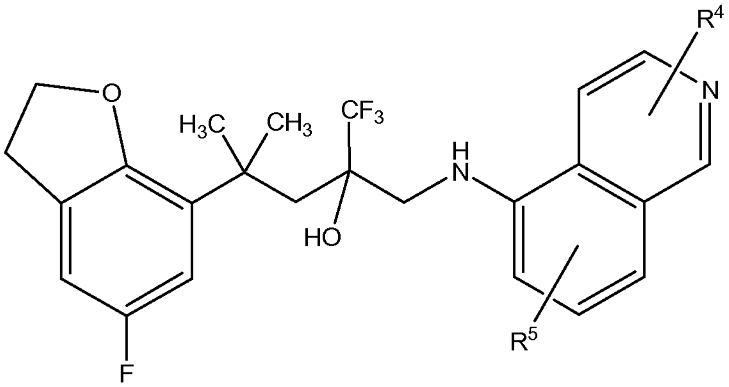

(III)

aufweist, wobei $R^4$ und $R^5$ jeweils unabhängig voneinander ausgewählt sind aus der Gruppe bestehend aus Wasserstoff, Halogen, Cyano, Hydroxy, $C_1$-$C_{10}$ AlkoxyGruppen, unsubstituierten $C_1$-$C_{10}$ linearen oder verzweigten Alkyl-Gruppen, substituierten $C_1$-$C_{10}$ linearen oder verzweigten Alkyl-Gruppen, unsubstituierten $C_3$-$C_{10}$ zyklischen Alkyl-Gruppen und substituierten $C_3$-$C_{10}$ zyklischen Alkyl-Gruppen, wobei besagtes Glaukom die Folge einer chronischen Entzündung ist.

**11.** Die Zusammensetzung zur Verwendung gemäß Anspruch 1 oder 2, wobei das DIGRA die Formel IV

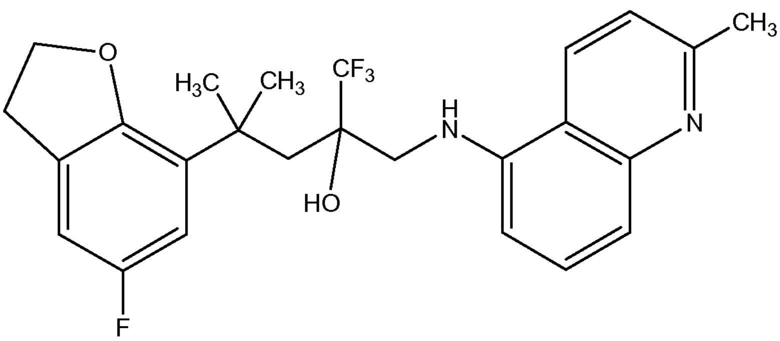

(IV)

aufweist.

**12.** Die Zusammensetzung zur Verwendung gemäß Anspruch 11, weiterhin umfassend einen zusätzlichen entzündungshemmenden Wirkstoff ausgewählt aus der Gruppe bestehend aus nicht-steroidalen entzündungshemmenden Arzneimitteln ("NSAIDs"), Peroxisome-Proliferator aktivierten Rezeptor ("PPAR") Liganden, Antihistaminarzneimitteln, Antagonisten zu proinflammatorischen Zytokinen, Inhibitoren proinflammatorischer Zytokine, Stickoxid-Synthaseinhibitoren, Kombinationen davon und Mischungen davon.

**13.** Die Zusammensetzung zur Verwendung gemäß Anspruch 1 oder 2, weiterhin umfassend einen zusätzlichen entzündungshemmenden Wirkstoff ausgewählt aus der Gruppe bestehend aus NSAIDs, PPAR-Liganden, Antihistaminarzneimitteln, Antagonisten zu proinflammatorischen Zytokinen, Inhibitoren proinflammatorischer Zytokine, Stickoxid-Synthaseinhibitoren, Kombinationen davon und Mischungen davon.

**14.** Die Zusammensetzung zur Verwendung gemäß Anspruch 6, weiterhin umfassend einen zusätzlichen entzündungshemmenden Wirkstoff ausgewählt aus der Gruppe bestehend aus NSAIDs, PPAR-Liganden, Antihistaminarzneimitteln, Antagonisten zu proinflammatorischen Zytokinen, Inhibitoren proinflammatorischer Zytokine, Stickoxid-Synthaseinhibitoren, Kombinationen davon und Mischungen davon.

**15.** Die Zusammensetzung zur Verwendung gemäß Anspruch 14, wobei besagter zusätzlicher entzündungshemmender Wirkstoff ausgewählt ist aus der Gruppe bestehend aus PPAR-Liganden, Inhibitoren proinflammatorischer Zytokine und Kombinationen davon.

**16.** Die Zusammensetzung zur Verwendung gemäß Anspruch 9, weiterhin umfassend einen zusätzlichen entzündungshemmenden Wirkstoff ausgewählt aus der Gruppe bestehend aus NSAIDs, PPAR-Liganden, Antihistaminarzneimitteln, Antagonisten zu proinflammatorischen Zytokinen, Inhibitoren proinflammatorischer Zytokine, Stickoxid-Synthaseinhibitoren, Kombinationen davon und Mischungen davon.

**17.** Die Zusammensetzung zur Verwendung gemäß Anspruch 10 oder 11, weiterhin umfassend einen zusätzlichen entzündungshemmenden Wirkstoff ausgewählt aus der Gruppe bestehend aus NSAIDs, PPAR-Liganden, Antihistaminarzneimitteln, Antagonisten zu proinflammatorischen Zytokinen, Inhibitoren proinflammatorischer Zytokine, Stickoxid-Synthaseinhibitoren, Kombinationen davon und Mischungen davon.

**18.** Die Zusammensetzung zur Verwendung gemäß Anspruch 16, wobei besagter zusätzlicher entzündungshemmender Wirkstoff einen Stickoxid-Synthaseinhibitor umfasst.

**19.** Die Zusammensetzung zur Verwendung gemäß Anspruch 17, wobei besagter zusätzlicher entzündungshemmender Wirkstoff einen Stickoxid-Synthaseinhibitor umfasst.

**20.** Verwendung eines DIGRA, eines pharmazeutisch zulässigen Salzes davon oder eines pharmazeutisch zulässigen Esters davon für die Herstellung eines Medikaments zur Behandlung oder Verhütung von Glaukom oder dem Fortschreiten davon, wobei das DIGRA die Formel I

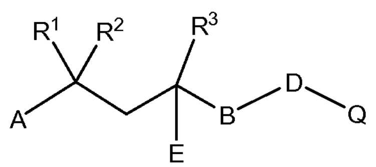

(I)

aufweist, wobei A eine mit einem Halogenatom substituierte Dihydrobenzofuranyl-Gruppe ist; Q eine mit einer $C_1$-$C_{10}$-Alkyl-Gruppe substituierte Quinolinyl- oder Isoquinolinyl-Gruppe ist; $R^1$ und $R^2$ jeweils unabhängig voneinander ausgewählt sind aus der Gruppe bestehend aus unsubstituierten und substituierten $C_1$-$C_5$-Alkyl-Gruppen; B eine $C_1$-$C_3$-Alkylen-Gruppe ist; D eine -NH-Gruppe ist und E eine Hydroxy-Gruppe ist, $R^3$ eine Trifluormethylgruppe ist und wobei besagtes Glaukom die Folge einer chronischen Entzündung ist.

**21.** Die Verwendung gemäß Anspruch 20, weiterhin beinhaltend die Verwendung eines zusätzlichen entzündungshemmenden Wirkstoffs für die Zubereitung.

**22.** Die Verwendung gemäß Anspruch 21, wobei besagter zusätzlicher entzündungshemmender Wirkstoff ausgewählt ist aus der Gruppe bestehend aus NSAIDs, PPAR-Liganden, Kombinationen davon und Mischungen davon.

**23.** Die Verwendung gemäß Anspruch 20, wobei besagtes DIGRA, pharmazeutisch zulässiges Salz davon oder ein pharmazeutisch zulässiger Ester davon in einer Zusammensetzung eines jeden der Ansprüche 3, 4, 7 und 8 enthalten ist.

**24.** Die Verwendung gemäß Anspruch 20, weiterhin beinhaltend die Verwendung eines weiteren therapeutischen oder prophylaktischen Wirkstoffs zur Behandlung, Reduzierung oder Verhütung von erhöhtem Augeninnendruck, von Verlust der retinalen Ganglienzellen oder beidem in besagter Zubereitung.

**25.** Die Verwendung gemäß Anspruch 24, wobei besagter weiterer therapeutischer oder prophylaktischer Wirkstoff ausgewählt ist aus der Gruppe bestehend aus Acetylcholinesteraseinhibitoren, muscarinisch-cholinergischen Agonisten, kohlenstoffhaltigen Anhydraseinhibitoren, Prostaglandinanaloga, β-adrenergischen Antagonisten, α-adrenergischen Agonisten, osmotischen Wirkstoffen und Kombinationen davon.

## Revendications

**1.** Composition comprenant un agoniste de récepteur de glucocorticoïde dissocié ("DIGRA"), un sel pharmaceutiquement acceptable de celui-ci, ou un ester pharmaceutiquement acceptable de celui-ci, pour utilisation pour le traitement ou la prévention d'un glaucome ou de la progression de celui-ci chez un sujet, dans laquelle le DIGRA est de formule I

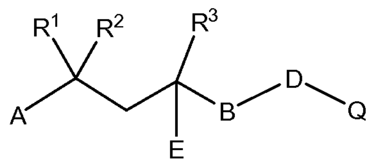

(I)

dans laquelle A est un groupe dihydrobenzofuranyle substitué par un atome d'halogène ; Q est un groupe quinolinyle ou isoquinolinyle substitué par un groupe alkyle en $C_1$ à $C_{10}$ ; $R^1$ et $R^2$ sont indépendamment choisis dans l'ensemble constitué par les groupes alkyle en $C_1$ à $C_5$ substitués et non substitués ; B est un groupe alkylène en $C_1$ à $C_3$ ; D est le groupe -NH- ; E est le groupe hydroxy, $R^3$ est un groupe trifluorométhyle,
dans laquelle ledit glaucome résulte d'une inflammation chronique.

**2.** Composition pour utilisation selon la revendication 1, dans laquelle ledit glaucome est choisi dans l'ensemble constitué par un glaucome à angle ouvert primaire, un glaucome à angle fermé primaire, un glaucome à angle ouvert secondaire, un glaucome à angle fermé secondaire, un glaucome pigmentaire, un glaucome néovasculaire, un glaucome pseudophakique, un glaucome malin, un glaucome uvéitique, un glaucome dû à une synéchie antérieure périphérique, et leurs combinaisons.

**3.** Composition pour utilisation selon la revendication 1 ou 2, laquelle composition provoque un niveau réduit d'au moins un effet secondaire indésirable chez un sujet par rapport à une autre composition comprenant au moins un glucocorticoïde, dans laquelle les deux dites compositions sont utilisées pour traiter, contrôler, réduire, améliorer ou soulager un état inflammatoire.

**4.** Composition pour utilisation selon la revendication 3, dans laquelle ledit niveau dudit au moins un effet secondaire indésirable est déterminé par un test in vitro.

**5.** Composition pour utilisation selon la revendication 3, dans laquelle ledit niveau dudit au moins un effet secondaire indésirable est déterminé in vivo.

**6.** Composition pour utilisation selon la revendication 3, dans laquelle ledit au moins un glucocorticoïde est choisi dans l'ensemble constitué par la dexaméthasone, la prednisone, la prednisolone, la méthylprednisolone, la médrysone, la triamcinolone, le triamcinolone acétonide, la fluorométholone, l'étabonate de lotéprednol, leurs sels physiologiquement acceptables, leurs combinaisons, et leurs mélanges.

**7.** Composition pour utilisation selon la revendication 3, dans laquelle ledit au moins un effet secondaire indésirable est choisi dans l'ensemble constitué par le glaucome, la cataracte, l'hypertension, l'hyperglycémie, l'hyperlipidémie,

et l'hyper-cholestérolémie.

**8.** Composition pour utilisation selon la revendication 3, dans laquelle le niveau dudit au moins un effet secondaire indésirable est déterminé à un moment choisi dans l'ensemble constitué par environ 14 jours, environ 30 jours, environ 2 mois, environ 3 mois, environ 4 mois, environ 5 mois, et environ 6 mois, après que la composition a été administrée au sujet pour la première fois et est présente chez celui-ci.

**9.** Composition pour utilisation selon la revendication 1 ou 2, dans laquelle le DIGRA est de formule I

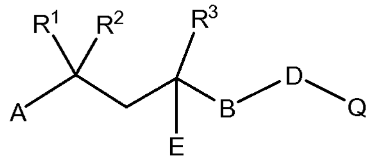

(I)

dans laquelle A est un groupe dihydrobenzofuranyle substitué par un atome de fluor ; Q est un groupe quinolinyle ou isoquinolinyle substitué par un groupe méthyle ; $R^1$ et $R^2$ sont indépendamment choisis dans l'ensemble constitué par les groupes alkyle en $C_1$ à $C_5$ substitués et non substitués ; B est un groupe alkylène en $C_1$ à $C_3$ ; D est le groupe -NH- ; E est le groupe hydroxy ; $R^3$ est un groupe trifluorométhyle.

**10.** Composition comprenant un agoniste de récepteur de glucocorticoïde dissocié ("DIGRA"), un sel pharmaceutiquement acceptable de celui-ci, ou un ester pharmaceutiquement acceptable de celui-ci, pour utilisation pour le traitement ou la prévention d'un glaucome ou de la progression de celui-ci chez un sujet, dans laquelle le DIGRA est de formule II

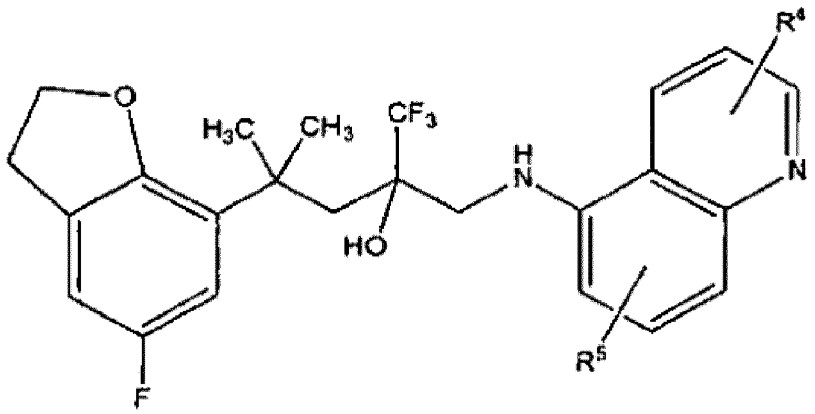

(II)

ou de formule III

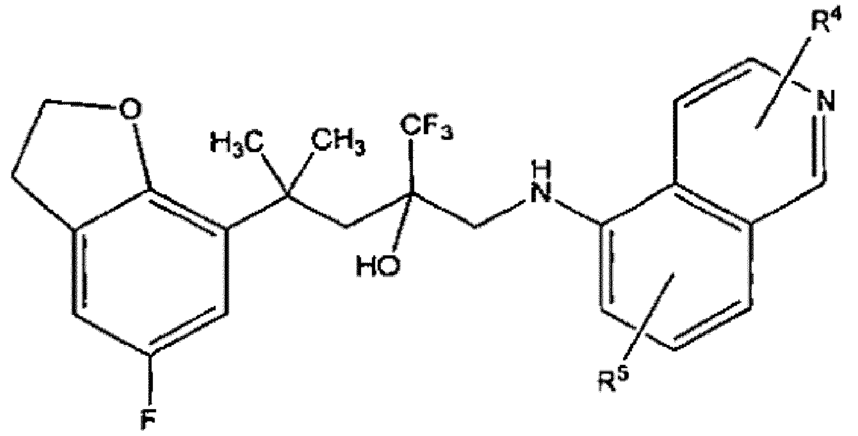

(III)

formules dans lesquelles $R^4$ et $R^5$ sont indépendamment choisis dans l'ensemble constitué par l'hydrogène, un halogène, cyano, hydroxy, les groupes alcoxy en $C_1$ à $C_{10}$, les groupes alkyle linéaires ou ramifiés en $C_1$ à $C_{10}$ non substitués, les groupes alkyle linéaires ou ramifiés en $C_1$ à $C_{10}$ substitués, les groupes alkyle cycliques en $C_3$ à $C_{10}$ non substitués, et les groupes alkyle cycliques en $C_3$ à $C_{10}$ substitués,
dans laquelle ledit glaucome résulte d'une inflammation chronique.

**11.** Composition pour utilisation selon la revendication 1 ou 2, dans laquelle le DIGRA est de formule IV

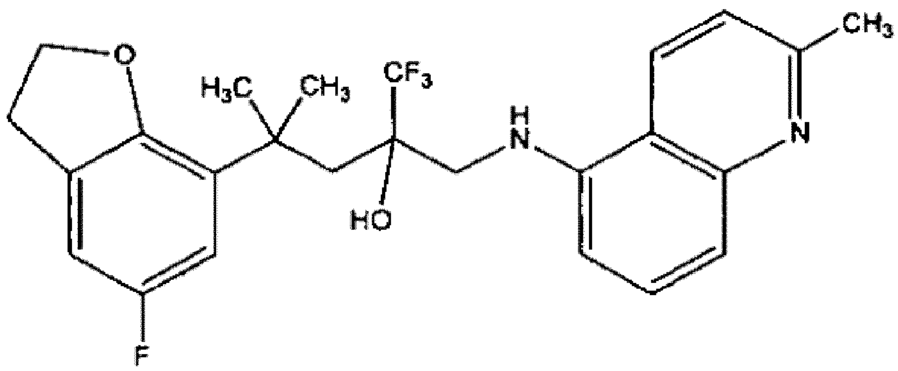

(IV)

**12.** Composition pour utilisation selon la revendication 11, comprenant en outre un agent anti-inflammatoire additionnel choisi dans l'ensemble constitué par les anti-inflammatoires non stéroïdiens ("AINS"), les ligands de récepteur activé par le proliférateur de peroxisome ("PPAR"), les antihistaminiques, les antagonistes des cytokines pro-inflammatoires, les inhibiteurs de cytokines pro-inflammatoires, les inhibiteurs d'oxyde nitrique synthase, leurs combinaisons, et leurs mélanges.

**13.** Composition pour utilisation selon la revendication 1 ou 2, comprenant en outre un agent anti-inflammatoire additionnel choisi dans l'ensemble constitué par les AINS, les ligands de PPAR, les antihistaminiques, les antagonistes des cytokines pro-inflammatoires, les inhibiteurs de cytokines pro-inflammatoires, les inhibiteurs d'oxyde nitrique synthase, leurs combinaisons, et leurs mélanges.

**14.** Composition pour utilisation selon la revendication 6, comprenant en outre un agent anti-inflammatoire additionnel choisi dans l'ensemble constitué par les AINS, les ligands de PPAR, les antihistaminiques, les antagonistes des cytokines pro-inflammatoires, les inhibiteurs de cytokines pro-inflammatoires, les inhibiteurs d'oxyde nitrique synthase, leurs combinaisons, et leurs mélanges.

**15.** Composition pour utilisation selon la revendication 14, dans laquelle ledit agent anti-inflammatoire additionnel est choisi dans l'ensemble constitué par les ligands de PPAR, les inhibiteurs de cytokines pro-inflammatoires, et leurs combinaisons.

**16.** Composition pour utilisation selon la revendication 9, comprenant en outre un agent anti-inflammatoire additionnel choisi dans l'ensemble constitué par les AINS, les ligands de PPAR, les antihistaminiques, les antagonistes des cytokines pro-inflammatoires, les inhibiteurs de cytokines pro-inflammatoires, les inhibiteurs d'oxyde nitrique synthase, leurs combinaisons, et leurs mélanges.

**17.** Composition pour utilisation selon la revendication 10 ou 11, comprenant en outre un agent anti-inflammatoire additionnel choisi dans l'ensemble constitué par les AINS, les ligands de PPAR, les antihistaminiques, les antagonistes des cytokines pro-inflammatoires, les inhibiteurs de cytokines pro-inflammatoires, les inhibiteurs d'oxyde nitrique synthase, leurs combinaisons, et leurs mélanges.

**18.** Composition pour utilisation selon la revendication 16, dans laquelle ledit agent anti-inflammatoire additionnel comprend un inhibiteur d'oxyde nitrique synthase.

**19.** Composition pour utilisation selon la revendication 17, dans laquelle ledit agent anti-inflammatoire additionnel comprend un inhibiteur d'oxyde nitrique synthase.

**20.** Utilisation d'un DIGRA, d'un sel pharmaceutiquement acceptable de celui-ci, ou d'un ester pharmaceutiquement acceptable de celui-ci, pour la préparation d'un médicament destiné à traiter ou prévenir un glaucome ou la progression de celui-ci, dans laquelle le DIGRA est de formule I

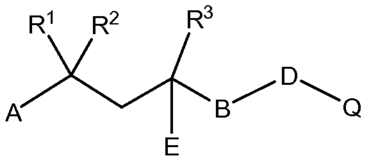

(I)

dans laquelle A est un groupe dihydrobenzofuranyle substitué par un atome d'halogène ; Q est un groupe quinolinyle ou isoquinolinyle substitué par un groupe alkyle en $C_1$ à $C_{10}$ ; $R^1$ et $R^2$ sont indépendamment choisis dans l'ensemble constitué par les groupes alkyle en $C_1$ à $C_5$ substitués et non substitués ; B est un groupe alkylène en $C_1$ à $C_3$ ; D est le groupe -NH- ; E est le groupe hydroxy, $R^3$ est un groupe trifluorométhyle,
et dans laquelle ledit glaucome résulte d'une inflammation chronique.

**21.** Utilisation selon la revendication 20, comprenant en outre l'utilisation d'un agent anti-inflammatoire additionnel pour la préparation.

**22.** Utilisation selon la revendication 21, dans laquelle ledit agent anti-inflammatoire additionnel est choisi dans l'ensemble constitué par les AINS, les ligands de PPAR, leurs combinaisons, et leurs mélanges.

**23.** Utilisation selon la revendication 20, dans laquelle ledit DIGRA, sel pharmaceutiquement acceptable de celui-ci, ou ester pharmaceutiquement acceptable de celui-ci, est incorporé dans une composition selon l'une quelconque des revendications 3, 4, 7 et 8.

**24.** Utilisation selon la revendication 20, comprenant en outre l'utilisation, dans ladite préparation, d'un autre agent thérapeutique ou prophylactique pour traiter, réduire ou prévenir une augmentation de la pression intraoculaire, une perte de cellules ganglionnaires rétiniennes, ou les deux.

**25.** Utilisation selon la revendication 24, dans laquelle ledit autre agent thérapeutique ou prophylactique est choisi dans l'ensemble constitué par les inhibiteurs d'acétylcholinestérase, les agonistes cholinergiques muscariniques, les inhibiteurs d'anhydrase carbonique, les analogues de prostaglandine, les antagonistes β-adrénergiques, les agonistes □-adrénergiques, les agents osmotiques, et leurs combinaisons.

FIG. 1A

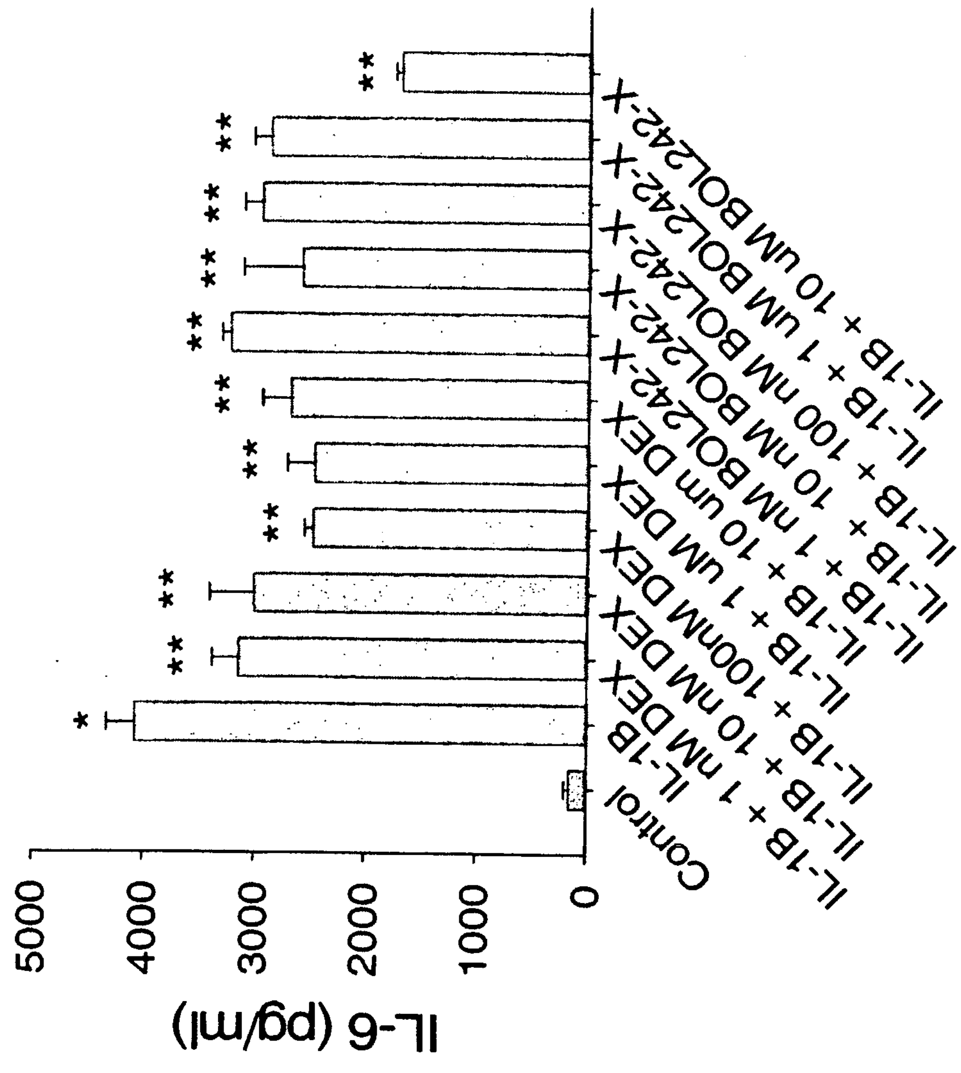

IL-6 (pg/ml)
5000
4000
3000
2000
1000
0
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1B

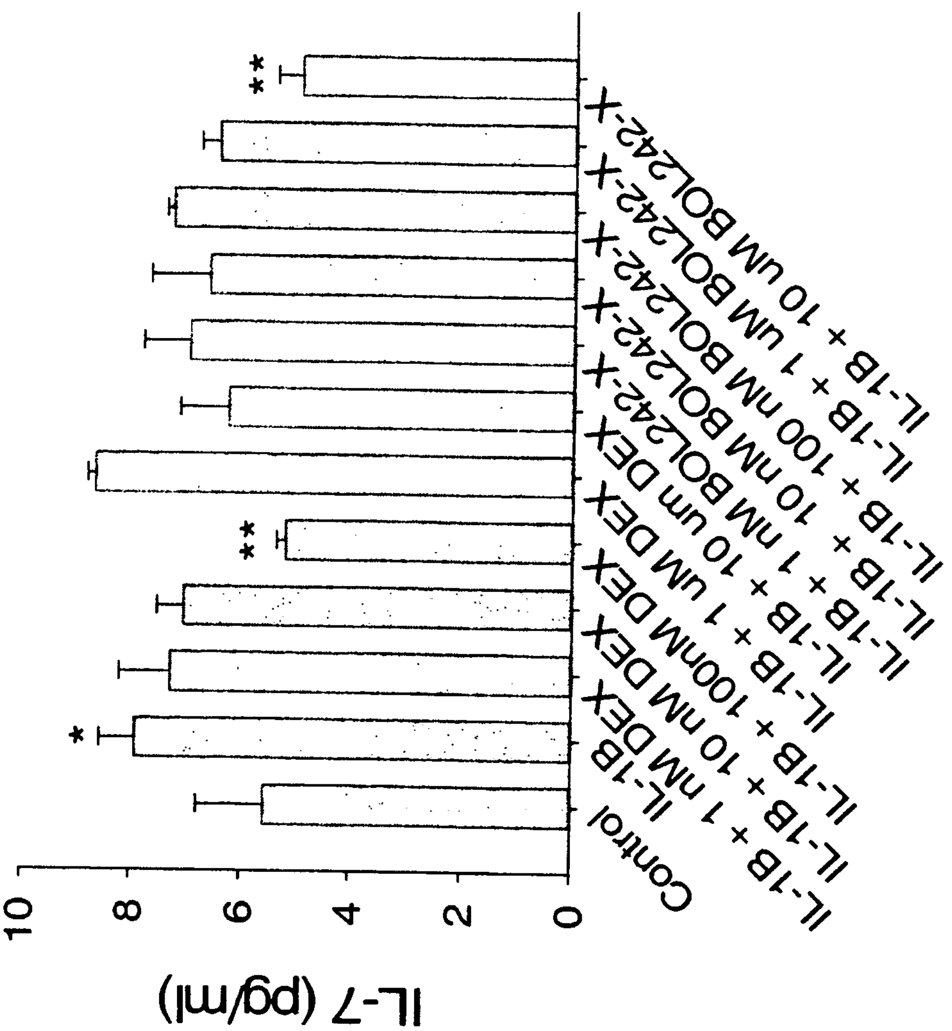
IL-7 (pg/ml)
10
8
6
4
2
0
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 1C

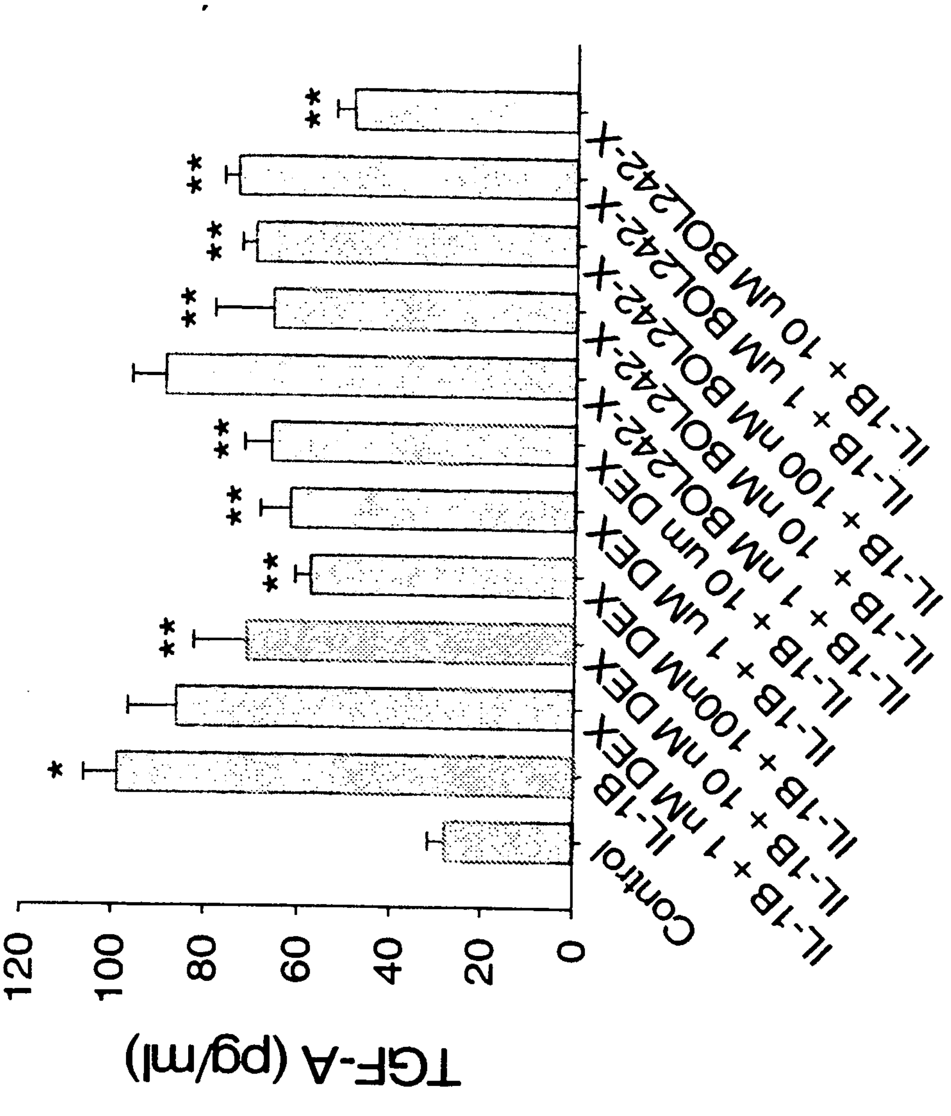
TGF-A (pg/ml)
0
20
40
60
80
100
120
*
**
Control
IL-1B
IL-1B + 1 nM DEX
IL-1B + 10 nM DEX
IL-1B + 100mM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 um BOL242-X

FIG. 1D

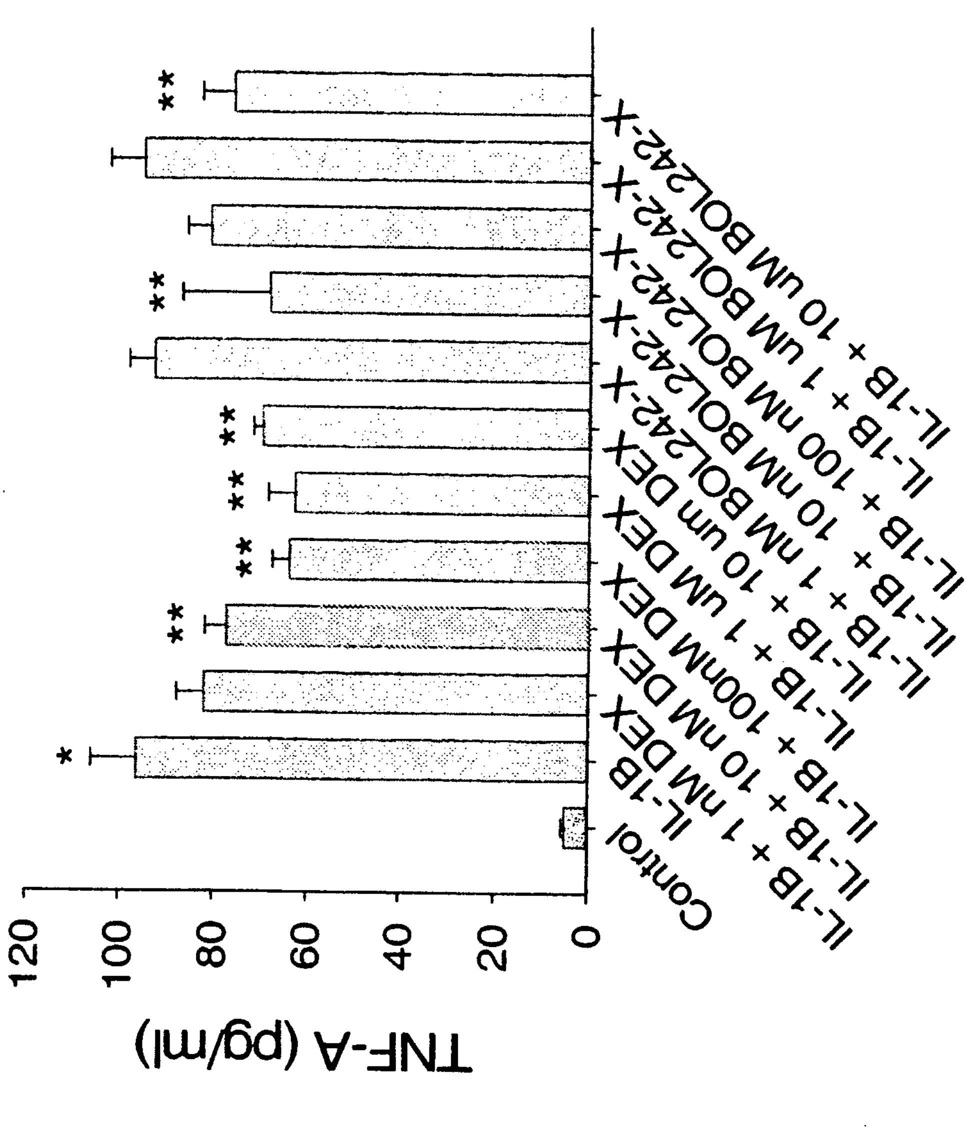
TNF-A (pg/ml)
0
20
40
60
80
100
120
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X
*
**

# FIG. 1E

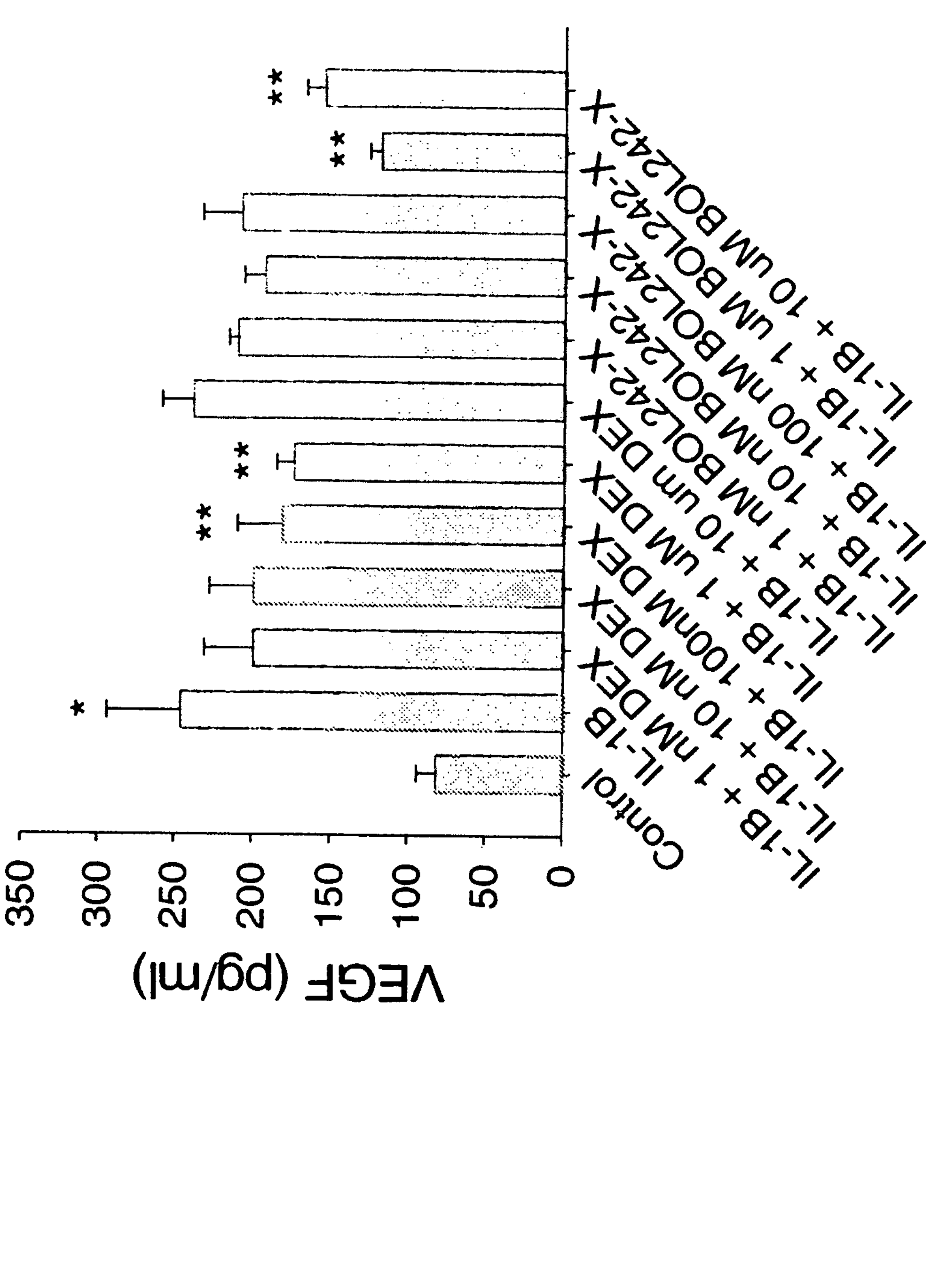
VEGF (pg/ml)
350
300
250
200
150
100
50
0
*
**
**
**
**
Control
IL-1B
IL-1B + 1 nM DEX
IL-1B + 10 nM DEX
IL-1B + 100nM DEX
IL-1B + 1 uM DEX
IL-1B + 10 uM DEX
IL-1B + 10 nM BOL242-X
IL-1B + 100 nM BOL242-X
IL-1B + 1 uM BOL242-X
IL-1B + 10 uM BOL242-X

FIG. 1F

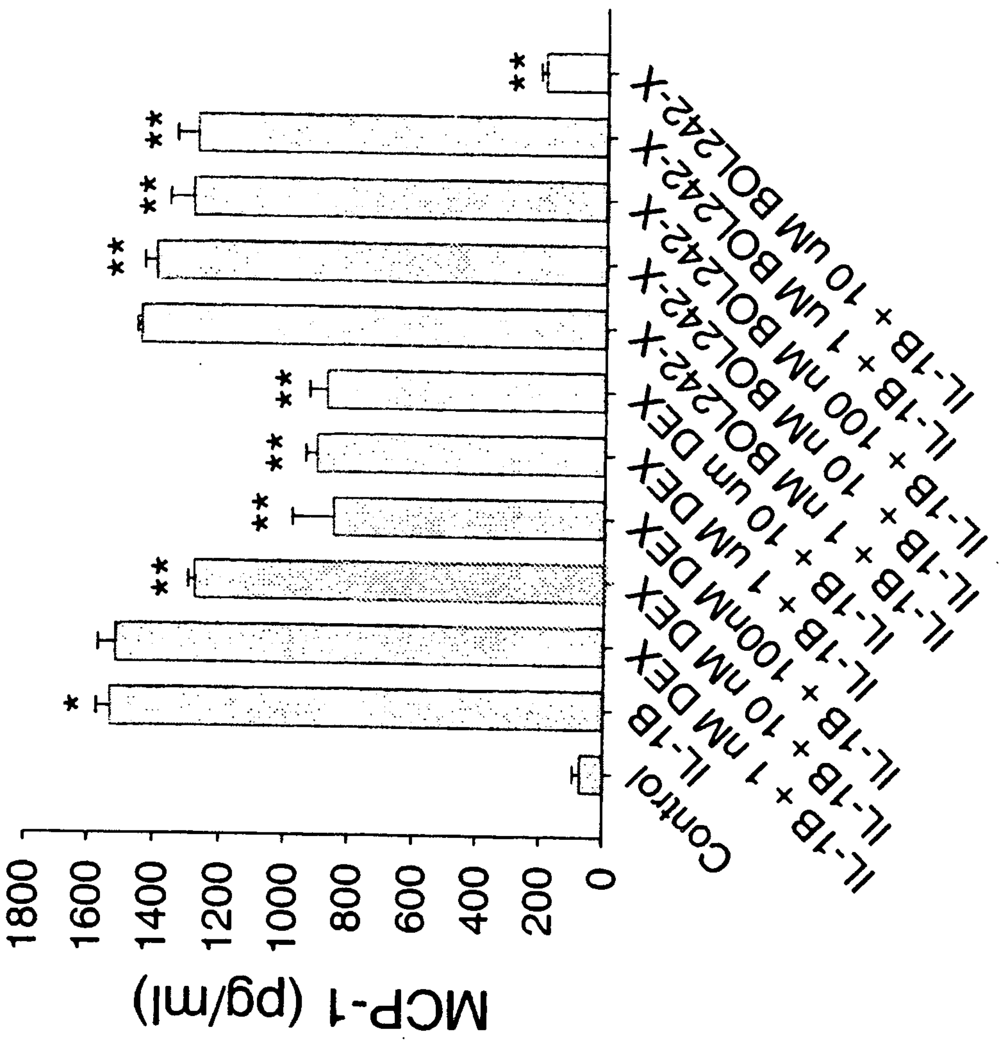
MCP-1 (pg/ml)
0
200
400
600
800
1000
1200
1400
1600
1800
Control
IL-1B
IL-1B + 1 nM DEX
IL-1B + 10 nM DEX
IL-1B + 100nM DEX
IL-1B + 1 um DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 2

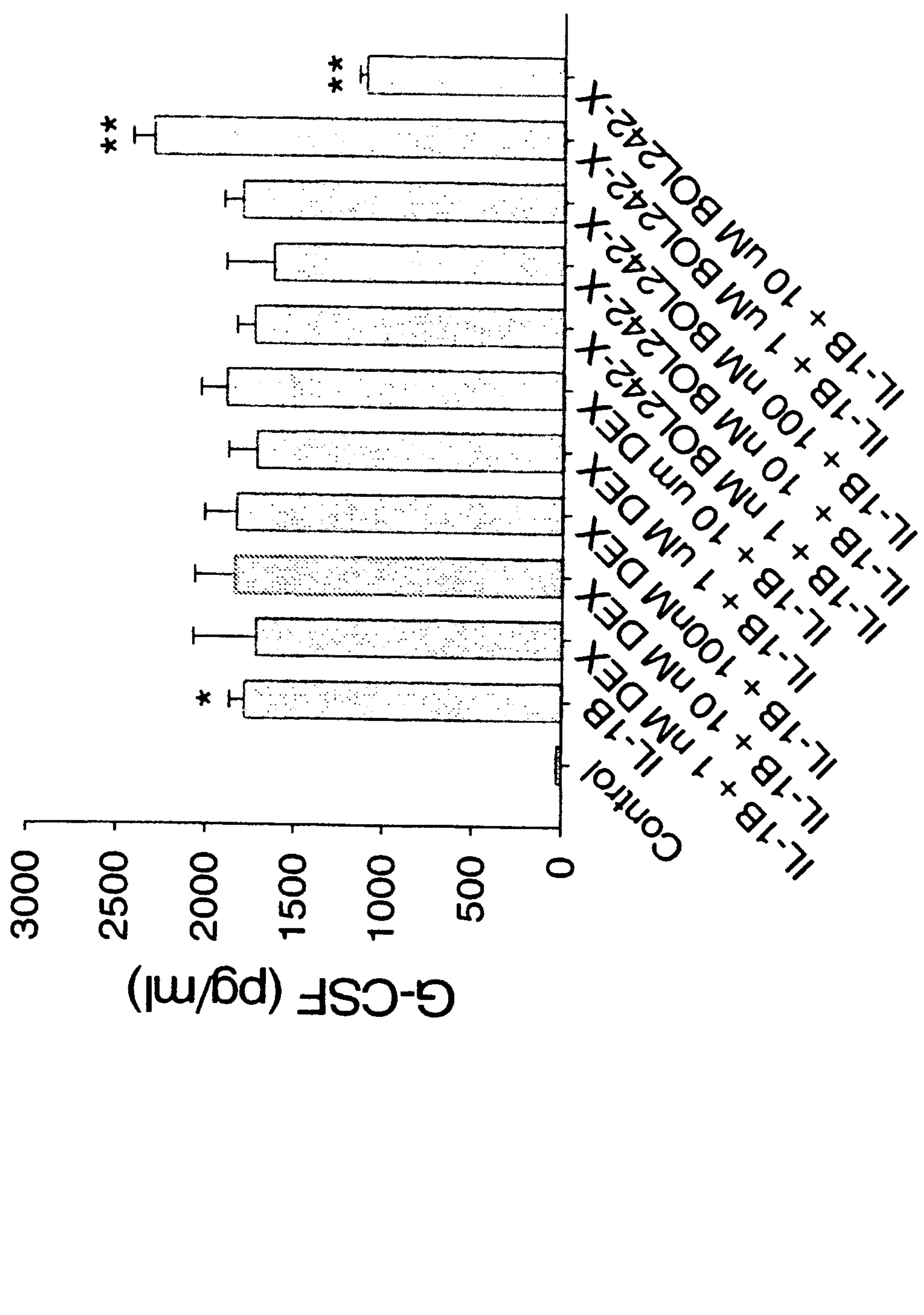
G-CSF (pg/ml)
0
500
1000
1500
2000
2500
3000
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 3A

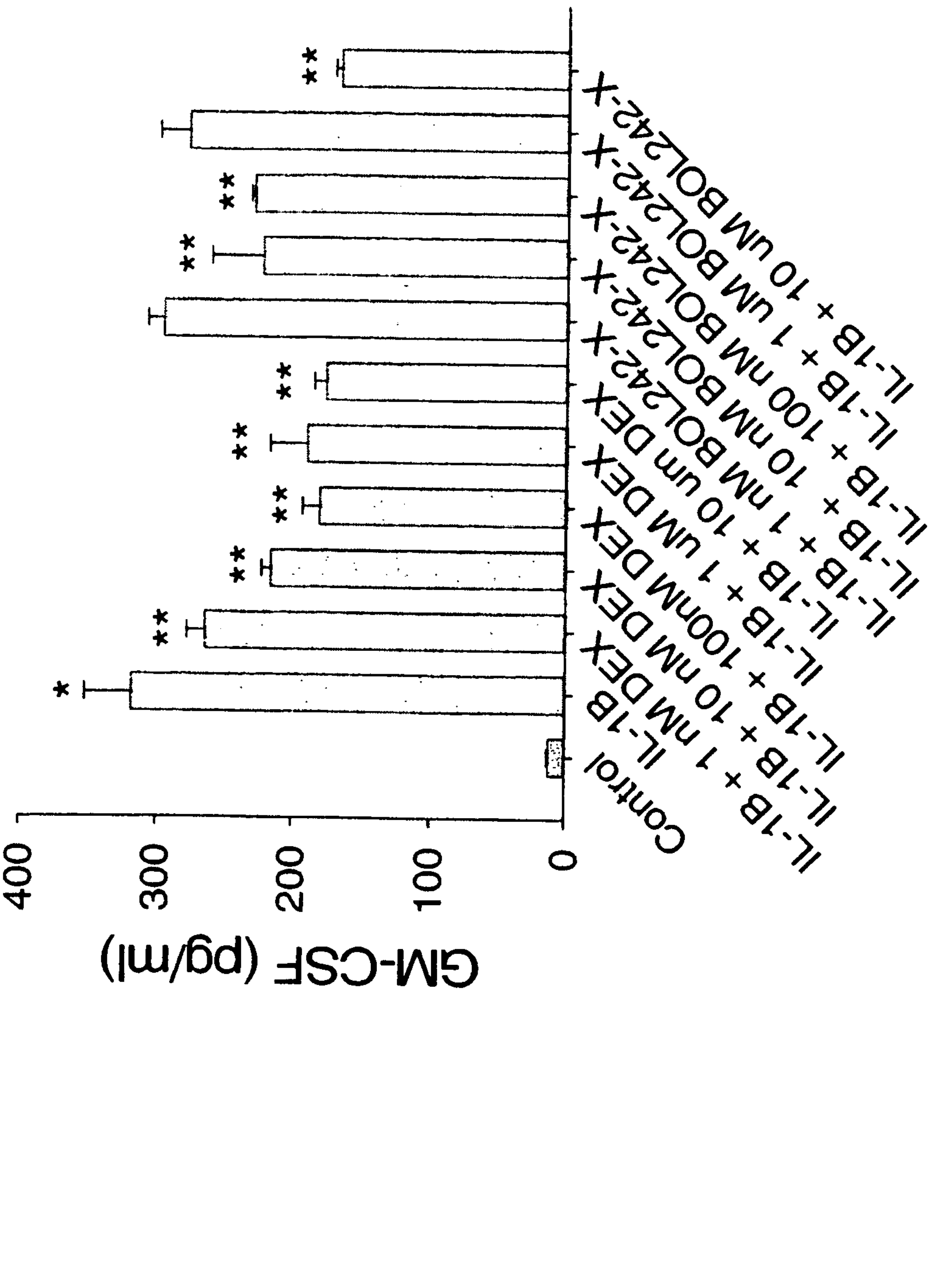
GM-CSF (pg/ml)
400
300
200
100
0
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X
*
**

FIG. 3B

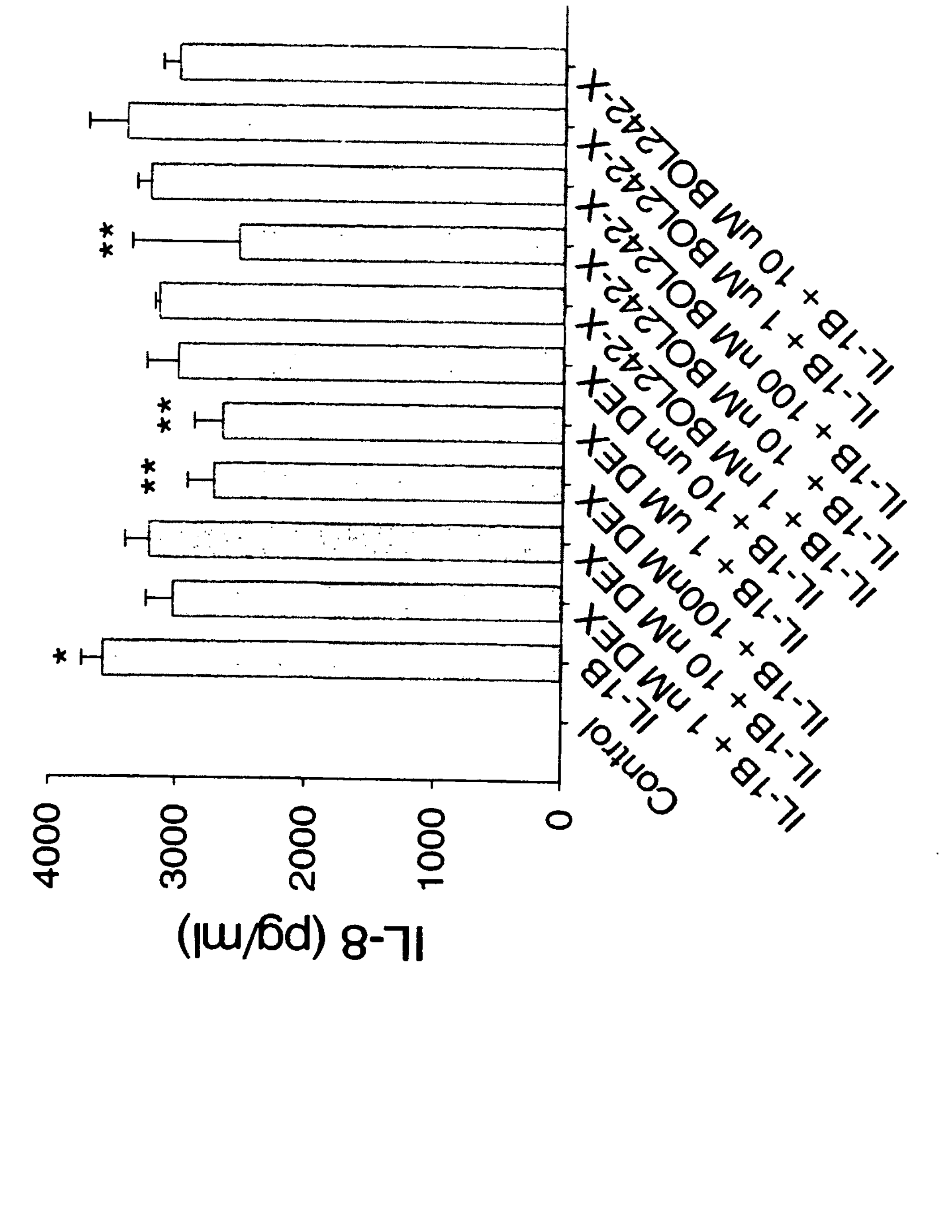
IL-8 (pg/ml)
0
1000
2000
3000
4000
*
**
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

FIG. 3C

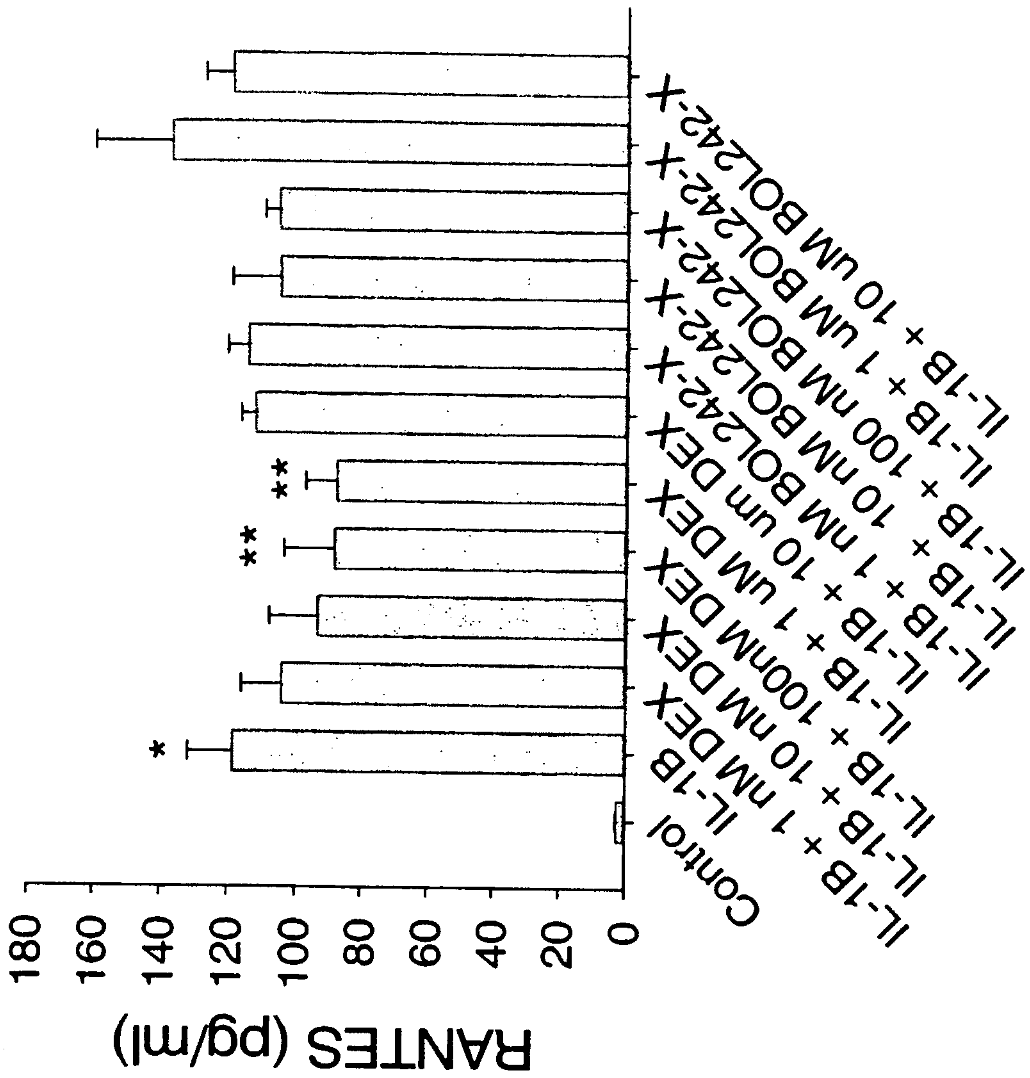
RANTES (pg/ml)
180
160
140
120
100
80
60
40
20
0
*
**
**
Control
IL-1B
IL-1B + 1 nM DEX
IL-1B + 10 nM DEX
IL-1B + 100nM DEX
IL-1B + 1 uM DEX
IL-1B + 10 um DEX
IL-1B + 1 nM BOL242-X
IL-1B + 10 nM BOL242-X
IL-1B + 100 nM BOL242-X
IL-1B + 1 uM BOL242-X
IL-1B + 10 uM BOL242-X

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60841437 B
- US 6903215 B2
- US 6960581 B2
- WO 03082787 A1
- WO 03082280 A1
- WO 0359899 A1
- WO 0304195 A1
- WO 2004063163 A1
- WO 2004075864 A1
- WO 2005095401 A1
- US 20060116396 A1
- WO 2008021729 A2
- US 6897224 B
- US 6903215 B
- US 6960581 B
- US 20060116396 A
- WO 2006050998A1 A
- US 20040029932 A
- US 20040162321 A
- US 20040224992 A
- US 20050059714 A
- US 20050176706 A
- US 20050203128 A
- US 20050234091 A
- US 20050282881 A
- US 20060014787 A
- US 20060030561 A
- US 6242196 B, Spiegelman
- US 6316465 B
- US 5378475 A
- US 5773019 A
- US 5902598 A
- US 6001386 A
- US 6051576 A
- US 6726918 A
- US 20040219512 A


### Non-patent literature cited in the description


- **M. RUDZINSKI ; H.U. SARAGOVI.** *Curr. Med. Chem.-Central Nervous System Agents,* 2005, vol. 5, 43
- **R.N. SAHA ; K. PAHAN.** *Antioxidants & Redox Signaling,* 2006, vol. 8 (5, 6), 929
- **R. KORHONEN et al.** *Curr. Drug Target-Inflam. & Allergy,* 2005, vol. 4, 471
- **A. IZZOTTI et al.** *Mutat. Res.,* 2006, vol. 612 (2), 105
- **G. TEZEL ; X. YANG.** *Expt'l Eye Res.,* 2005, vol. 81, 207
- **G. TEZEL et al.** *Invest. Ophthalmol. & Vis. Sci.,* 2001, vol. 42 (8), 1787
- **P.J. BARNES.** *Clin. Sci.,* 1998, vol. 94, 557-572
- **S. WISSINK et al.** *Mol. Endocrinol.,* 1998, vol. 12 (3), 354-363
- **P.J. BARNES ; M. KARIN.** *New Engl. J. Med.,* 1997, vol. 336, 1066-1077
- **H. SCHÄCKE et al.** *Pharmacol. Ther.,* 2002, vol. 96, 23-43
- **C-Y. JIANG et al.** *Nature,* 1998, vol. 391, 82-86
- **A.E. GIORGINI et al.** *Horm. Metab. Res.,* 1999, vol. 31, 1-4
- **X. XIN et al.** *J. Biol. Chem.,* 1999, vol. 274, 9116-9121
- **MARTINDALE.** The Complete Drug Reference. Pharmaceutical Press, London, 1411-1416
- **REMINGTON.** The Science and Practice of Pharmacy. Lippincott Williams & Wilkins, New York, 2006, 291
- **EAKINS KE.** Prostaglandin and non prostaglandin-mediated breakdown of the blood-aqueous barrier. *Exp. Eye Res.,* 1977, vol. 25, 483-498
- **NEUFELD AH ; SEARS ML.** The site of action of prostaglandin E2 on the disruption of the blood-aqueous barrier in the rabbit eye. *Exp. Eye Res.,* 1973, vol. 17, 445-448
- **UNGER WG ; COLE DP ; HAMMOND B.** Disruption of the blood-aqueous barrier following paracentesis in the rabbit. *Exp. Eye Res.,* 1975, vol. 20, 255-270
- Autacoids and Neuropeptides. **STJERNSCHANTZ J.** Pharmacology of the Eye. Springer-Verlag, New York, 1984, 311-365
- **WILLIAMS RN ; PATERSON CA ; EAKINS KE ; BHATTACHERJEE P.** Quantification of ocular inflammation: evaluation of polymorphonuclear leukocyte infiltration by measuring myeloperoxidase activity. *Curr. Eye Res.,* 1983, vol. 2, 465-469